# EUROPÄISCHE PATENTSCHRIFT

(11) **EP 3 206 643 B1**
(45) Veröffentlichungstag und Bekanntmachung des Hinweises auf die Patenterteilung: **25.08.2021**
(21) Anmeldenummer: 15781898.0
(22) Anmeldetag: 09.10.2015
(51) Int. Cl.: A61F 9/008

(54) **SYSTEME FÜR DIE KURZPULS-LASER-AUGENCHIRURGIE**
SYSTEMS FOR SHORT-PULSE-LASER EYE SURGERY
SYSTÈMES POUR LA CHIRURGIE DES YEUX UTILISANT UN LASER À COURTES IMPULSIONS

(30) Priorität: 17.10.2014 DE 102014221174; 08.10.2015 DE 102015219507
(43) Veröffentlichungstag der Anmeldung: 23.08.2017
(73) Patentinhaber: Carl Zeiss Meditec AG, 07745 Jena (DE)
(72) Erfinder: RILL, Michael, Stefan, 07751 Jena (DE); ANDREWS, Peter, 73447 Oberkochen (DE); DAMM, Tobias, 81545 München (DE); POMRAENKE, Robert, 07743 Jena (DE); RINGLING, Jens, 12623 Berlin (DE); WOLLWEBER, Thomas, 06648 Eckartsberga (DE); OESTREICH, Stephan, 12161 Berlin (DE); BERGT, Michael, 99425 Weimar (DE); MENAPACE, Rupert, 1090 Wien (AT); FABIAN, Ekkehard, 83022 Rosenheim (DE); PAPASTATHOPOULOS, Evangelos, 07745 Jena (DE); KÜHNER, Martin, 07639 Bad Klosterlausnitz (DE); STEINMETZ, Dietmar, 07751 Bucha (DE); HEINZ, Holger, 07745 Jena (DE); KOCH, Sascha, 07751 Jena Isserstedt (DE); LAQUA, Stephan, 07745 Jena (DE); NOBIS, Thomas, 07743 Jena (DE)
(74) Vertreter: Rößner, Ulrike
(86) Internationale Anmeldenummer: PCT/EP2015/073390
(87) Internationale Veröffentlichungsnummer: WO 2016/058931

(56) Entgegenhaltungen:
- WO-A1-2012/170966
- WO-A1-2013/000487
- US-A1- 2013 102 922
- US-A1- 2013 265 545

## Beschreibung

Die vorliegende Erfindung betrifft ein System für die Kurzpuls-Laser-Augenchirurgie mit einer Kurzpuls-Laserquelle, Strahlführungsmittel und einen Applikator-Kopf zur Leitung einer Kurzpuls-Laserstrahlung von der Kurzpuls-Laserquelle auf das zu operierende Auge, einem Operations-Mikroskop mit einem Mikroskop-Kopf und einer Steuereinheit, einem Gehäuse sowie einem ersten Gelenkarm an dem der Mikroskop-Kopf angeordnet ist und einen zweiten Gelenkarm an dem ein Applikator-Kopf angeordnet ist, und mit einer Schnittstelle am Applikator-Kopf und am Mikroskop-Kopf zum mechanischen und optischen Verbinden bzw. Lösen von Applikator-Kopf und Mikroskop-Kopf.

Die Offenbarung betrifft auch ein Kurzpuls-Lasersystem für die Augenchirurgie mit einer Kurzpuls-Laserquelle, einem die Divergenz einer Kurzpuls-Laserstrahlung der Kurzpuls-Laserquelle variierendem Linsensystem, einem x/y-Scansystem, einem Gelenkarm und einem in x- und y-Richtung beweglichen Objektiv; eine Patienten-Schnittstelle zur Fixierung der relativen Lage eines Auges zu einem optischen System für die Kurzpuls-Laser-Augenchirurgie und ein Computerprogramm-Produkt zur Kodierung einer Steuereinheit eines Kurzpuls-Lasersystems für die Augenchirurgie.

Die Offenbarung betrifft weiterhin ein Verfahren zur Positionierung eines Applikator-Kopfes und eines Mikroskop-Kopfes in einem System für die Kurzpuls-Laser-Augenchirurgie, Verfahren für die Schnittführung mit einem Kurzpuls-Lasersystem für die Augenchirurgie und Referenzierungsverfahren für verschiedene Schnitte und für eine Intraokularlinse.

Katarakt-Operationen sind die am häufigsten durchgeführten Operationen am menschlichen Auge und stehen daher im Fokus ständiger Verbesserungen bzgl. der Qualität des Operationsergebnisses, Effizienz in der Operationsdurchführung und Risikominimierung. Durch jüngste Entwicklungen und Fortschritte in der ophthalmologischen Femtosekunden (fs)-Lasertechnologie, vor allem im Bereich der refraktiven Augenchirurgie, und der Optischen Kohärenz-Tomographie (OCT) als Bildgebungstechnologie werden Kataraktoperationen zunehmend automatisiert. Hierbei werden Kurzpuls-Laser eingesetzt, um Augengewebe mittels Photodisruption zu "schneiden". Diese Technologie wird im Folgenden als lasergestützte Kataraktchirurgie (LCS) bezeichnet. Nach aktuellen Anwendungsprinzipien werden im Rahmen der LCS die Kapsulotomie (kreisrundes Aufscheiden des vorderen Kapselsacks der Augenlinse), die Linsenfragementierung (Zerteilen des Augenlinsenkerns), Zugangsschnitte in der Cornea (Hauptzugangs- und Hilfsschnitte), sowie arkuate Inzisionen (kreisförmige Schnitte zum Reduzieren eines Hornhautastigmatismus) durchgeführt, wobei letzteres über dem Umfang der klassischen Kataraktchirurgie signifikant hinausgeht, und das Gebiet der refraktiven Augenchirurgie berührt.

In US 6,325,792 B1 wird vorgeschlagen, Pulse eines Femtosekunden-Lasers in die Augenlinse zu fokussieren, um die Augenlinse zu "verflüssigen" - dies entspricht der oben genannten Linsenfragmentierung -oder aber um die Kapsulotomie zu schneiden. Die Positionierung des Pulsfoki des Femtosekunden-Lasers erfolgt dabei anhand einer Ultraschall-Bildgebung.

In US 5,246,435 wird offenbart, Pulse eines Kurzpuls-Lasers in einem dreidimensionalen Schnittmuster in die natürliche Linse des Auges zu fokussieren, um durch die Schnitte und die anschließende Blasenbildung die Linse in Bruchstücke zu fragmentieren und dadurch zu verflüssigen.

In US 6,454,761B1 wird vorgeschlagen, die Optische Kohärenz-Tomographie (OCT) anstelle der Ultraschall-Bildgebung für die automatische Positionierung von Laserimpulsen bei augenchirurgischen Operationen an der Cornea oder anderen transparenten Strukturen, z.B. beim Beseitigen eines Katarakts in der Augenlinse, zu verwenden.

Erst vor wenigen Jahren erlaubte die zunehmende Reife der Femtosekunden-Lasertechnologie und der OCT-Technologie eine Kombination und Integration dieser beiden Technologien und die Etablierung von weitestgehend automatisierten Femtosekunden-Lasersystemen in der Kataraktchirurgie. Zum Ablenken der Femtosekunden-Pulse werden zum einen feststehende Objektive und schnelle Spiegel-Scannern zur lateralen x/y-Ablenkung des Laserstrahles im Auge und langsam verstellbare Linsen zur z-Ablenkung der Fokusposition entlang einer optischen Achse des Auges eingesetzt. Solche Systeme werden etwa in US 2006/195076 A1 oder US 2009/131921 A1 beschrieben. Zum anderen sind auch Systeme bekannt, bei denen das Objektiv lateral langsam bewegt wird, wobei sich eine schnell verstellende Linse zur z-Ablenkung des Fokus entlang der optischen Achse des Auges verwendet wird. Ein solches System ist in DE10 2011 085 046 A1 beschrieben.

Während in den ersten Entwicklungsjahren der LCS einige anwendungsbedingte Probleme insbesondere durch die Einführung eines Flüssigkeitsinterfaces als mechanisch-optischer Kontakt zwischen Lasersystem und Auge gelöst wurden, siehe US 2012/0078241 A1 oder US 6,019,472, stand die Integration der Technologien in ein Gerät und weniger die Integration der Technologien in einen Gesamtarbeitsablauf bzw. ein Arbeitsumfeld im Vordergrund. Insbesondere das Zusammenspiel zwischen dem Femtosekunden-Laser und dem weiterhin bei Katarakt-Operationen notwendigen Operations-Mikroskop zeigt in den am Markt verfügbaren Systemen erhebliche Defizite.

Die meisten der derzeit bekannten Systeme sind unabhängig vom Operations-Mikroskop und stehen aufgrund Ihrer Größe oftmals außerhalb des später für die eigentliche Implantierung der Intraokularlinse (IOL) genutzten Operationssaals. Dadurch ist in der Regel ein zeitaufwendiges Umpositionieren und Umbetten des Patienten notwendig. Erst in jüngerer Zeit wurde dieses Defizit erkannt und entsprechende Verbesserungen vorgeschlagen:
In DE 10 2010 022 298 A1 und US 2012/316544 A1 wird vorgeschlagen, den Femtosekunden-Laser direkt und im Operationsablauf permanent mit einem Operations-Mikroskop zu koppeln. Dafür sind jedoch die benötigten Komponenten nach aktuellem Stand der Technik noch zu groß, so dass ein solches System während der IOL-Implantierungsphase zu groß und daher für den Chirurgen zu einschränkend und hinderlich wäre.

Die WO 2013/0003487 A1 hingegen schlägt ein System aus Lasersystem und Operationsmikroskop vor, in dem der Mikroskop-Kopf und der Laser-Applikationskopf funktional zueinander positioniert oder wieder getrennt werden können. Laser-Applikationskopf und Mikroskop-Kopf sind hierzu an einem Schwenkarm bzw. einem Verschiebeschwenkarm angeordnet, die Bewegungen zueinander nur in einer Ebene zulassen, was die Flexibilität dieses Systems erheblich einschränkt.

Die WO 2012/170966 A1 bzw. die US 2012/0316544 A1 beschreiben ein System für die Femtosekunden-Laserchirurgie, das zwei bewegliche Arme aufweist, wobei der erste Arm ein Operationsmikroskop aufweist. Durch den zweiten Arm wird der Laserstrahl zum Laseraustrittsort und über einen halbdurchlässigen Spiegel zum Anwendungsort geleitet. Diese Arme sind in der Nähe des Laseraustrittsorts über eine Klammer nichtlösbar, aber derart beweglich miteinander verbunden, dass sie zwei verschiedene Positionen zueinander einnehmen können. In einer ersten Position befindet sich der Laseraustrittsort des zweiten Arms unter das Operationsmikroskop in dessen Beobachtungsbereich, und in einer zweiten Position ist der Laseraustrittsort des zweiten Arms aus dem Beobachtungsbereich des Operationsmikroskops herausgeschwenkt. Trotzdem befindet sich dieser zweite Arm mit seinem Laseraustrittsort während der Behandlungsschritte, in denen keine Laserstrahlung benötigt wird, in der Nähe des Arbeitsbereichs und schränkt die Arbeitsfreiheit des Chirurgen ein. Zudem ist es ein potentielles Sicherheitsrisiko, den zweiten Arm mit seinem Laseraustrittsort stets mitzuführen, auch wenn nur manuelle Operationsschritte durchgeführt werden.

In der WO 2008/098388 A1 wird für die Cornea-refraktive Augenchirurgie ein Femtosekunden-Laser bei Bedarf unter ein Operations-Mikroskop, quasi zwischen dem Operations-Mikroskop und dem Patienten, eingeschoben und an das Auge angedockt. Hier arbeiten das Operations-Mikroskop und der Femtosekunden-Laser quasi sequentiell und unabhängig voneinander. Vor allem aber sind sie nach wie vor separate Geräte.

Des Weiteren haben sich bei den etablierten Systemen eine Reihe von Defiziten bzgl. spezifischer Komponenten gezeigt, welche die Qualität des Operationsergebnisses, die Effizienz in der Operationsdurchführung oder die Risikominimierung negativ beeinträchtigen.

Ein Mikroobjektiv-Scan, wie in WO 2008/098388 A1 beschrieben, ist zwar relativ zeiteffizient bzgl. z-Ablenkung für Kapsulotomie-Schnitte, oder für die Linsenfragmentierung wie in DE 10 2011 085 046 A1 gezeigt. Bzgl. Zugangsschnitten, die nicht nur eine kleinräumige Bewegung entlang der optischen Achse des Auges, sondern auch eine kleine laterale Bewegung des Mikroobjektives vorsehen, wie in US 2007/173794 A1 offenbart, ist diese Lösung jedoch sehr zeitintensiv.

Ferner ist die Schnittführung bei Systemen mit schneller z-Ablenkung für die Kapsulotomie zeitkritisch. Während bei schnellen Galvoscan-Systemen eine geschlossene Bahn in einer lateralen x/y-Ebene für die Kapsulotomie kein Problem darstellt, ist bei Systemen mit schneller z-Ablenkung, bei den das Schließen der Bahn erst nach einiger Zeit erfolgt, und sich das Auge in dieser Zeit bewegen kann, sicherheitskritisch. Auch bei cornealen Zugangs- und Hilfsschnitten kommt der Vorteil einer schnellen z-Ablenkung der Laserstrahls nicht zum Tragen, da auch hier vor allem lange laterale Bahnen zurückgelegt werden müssen.

Während sich obige Punkte im Besonderen auf Systeme mit reinen Mikroobjektiv-Scans beziehen, ergeben sich eine Reihe von Verbesserungen für Systeme mit kombiniertem Mikroobjektiv- und Spiegel-Scan. Kombinierte Scan-Systeme sind reinen Spiegel-Scan-Systemen bzgl. der Schnittqualität sogar potentiell überlegen.

Auch die derzeit verwendeten Kontaktgläser bzw. Patienten-Schnittstellen sind in ihrer Handhabung kompliziert, teuer in der Herstellung, weisen viele fehleranfällige Komponenten auf und sind häufig ungünstig dimensioniert.

Schlussendlich werden in heute bekannten Systemen auch die OCT-Signale durch viele Reflexe im System gestört. Weiterhin weisen etablierte OCT-Lösungen ebenfalls viele fehleranfällige und langsame Komponenten auf.

Aufgabe der vorliegenden Erfindung ist es deshalb, Systeme und Verfahren für die Kurzpuls-Laser-Augenchirurgie zu beschreiben, mit denen die Qualität des Operationsergebnisses und der typische Arbeitsablauf verbessert, die Effizienz der Operationsdurchführung und die Sicherheit der Operation erhöht werden kann.

Diese Aufgabe wird durch ein System für die Kurzpuls-Laser-Augenchirurgie gemäß Anspruch 1 gelöst.

Ein solches System umfasst ein Kurzpuls-Lasersystem, das eine Kurzpuls-Laserquelle, ein Strahlführungsmittel und einen Applikator-Kopf zur Leitung einer Kurzpuls-Laserstrahlung von der Kurzpuls-Laserquelle auf das zu operierende Auge enthält. Eine Kurzpuls-Laserquelle ist dabei eine Laserquelle, die das Licht nicht kontinuierlich sondern in gepulster Form emittiert. Das bedeutet, dass das Licht in zeitlich begrenzten Portionen emittiert wird. Üblicherweise liegen die Pulsraten eines solchen Kurzpuls-Lasers im Femtosekunden- oder Pikosekundenbereich. Aber auch Pulsraten im Attosekundenbereich sind möglich. Durch die gepulste Lichtemission können sehr hohe Energien realisiert werden, die für Laser-Gewebe-Wechselwirkungen via Mehrphotonen-Absorption, wie z.B. der Photodisruption oder Plasmainduzierten Photoablation, benötigt werden. Dies ist bei allen Anwendungen der Fall, bei denen nicht ausschließlich an der Oberfläche Material abgetragen wird, sondern Wechselwirkungen in allen drei Dimensionen erzielt werden soll.

Ein Strahlführungsmittel sorgt dafür, dass die von der Kurzpuls-Laserquelle emittierte Kurzpuls-Laserstrahlung in einer vorgesehenen Art und Weise zu einem Austrittsort der Kurzpuls-Laserstrahlung aus dem System geleitet wird. Ein Strahlführungsmittel kann deshalb beispielsweise durch einen Lichtleiter oder durch ein Spiegelsystem realisiert sein. Auch ist es möglich, dass das Strahlführungsmittel durch eine Gesamtheit dieser oder ähnlicher Komponenten realisiert ist.

Der Applikator-Kopf, der sich an dem der Kurzpuls-Laserquelle entgegengesetzten Ende des Strahlführungsmittels anschließt, bildet den Austrittsort der Kurzpuls-Laserstrahlung. Üblicherweise enthält er eine Optik, wie eine Objektivlinse oder, in einem komplizierteren Aufbau, ein Objektiv mit mehreren optischen Elementen, insbesondere mit mehreren Linsen.

Vorteilhaft ist es, wenn das Kurzpuls-Lasersystem weiterhin ein x/y-Ablenksystem, auch als x/y-Scansystem bezeichnet, sowie ein Ablenksystem bzw. Scansystem für die z-Richtung und/oder ein die Divergenz variierendes Linsensystem aufweist. Die Möglichkeit, den Fokus der Kurzpuls-Laserstrahlung in x-Richtung und y-Richtung sowie in z-Richtung in einem Volumen, das dem Austrittsort der Kurzpuls-Laserstrahlung folgt, abzulenken, kann auch durch mehrere Ablenkvorrichtungen für jeweils eine Richtung realisiert sein, beispielsweise einen Scanner für eine langsame Bewegung über einen größeren Bereich und einen für eine sehr schnelle Bewegung über einen kleinen Bereich. Insbesondere von Interesse sind optimale Lösungen für die Ablenkung in z-Richtung.

Insbesondere kann deshalb das Kurzpuls-Lasersystem ein die zusätzlichen technischen Merkmale des abhängigen Anspruchs 13 umfassendes Kurzpuls-Lasersystem sein.

Das System für die Kurzpuls-Laser-Augenchirurgie umfasst weiterhin ein Operations-Mikroskop mit einem Stativ und einem Mikroskop-Kopf. Der Mikroskop-Kopf enthält die Optik des Operations-Mikroskops. Mit einem solchen Operations-Mikroskop ist es möglich, sich zu jeder Zeit einen optischen Überblick über den Stand der Behandlung zu verschaffen. Das Operations-Mikroskop trägt aber auch dazu bei, dass ein zu behandelndes Auge zum System entsprechend ausgerichtet werden kann.

Das System für die Kurzpuls-Laser-Augenchirurgie umfasst auch eine Steuereinheit, welche zur Steuerung des Systems für die Durchführung einer Kurzpuls-Laser-Augenchirurgie eingerichtet ist. Die Steuereinheit kann einteilig oder mehrteilig ausgestaltet sein. Die Komponenten des Systems für die Kurzpuls-Laser-Augenchirurgie sind vorteilhaft mit der Steuereinheit über Kommunikationswege verbunden. Im Falle einer Mehrteiligkeit der Steuereinheit sind auch alle Komponenten der Steuereinheit vorteilhaft über Kommunikationswege miteinander verbunden. Solche Kommunikationswege können mittels entsprechender Kabel und/oder auch kabellos realisiert werden.

Weiterhin umfasst das System für die Kurzpuls-Laser-Augenchirurgie ein Gehäuse, das mindestens die Kurzpuls-Laserquelle umschließt, sowie einen ersten und einen zweiten Gelenkarm, der am Gehäuse oder an einer Verlängerung des Gehäuses angeordnet ist. Ein Gelenkarm umfasst mehrere Gelenkglieder, die zueinander beweglich sind. Die Gelenkglieder sind dabei so angeordnet, dass jeweils zwei Gelenkglieder durch mindestens ein Gelenk beweglich verbunden sind.

Am ersten Gelenkarm, vorteilhaft an dem den Gehäuse abgewandten Ende des Gelenkarms, ist der Mikroskop-Kopf angeordnet. Dieser Gelenkarm bildet somit - zusammen mit dem Gehäuse - das Stativ des Operations-Mikroskops. Am zweiten Gelenkarm, wiederum vorteilhaft an dem vom Gehäuse abgewandten Ende des Gelenkarms, ist der Applikator-Kopf angeordnet. Die Länge des zweiten Gelenkarms, an dem der Applikator-Kopf angebracht ist, ist vorteilhaft so ausgelegt, dass der gesamte Arbeitsbereich des Mikroskop-Kopfes des Operations-Mikroskops, der am ersten Gelenkarm angeordnet ist, in einem Halbkreis von ca.180° vor dem System für die Kurzpuls-Laser-Augenchirurgie ausgenutzt werden kann. Damit sind insbesondere auch die Längen der Gelenkglieder des zweiten Gelenkarms entsprechend ausgelegt.

Dabei ist eine Schnittstelle zwischen Applikator-Kopf und Mikroskop-Kopf vorgesehen, mit welcher der Applikator-Kopf und der Mikroskop-Kopf mechanisch und optisch miteinander verbunden und wieder gelöst werden können.

Die Schnittstelle zeichnet sich bevorzugt durch eine erste Struktur am ersten Gelenkarm und/oder am Mikroskop-Kopf und eine zweite Struktur am zweiten Gelenkarm und/oder am Applikator-Kopf aus, die entweder nach dem Schlüssel-Schloss-Prinzip aufeinander abgestimmt sind oder über ein Zwischenstück miteinander verbunden werden können.

Den Applikator-Kopf und den Mikroskop-Kopf mechanisch und optisch miteinander zu verbinden heißt dabei, neben der mechanischen Verbindung und damit der Herstellung einer festen Beziehung von Applikator-Kopf und Mikroskop-Kopf zueinander, beide so miteinander zu verbinden, dass der Abbildungsstrahlengang des Operations-Mikroskop durch den Applikator-Kopf hindurch verläuft. Dadurch wird ein optischer Weg für die mit dem Operations-Mikroskop zu beobachtenden Strukturen des Auges durch den Applikator-Kopf hindurch bereit gestellt.

Erfindungsgemäß durchläuft das Strahlführungsmittel für die Kurzpuls-Laserstrahlung den zweiten Gelenkarm. Das Strahlführungsmittel ist deshalb so ausgestaltet, dass es allen Bewegungen des zweiten Gelenkarms folgen kann und in jeder Position des zweiten Gelenkarms die Kurzpuls-Laserstrahlung zu ihrem Austrittsort am Applikator-Kopf in gleicher Qualität führen kann.

Weiterhin erfindungsgemäß sind der Applikator-Kopf und der Mikroskop-Kopf sowohl unabhängig voneinander als auch miteinander verbunden in einem dreidimensionalen Volumen beweglich. Die durch ihre Anordnung an den Gelenkarmen bedingte Beweglichkeit des Applikator-Kopfes und des Mikroskop-Kopfes in jede beliebige Richtung ohne jegliche mechanische durch das System bedingte Einschränkung des dreidimensionalen Volumens ist demnach auch gegeben, wenn der Applikator-Kopf und der Mikroskop-Kopf miteinander verbunden sind. Dies bedingt entsprechende zusätzliche Freiheitsgrade im ersten und zweiten Gelenkarm. Durch die Beweglichkeit des Applikator-Kopfes allein, vor allem aber verbunden mit dem Mikroskop-Kopf, ist der Austrittsort oder Kurzpuls-Laserstrahlung ebenfalls im dreidimensionalen Volumen beweglich - in einer bevorzugten Variante auch bezüglich seiner Strahlrichtung am Austrittsort So ist es beispielsweise auch möglich, den Patienten in nicht liegender Position, oder aber zwar in liegender Position, jedoch mit angestellter Liegeposition zu behandeln.

Mit dem vorliegenden System für die Kurzpuls-Laser-Augenchirurgie ist dabei nicht nur das Schneiden von Gewebe mittels plasmainduzierter Ablation und/oder Photodisruption- möglich, sondern auch das Verkleben von Gewebe mittels Koagulation sowie eine Abtragung von Gewebe durch ablative Effekte der Kurzpuls-Laserstrahlung mit einem solchen System erreichbar. Lediglich die Eigenschaften der Kurzpuls-Laserstrahlung müssen entsprechend den Anwendungszielen eingestellt werden.

In einer bevorzugten Ausgestaltung umfasst das erfindungsgemäße System für die Kurzpuls-Laser-Augenchirurgie weiterhin ein optisches Kohärenz-Tomographie (OCT)-Modul, das eine OCT-Lichtquelle, ein Interferometer und einen Detektor enthält. Das OCT-Modul kann dabei ebenfalls vom Gehäuse umschlossen sein.

Besonders bevorzugt ist das System für die Kurzpuls-Laser-Augenchirurgie, das ein OCT-Modul umfasst, welches wahlweise für eine Einkopplung einer von der OCT-Lichtquelle ausgesendeten Strahlung in den Mikroskop-Kopf oder in den Applikator-Kopf eingerichtet ist. Dies kann beispielsweise mit Hilfe einer oder mehrerer optischer Schaltstellen, die im Strahlengang der von der OCT-Lichtquelle ausgesendeten Strahlung wie auch der vom einem Beobachtungsziel im Auge zurückkommenden Strahlung der OCT-Lichtquelle vorgesehen sind. Sie können wahlweise so eingestellt werden, dass das OCT-Modul das Beobachtungsziel im Auge über den Mikroskop-Kopf oder über den Applikator-Kopf erreichen kann.

Die Einkopplung der Strahlung der OCT-Lichtquelle über den Applikator-Kopf hat den Vorteil, dass sie einfach und mechanisch stabil mit der therapeutischen Kurzpuls-Laserstrahlung überlagert werden kann. Damit können beide Strahlengänge zueinander kalibriert werden. Diese Variante wird daher in der Praxis für die Planung und Kontrolle der Kurzpuls-Laserbehandlung verwendet.
Die Einkopplung der Strahlung der OCT-Lichtquelle über den Mikroskop-Kopf ermöglicht hingegen dem Chirurgen, tomographische Aufnahmen des Patientenauges während und/oder nach der manuellen Operationsphase zu tätigen. Beispielsweise können mit Hilfe dieser Technologie Intraokularlinsen präzise ausgerichtet oder freie Partikel im Kammerwasser identifiziert und entfernt werden.

Weiterhin ist es technisch vorteilhaft, einen Ringspiegel zum Zusammenführen der Kurzpuls-Laserstrahlung und der von der OCT-Lichtquelle ausgesendeten Strahlung in das System für die Kurzpuls-Laser-Augenchirurgie einzubinden. Dabei erfolgt die Zusammenführung vorzugsweise derart, dass die Kurzpuls-Laserstrahlung am Ringspiegel reflektiert wird, während die von der OCT-Lichtquelle des OCT-Modules ausgesendete kurzkohärente Strahlung durch ein Loch im Ringspiegel in Richtung des Auges hindurch propagiert und der OCT-Detektor die reflektierte Strahlung der OCT-Lichtquelle vom Auge durch das Loch im Ringspiegel detektiert. Der Ringspiegel kann hierfür beweglich sein. Bevorzugt ist eine 90°-Stellung der Einkopplung der von der OCT-Lichtquelle ausgesendeten Strahlung in den Strahlengang der Kurzpuls-Laserstrahlung, wobei die Ringblende dabei in einer 45°-Stellung angeordnet ist.

In einem bevorzugten System für die Kurzpuls-Laser-Augenchirurgie weisen sowohl der erste Gelenkarm als auch der zweite Gelenkarm jeweils mindestens drei Gelenke auf.

Beim Vorliegen von drei Gelenken müssen mindestens zwei dieser drei Gelenke, idealerweise alle drei Gelenke, die Funktion eines Kugelgelenks erfüllen, d.h. nicht nur eine Rotationsmöglichkeit um eine einzige Achse bieten. Ein solches Gelenk muss es vielmehr ermöglichen, dass ein Gelenkglied zu dem benachbarten Gelenkglied, die beide durch das Gelenk miteinander in beweglicher Verbindung stehen, einen beliebigen Winkel im Raum beschreiben kann, wobei der Aktionsradius ggf. durch andere bauliche Hindernisse auf einen Teilbereich des Raumes eingeschränkt sein kann, jedoch nicht auf eine Bewegung innerhalb einer Ebene.

In einer speziellen Ausführung kann eines der drei Gelenke eine einzige Rotationsachse aufweisen. Bevorzugt erfüllen jedoch bei Vorliegen von nur drei Gelenken alle drei Gelenke die Funktion eines Kugelgelenks. Auf diese Art und Weise ist die optimale Beweglichkeit des ersten und des zweiten Gelenkarms, die beide am Gehäuse oder an einer Verlängerung des Gehäuses angeordnet sind, sowohl im miteinander verbundenen Zustand als auch unabhängig voneinander im dreidimensionalen Volumen gesichert.

Werden hingegen Gelenke genutzt, die jeweils nur eine Rotationsmöglichkeit um eine Achse bieten, so wird eine vergleichbare Beweglichkeit mit mindestens fünf Gelenken pro Gelenkarm realisiert, die unterschiedliche Rotationsachsen aufweisen. Davon sollten drei Gelenke die Rotation um senkrechte Achsen und zwei Gelenke die Rotation um horizontale Achsen ermöglichen, d.h. Kippachsen darstellen, die zu einem Verkippen des nach dem Gelenk folgenden Gelenkglieds führen.

Bevorzugt ist in dieser Variante - also bei Einsatz von Gelenken mit jeweils einer Rotationsmöglichkeit um eine Achse - ein Gelenkarm, der sechs Gelenke mit jeweils einer Rotationsachse pro Gelenk aufweist. In diesem Fall sollten drei Gelenke die Rotation um senkrechte Achsen und weitere drei Gelenke die Rotation um horizontale Achsen ermöglichen. Hier ist das Verkippen des nach dem Gelenk folgenden Gelenkgliedes bzw. eines Endstücks wie des Applikator-Kopfes oder des Mikroskop-Kopfes möglich.

Zusammenfassend sollen demnach die Gelenke eines jeden Gelenkarms bevorzugt sechs Freiheitsgrade verwirklichen, die durch je drei senkrechte und drei horizontale Rotationsachsen gegeben werden, wobei sich senkrechte und horizontale Rotationsachsen entlang eines Gelenkarms abwechseln. Insbesondere bietet ein Paar aus einem Gelenk mit senkrechter Rotationsachse und einem Gelenk mit horizontaler Rotationsachse, die in nächster Nähe zueinander angeordnet sind, dieselbe Funktion wie ein Kugelgelenk.

Des Weiteren bevorzugt weist das System für die Kurzpuls-Laser-Augenchirurgie eine Koppelstellung auf, die in der Steuereinheit derart kodiert ist, dass ein Verbinden von Applikator-Kopf und Mikroskop-Kopf über die Schnittstelle bei senkrechter Stellung sowohl des Applikator-Kopfes als auch des Mikroskop-Kopfes erfolgt, um jegliche mechanische Verspannungen zu vermeiden. Sind Applikator-Kopf und Mikroskop-Kopf erst einmal über die Schnittstelle verbunden, ist dann vorzugsweise ein gemeinsames Verkippen dieser Köpfe weiterhin möglich.

Das System für die Kurzpuls-Laser-Augenchirurgie ist vorteilhaft als mobiles System vorgesehen. Es enthält hierfür eine Vorrichtung zum Transport. Insbesondere kann diese als Rollensystem ausgestaltet sein, so dass das System für die Kurzpuls-Laser-Augenchirurgie für den Transport beispielsweise innerhalb eines Raumes oder von einem Raum in den anderen manuell beweglich bzw. fahrbar ist. In einer besonderen Ausführung wird das Rollensystem von einem Elektromotor unterstützt, um das ggf. schwere System für die Kurzpuls-Laser-Augenchirurgie in einfacher, präziser und ergonomischer Weise zu bewegen.

In einer vorteilhaften Ausgestaltung des Systems für die Kurzpuls-Laser-Augenchirurgie ist die Steuereinheit kodiert für eine automatische Nachführung der Lage der Kurzpuls-Laserstrahlung in Abhängigkeit von der Stellung des zweiten Gelenkarms. Dies dient der Korrektur von Lageabweichungen insbesondere des Fokuspunktes einer Kurzpuls-Laserstrahlung durch elastische Deformationen, die abhängig von der Stellung des zweiten Gelenkarms und einer gegebenen Gewichtsverteilung in und an diesem zweiten Gelenkarm sind, der Strahlführungsmittel enthält. Die Stellung des Gelenkarms definiert sich dabei durch die relativen Positionen der Gelenkglieder zueinander.

Die Nachführung der Lage der Kurzpuls-Laserstrahlung erfolgt dabei vorzugsweise automatisch in Bezug auf eine Justierpostion des zweiten Gelenkarms. In dieser Justierposition wird die optische Übertragung der Kurzpuls-Laserstrahlung ausgerichtet, in Folge dann die Abweichungen einer jeden von der Justierposition abweichenden Position des zweiten Gelenkarms bestimmt und die jeweilig notwendige Korrektur für diese Position festgelegt.

In einer weiteren vorteilhaften Ausgestaltung des Systems für die Kurzpuls-Laser-Augenchirurgie weist der zweite Gelenkarm mindestens eine Vorrichtung für einen vom ersten Gelenkarm unabhängigen Gewichtsausgleich auf.

Während eines chirurgischen Eingriffs ist der zweite Gelenkarm, an dem der Applikator-Kopf angeordnet ist zwar während der gesamten Nutzungsdauer des Kurzpuls-Lasersystems und folglich auch des Applikator-Kopfes mit dem ersten Gelenkarm, an dem der Mikroskop-Kopf angeordnet ist, verbunden. Jedoch trägt der zweite Gelenkarm ein verhältnismäßig hohes Gewicht: Ein Applikator-Kopf kann ein Gewicht von mehreren Kilogramm aufweisen. Dies kann nicht in jeder Position stabil vom ersten Gelenkarm getragen werden. Ein unabhängiger Gewichtsausgleich garantiert deshalb eine hohe Stabilität und Flexibilität der Bewegungen. Für applikative Zwecke kann es außerdem sinnvoll sein, während einer Behandlung den Mikroskop-Kopf vom Applikator-Kopf zu trennen. In diesem Fall müssen ebenfalls beide Gelenkarme einen eignen Gewichtsausgleich haben, um unkontrollierte und damit sicherheitsgefährdende Bewegungen des Systems zu vermeiden.

Der Gewichtsausgleich erfolgt dabei bzgl. einer ersten Kippachse, also einem ersten Gelenk, das eine Rotation um eine horizontale Achse ermöglicht. Wenn erforderlich, kann ein weiterer Gewichtsausgleich bzgl. einer weiteren Kippachse erfolgen.

Der Gewichtsausgleich kann beispielsweise realisiert werden durch eine Druckfeder in einem parallel zu einem Gelenkglied verlaufenden und entsprechend parallel beweglichen Federarmglied. In diesem ist eine Druckfeder enthalten, die an einem Zahnriemen zieht, der über zwei Zahnriemenräder in das parallel verlaufende Gelenkglied umgelenkt wird.

Eine solche Vorrichtung für einen unabhängigen Gewichtsausgleich des zweiten Gelenkarms, der ein Strahlführungsmittel enthält und den Applikator-Kopf als Austrittsort für Kurzpuls-Laserstrahlung trägt, ist eine sehr vorteilhafte Variante der Ausgestaltung des Systems für die Kurzpuls-Laser-Augenchirurgie. Sie ist jedoch ebenfalls von Vorteil für ein Kurzpuls-Lasersystem für die Augenchirurgie wie unten beschrieben, das einen Gelenkarm mit Strahlführungsmitteln und ein Objektiv umfasst, um einen Gewichtsausgleich für jede mögliche Stellung des Gelenkarms zu erreichen, so dass der Gelenkarm, der durch seine Bewegung auch den Austrittsort der Kurzpuls-Laserstrahlung aus dem Kurzpuls-Lasersystem bestimmt in jeder Position stabil verbleibt und optische Lagefehler gering gehalten werden.

In einer weiteren Ausgestaltung weist das System für die Kurzpuls-Laser-Augenchirurgie eine Parkposition und/oder eine Transportposition für den zweiten Gelenkarm mit dem Applikator-Kopf am Gehäuse auf, die auf die Geometrie des zweiten Gelenkarms und/oder des Applikator-Kopfes abgestimmt ist. Bevorzugt ist auch hier eine Struktur am Applikator-Kopf und/oder am zweiten Gelenkarm und eine Struktur am Gehäuse, die nach dem Schlüssel-Schloss-Prinzip aufeinander abgestimmt sind, vorgesehen. Unterstützt wird dies vorzugsweise durch die Kodierung einer entsprechenden Position des Applikator-Kopfes, die durch die Bewegung des zweiten Gelenkarms bestimmt ist, in der Steuereinheit des Systems für die Kurzpuls-Laser-Augenchirurgie.

Alternativ ist aber auch eine Parkposition und/oder eine Transportposition am Gehäuse allein durch die Kodierung einer entsprechenden Position des Applikator-Kopfes in der Steuereinheit des Systems für die Kurzpuls-Laser-Augenchirurgie möglich.

Unabhängig von der Ausgestaltung der Parkposition und/oder der Transportposition, insbesondere von der Art und Weise, in diese zu gelangen, ist die Parkposition und/oder die Transportposition aber dadurch gekennzeichnet, dass sie eine Position am Gehäuse bezeichnet, in welcher der Applikator-Kopf von dem Arbeitsbereich eines Bedieners des Systems, also insbesondere des Arztes, so weit entfernt ist, dass eine Kontamination des Bedieners durch versehentliche Berührung der Bauelemente unwahrscheinlich ist und der Applikator-Kopf möglichst geschützt vor Kollisionen mit anderen Teilen des Systems aber insbesondere mit seiner Umgebung ist. Allerdings ist es auch vorteilhaft, wenn die Parkposition nicht zu weit vom Arbeitsbereich des Bedieners entfernt ist, so dass die Kopplung von Applikator-Kopf und Mikroskop-Kopf trotzdem ohne größeren körperlichen Aufwand des Bedieners bewerkstelligt werden kann.

Die Parkposition ist weiterhin vorteilhaft so gestaltet, dass der Applikator-Kopf soweit zugänglich ist, dass eine Patienten-Schnittstelle zur Fixierung der Lage eines Auges zum System für die Kurzpuls-Laser-Augenchirurgie an das System für die Kurzpuls-Laser-Augenchirurgie ohne räumliche Behinderungen angekoppelt werden kann.

Bevorzugt ist im System für die Kurzpuls-Laser-Augenchirurgie zum Lösen oder Verbinden der Schnittstelle zwischen Applikator-Kopf und Mikroskop-Kopf ein von einem Bediener zu schaltender oder ein automatisch zu schaltender Mechanismus vorgesehen. In einer speziellen Ausführung erfolgt das Verbinden der Schnittstelle zwischen Applikator-Kopf und Mikroskop-Kopf mittels Bajonett-Verschluss.

Weiterhin sind bevorzugt am ersten Gelenkarm und/oder am zweiten Gelenkarm und/oder am Applikator-Kopf und/oder am Mikroskop-Kopf des Systems für die Kurzpuls-Laser-Augenchirurgie verstellbare Elemente vorgesehen, die eine von der Steuereinheit gesteuerte Bewegung des Mikroskop-Kopfes und/oder des Applikator-Kopfes ermöglichen. Solche verstellbaren Elemente können durch Motoren und/oder andere Antriebselemente realisiert sein.

Auch ist es vorteilhaft, wenn das Strahlführungsmittel, das den zweiten Gelenkarm des Systems zur Durchführung einer Kurzpuls-Laser-Augenchirurgie durchläuft, eine photonische Kristallfaser mit hohlem Kern aufweist. Eine solche photonische Kristallfaser genügt besonders gut der Bedingung, dass das Strahlführungsmittel alle Bewegungen des zweiten Gelenkarms mitvollziehen und in jeder Position des zweiten Gelenkarms die Kurzpuls-Laserstrahlung zu ihrem Austrittsort am Applikator-Kopf in gleicher Qualität führen soll.

Unter den Kurzpuls-Laserquellen sind in der Augenchirurgie die Femtosekunden(fs)-Laserquellen die mit Abstand am häufigsten eingesetzten Laserquellen. Sie haben sich als besonders geeignet und gut beherrschbar für diese Anwendungen erwiesen. Deshalb ist es von Vorteil, auch für ein System zur Kurzpuls-Laser-Augenchirurgie solch eine Femtosekunden-Laserquelle zu verwenden.

Bevorzugt enthält das System für die Kurzpuls-Laser-Augenchirurgie einen konfokalen Detektor. Durch Aufnahme eines A-Scans - also eines eindimensionalen Scans entlang der optischen Achse - und/oder eines B-Scans - eines zweidimensionalen Scans entlang der optischen Achse und senkrecht dazu - zweier Strukturen eines Auges mittels eines OCT-Moduls sowie eines Intensitätsprofils des Signals des konfokalen Detektors beim Durchfahren der z-Fokuslage der Kurzpuls-Laserstrahlung durch die beiden Strukturen kann ein Offset und ein Skalierungsfaktor zwischen den OCT-Signalen und dem Intensitätsprofil ermittelt werden. Dies erlaubt es in Folge, die Fokusposition der Kurzpuls-Laserstrahlung unter Verwendung von OCT-Signalen, insbesondere von OCT-Bildern, zu steuern.

Vorzugsweise beträgt die Kohärenzlänge der OCT-Lichtquelle in Luft mehr als 45mm, besonders bevorzugt mehr als 60 mm. Dadurch wird es möglich, dass der gesamte Vorderkammerabschnitt eines Auges innerhalb eines A-Scans erfasst wird, ohne dass die optische Weglänge des Referenzstrahlenganges angepasst werden muss, selbst dann, wenn sich durch eine laterale Objektivbewegung der optische Weg zum Auge ändert.

Die oben genannte Aufgabe wird weiterhin gelöst durch ein (nicht beanspruchtes) Verfahren zur Positionierung eines Applikator-Kopfes und eines Mikroskop-Kopfes in einem oben beschriebenen System für die Kurzpuls-Laser-Augenchirurgie. Eine solches Verfahren enthält die folgenden Schritte:
(a) Applikator-Kopf und Mikroskop-Kopf werden zunächst zu einer Applikator-Mikroskop-Kopf-Einheit zusammengeführt und miteinander verbunden.
(b) Die Applikator-Mikroskop-Kopf-Einheit wird über einem zu operierenden Auge durch freie Bewegung der Applikator-Mikroskop-Kopf-Einheit in einem dreidimensionalen Volumen positioniert.
(c) Die Applikator-Mikroskop-Kopf-Einheit wird abgesenkt, bis der Applikator-Kopf in einer vorab definierten Position über dem Auge steht und/oder eine am Applikator-Kopf lösbar angebrachte Patienten-Schnittstelle Kontakt mit dem Auge hat. Eine solche Patienten-Schnittstelle, die auch "Patienteninterface" genannt wird, kann insbesondere durch ein Kontaktglas realisiert werden, wobei ein Kontaktglas hier als Überbegriff für verschiedene Ausführungen wie ein Flüssigkeits-Interface oder ein Gel-Interface oder ein Festkörper-Interface verstanden werden soll. Die unten beschriebene Patienten-Schnittstelle kann hier zum Einsatz kommen. Hat in diesem Schritt die Patienten-Schnittstelle Kontakt mit dem Auge, so wird hierbei in der Regel eine wiederlösbare, feste Verbindung zwischen dem Auge und der Patienten-Schnittstelle mittels eines Vakuums, also durch Ansaugen des Auges an die Patienten-Schnittstelle, hergestellt.
(d) Die Linse des Auges und/oder der Kapselsack, und/oder die Cornea wird mittels eines Fokus einer Kurzpuls-Laserstrahlung, also beispielsweise eines Femtosekunden(fs)-Lasers, bearbeitet.
(e) Nach der Behandlung wird die Applikator-Mikroskop-Kopf-Einheit wieder angehoben.
(f) Der Applikator-Kopf der Applikator-Mikroskop-Kopf-Einheit wird in die Parkposition am Gehäuse gebracht. In einer bevorzugten Ausführung rastet der Applikator-Kopf und/oder der zweite Gelenkarm in dieser Parkposition ein und wird mechanisch festgehalten.
(g) Der Mikroskop-Kopf wird vom Applikator-Kopf getrennt. Bevorzugt erfolgt dies durch ein automatisches Lösen nach korrektem Positionieren in Parkposition.
(h) Der Mikroskop-Kopf wird über dem Auge des Patienten positioniert.
(i) Weitere Operationsschritte wie z.B. die Phacoemulsifikation und/oder die Absaugens der verflüssigten Linse und/oder das Einsetzens der intraokularen Linse (IOL) werden durchgeführt.
(j) Der Mikroskop-Kopf wird in eine Position außerhalb des Operationsfeldes verbracht oder aber zum Applikator-Kopf geführt und mit ihm verbunden.

Eine solches Verfahren ist insbesondere in der Kataraktchirurgie mittels Kurzpuls-Laserstrahlung (LCS) von Bedeutung, da hier in einem Teil der chirurgischen Arbeitsschritte der Fokus dieser Laserstrahlung über den Applikator-Kopf in ein zu bearbeitendes Gewebe im Auge gerichtet werden soll, gleichzeitig aber auch eine Beobachtungsmöglichkeit mittels eines Operations-Mikroskops gewünscht wird, und in einem weiteren Teil der chirurgischen Arbeitsschritte nur das Operations-Mikroskop allein benötigt, gleichzeitig jedoch eine Arbeitsfreiheit über dem zu behandelnden Auge gewünscht wird.

Die oben genannte Aufgabe wird auch gelöst durch ein Kurzpuls-Lasersystem für die Augenchirurgie umfassend eine Kurzpuls-Laserquelle, die eine Kurzpuls-Laserstrahlung erzeugt. Insbesondere kann an dieser Stelle eine Femtosekunden (fs)-Laserquelle genutzt werden.

Das Kurzpuls-Lasersystem umfasst weiterhin ein die Divergenz der Kurzpuls-Laserstrahlung, die durch die Kurzpuls-Laserquelle erzeugt wird, variierendes Linsensystem sowie ein x/y-Scansystem für die Kurzpuls-Laserstrahlung.

Das die Divergenz der Kurzpuls-Laserstrahlung variierende Linsensystem enthält dabei mindestens eine Linse, vorteilhalft jedoch ein System verschiedener im Strahlengang der Kurzpuls-Laserstrahlung hintereinander folgender Linsen, von denen mindestens eine in ihrer Position einstellbar ist. Dies Anordnung bietet eine Möglichkeit, die Kurzpuls-Laserstrahlung zu fokussieren und den Fokus der Kurzpuls-Laserstrahlung in z-Richtung, d.h., entlang der optischen Achse zu verschieben. Durch das die Divergenz der Kurzpuls-Laserstrahlung variierende Linsensystem wird somit auch eine z-Position der Kurzpuls-Laserstrahlung in einem zu behandelnden Auge festgelegt.

Das Kurzpuls-Lasersystem umfasst schließlich einen Gelenkarm und ein in x- und y-Richtung bewegliches Objektiv, wobei der Gelenkarm Strahlführungsmittel enthält. Das Strahlführungsmittel sorgt dafür, dass die von der Kurzpuls-Laserquelle emittierte Kurzpuls-Laserstrahlung in einer vorgesehenen Art und Weise zu einem Austrittsort der Kurzpuls-Laserstrahlung aus dem System, hier das in x und y-Richtung bewegliche Objektiv, geleitet wird . Das Strahlführungsmittel kann auch durch eine Gesamtheit verschiedener Komponenten realisiert sein.

Der Gelenkarm mit mindestens zwei Gelenken, die jeweils die Funktion eines Kugelgelenks erfüllen und mindestens zwei Gelenkglieder enthalten, ist beweglich. Bevorzugt ist er frei im dreidimensionalen Raum beweglich. Das Objektiv ist in einer Ausgestaltung auch in z-Richtung verstellbar.

In verschiedenen Ausgestaltungen des Kurzpuls-Lasersystems für die Augenchirurgie enthält das x/y-Scansystem einen x/y-Spiegel-Scanner oder einen kardanisch aufgehängten Spiegel-Scanner oder einen Spiegel-Scanner für die x-Richtung mit nachgeschaltetem Element zur Rotationsdrehung um die optische Achse. Ein x/y-Spiegel-Scanner kann dabei auch einen separaten x-Scanner und y-Scanner umfassen, die gemeinsam als x/y-Scansystem wirken.

In vorteilhafter Weise können auch mehrere Scanner für jeweils eine Richtung vorgesehen sein, beispielsweise ein Scanner für eine langsame Bewegung über einen größeren Bereich und ein Scanner für eine sehr schnelle Bewegung über einen kleinen Bereich. Insbesondere von Interesse ist dies in z-Richtung, also entlang der optischen Achse, bevorzugt in Kombination mit mindestens einer lateralen Richtung x oder y.

Weiterhin kann ein Kurzpuls-Lasersystem für die Augenchirurgie eine Patienten-Schnittstelle enthalten, die zur Fixierung der Lage eines Auges zu einem System für die Kurzpuls-Laser-Augenchirurgie genutzt wird. In einer Ausgestaltung umfasst die Patienten-Schnittstelle ein Kontaktglas, wobei ein Kontaktglas hier als Überbegriff für verschiedene Ausführungen wie ein Flüssigkeits-Interface oder ein Gel-Interface oder ein Festkörper-Interface verstanden werden soll. Insbesondere kann das Kurzpuls-Lasersystem ein im folgenden beschriebenes Kontaktglas enthalten.

Das Bildfeld des beweglichen Objektivs des Kurzpuls-Lasersystem für die Augenchirurgie ist im Querschnitt bevorzugt größer als 1,0mm aber kleiner als 6,0mm, besonders bevorzugt größer als 1,5mm aber kleiner als 3,0mm. Das Bildfeld befindet sich dabei in einer Bildfeldebene, in der es durch eine Scanbewegung in x- und/oder y-Richtung bewegt werden kann. Auch die Bildfeldebene selbst kann entlang der optischen Achse durch eine Scanbewegung in z-Richtung bewegt werden. Der Querschnitt des beweglichen Objektivs richtet sich insbesondere nach dem Scanbereich des x/y-Scansystems.. Somit lässt sich der Fokus der Kurzpuls-Laserstrahlung an jedem Ort des dreidimensionalen Scanvolumens durch Überlagerungen der Strahlablenkungen vom beweglichen Objektiv und von den Spiegel-Scannern gezielt ablegen.

Die Optik, die im Strahlengang der Kurzpuls-Laserstrahlung angeordnet ist, sowie das die Divergenz der Kurzpuls-Laserstrahlung variierende Linsensystem sind vorzugsweise auf einer Optikbank befestigt. Die Optikbank selbst ist mit drei Punkten auf, an oder innerhalb eines Gehäuses befestigt, an dem vorzugsweise auch der Gelenkarm angeordnet ist. Alle Deformationen der Befestigungsfläche im Gehäuse haben damit keinen Einfluss auf den Justierzustand der Optik auf der Optikbank, aber auf die Position der Optikbank zum Eintritt in den Gelenkarm mit seinem Strahlführungsmittel. Änderungen dieser Position können mit einer Strahlstabilisierung ausgeglichen werden.

Eine vorteilhafte Ausgestaltung des Kurzpuls-Lasersystems für die Augenchirurgie umfasst weiterhin ein System zur Stabilisierung eines Strahldurchgangs durch den Gelenkarm, das eine Lichtquelle an einem Ende des Gelenkarms und einen positionsempfindlichen Lichtsensor am anderen Ende des Gelenkarms enthält.

Dabei erfolgt die Lichteinkopplung unter einem Winkel zur optischen Achse des Gelenkarmes, d.h., dass beispielsweise die Lichtquelle nicht auf der optischen Achse sitzt oder aber, dass die Lichtquelle auf der optischen Achse sitzt, aber in Richtung der optischen Achse nicht symmetrisch abstrahlt.

Diese Strahlstabilisierung erlaubt es, trotz verschiedenen Stellungen des Gelenkarmes, die Ablenkung der Kurzpuls-Laserstrahlung in x- und/oder y-Richtung durch das x/y-Ablenkungssystem bzw. das x/y Scansystem zur Positionierung des Fokus vorzugsweise durch eine x/y-Positionierung eines beweglichen Objektivs richtungsgenau zu positionieren und mechanische Toleranzen des Gelenkarms und der Ausrichtung des x/y-Scansystems auszugleichen.

Die o.g. Aufgabe wird weiterhin gelöst durch ein Verfahren zur Schnittführung, insbesondere in kornealem Augengewebe, mittels eines Kurzpuls-Lasersystems für die Augenchirurgie, insbesondere eines Kurzpuls-Lasersystems wie oben beschrieben, das die folgenden Schritte enthält:
(a) Ein Objektiv des Kurzpuls-Lasersystems wird in x und y-Richtung, also senkrecht zur optischen Achse, derart positioniert, dass mindestens ein Teil der in eine Bildfeldebene zu projizierenden x- und y-Fokuslagen eines Schnittmusters innerhalb des Bildfeldes des positionierten Objektivs angeordnet sind. Dabei wird durch die Fokuslage in x-, y- und z-Richtung die jeweilige Position des Fokus einer Kurzpuls-Laserstrahlung des Kurzpuls-Lasersystems in einem dreidimensionalen Bearbeitungsvolumen, in dem das Auge eines Patienten behandelt werden kann, bestimmt.
(b) Die Fokuslagen des Schnittmusters werden, vorzugsweise durch einen Puls der Kurzpuls-Laserstrahlung, durch das in seiner x- und y-Lage feststehende Objektiv unter Verwendung eines x/y-Scansystems und unter nach jedem x/y-Scan erfolgender oder parallel zu dem x/y-Scan erfolgender Verstellung der Fokuslage in z-Richtung entlang der optischen Achse mittels eines die Divergenz variierenden Linsensystems und/oder des Objektivs abgebildet. Ein x/y-Scan ist dabei eine Bewegung des Fokus der Kurzpuls-Laserstrahlung in einer lateralen x/y-Ebene. Die Energie eines Pulses der Kurzpuls-Laserstrahlung in seinem Fokuspunkt wird so gewählt, dass eine plasmainduzierte Photoablation oder Photodisruption des Gewebes bewirkt wird. Durch eine entsprechende Wahl des Abstands der abzubildenden Fokuslagen wird ein Schnitt im Gewebe dadurch erzeugt, dass sich Wirkbereiche der jeweiligen Pulse einer Fokuslage und einer benachbarten Fokuslage mindestens berühren, ggf. teilweise überschneiden.
(c) Die Schritte (a) und (b) werden für ein weiteres Bildfeld wiederholt, für das das Objektiv an anderer lateraler Position erneut positioniert wird, solange bis das gesamte Schnittmuster erzeugt wurde. Das gesamte Schnittmuster wurde erzeugt, wenn alle Fokuslagen des Schnittmusters abgebildet wurden.

Insbesondere wird die oben genannte Aufgabe gelöst durch ein Verfahren zur Schnittführung für die Zertrümmerung einer Augenlinse mittels eines Kurzpuls-Lasersystems für die Augenchirurgie, vorzugsweise eines Kurzpuls-Lasersystems wie oben beschrieben, das die folgenden Schritte enthält:
(a) Der Fokuspunkt einer Kurzpuls-Laserstrahlung wird in ein zu bearbeitendes Gewebe der Augenlinse positioniert, wobei durch eine entsprechende Energie im Fokuspunkt der Kurzpuls-Laserstrahlung eine plasmainduzierte Photoablation oder Photodisruption des Gewebes der Augenlinse bewirkt wird.
(b) Ein Objektiv des Kurzpuls-Lasersystems erfährt einen Vorschub in lateraler Richtung in einer ersten Meridianebene der Augenlinse um eine Länge (L) mit einer Überlagerung einer oszillierenden Verschiebung des Fokuspunktes, die eine Hauptkomponente entlang der optischen Achse aufweist, mit einer Amplitude (A) zum Positionieren weiterer Fokuspunkte der Kurzpuls-Laserstrahlung in einer Teilfläche der Meridianebene. Die oszillierende Verschiebung der Fokuskomponente kann dabei auch allein entlang der optischen Achse verlaufen, also nur eine Komponente entlang der optischen Achse aufweisen. Unter einer Meridianebene wird eine beliebige parallel zur optischen Achse verlaufende Ebene verstanden.
(c) Der Fokuspunkt der Kurzpuls-Laserstrahlung wird entlang der optischen Achse um eine Höhe (H) verschoben, wobei die Höhe (H) so gewählt wird, dass sich bei einer Wiederholung des Schrittes (b) neu positionierte Fokuspunkte der Kurzpuls-Laserstrahlung nicht mit einem bislang positionierten Fokuspunkt überlappen.
(d) Die Schritte (b) und (c) werden wiederum wiederholt, wobei der Schritt (b) wie auch der Schritt (c) unter Umkehrung der Vorschubrichtung in lateraler Richtung wie auch entlang der optischen Achse erfolgen kann, bis in der gesamten ersten Medianebene Fokuspunkte positioniert wurden.
(e) Die Schritte (b) bis (d) werden wiederholt zur Positionierung von Fokuspunkten in einer weiteren Meridianebene der Augenlinse, bis die Augenlinse derart mit Fokuspunkten der Kurzpuls-Laserstrahlung durchsetzt ist, dass die dann durch plasmainduzierte Photoablation oder Photodisruption entstandenen Teilstücke eine Maximalgröße nicht überschreiten. Die Maximalgröße wird dabei bestimmt durch die Absaugmöglichkeiten und die Größe der Kapsulorhexis bzw. Kapsulotomie.

Bevorzugt werden in dem Verfahren zur Schnittführung für die Zertrümmerung der Augenlinse Fokuspunkte der Kurzpuls-Laserstrahlung nur bei der Bewegung des Fokus der Kurzpuls-Laserstrahlung von der posterioren zur anterioren Seite der Augenlinse positioniert, wobei wiederum bevorzugt die Bewegung von der von der anterioren zur posterioren Seite der Linse schneller als die Bewegung von der posterioren zur anterioren Seite der Linse durchgeführt wird.

Alternativ können aber auch über den ganzen Oszillations-Zyklus Fokuspunkte der Kurzpuls-Laserstrahlung positioniert werden.

Weiterhin bevorzugt wird beim Positionieren von Fokuspunkten der Kurzpuls-Laserstrahlung verschiedener Medianebenen wie auch verschiedener Teilflächen einer Meridianebene ein Abstand von 10-50µm eingehalten.

Auch findet bevorzugt der Wechsel der Meridianebenen im Bereich der Schnittlinie der Meridianebenen statt.

Die oben genannte Aufgabe wird des Weiteren gelöst durch ein Verfahren zur Schnittführung einer Kapsulotomie mittels eines Kurzpuls-Lasersystems für die Augenchirurgie, bei dem eine Öffnung, insbesondere des Kapselsacks, dadurch erzeugt wird, dass Fokuspunkte einer Kurzpuls-Laserstrahlung mittels eines x/y-Scansystems so in ihren x- und y-Fokuslagen positioniert werden, dass in n Schritten von 1 bis N, wobei N eine natürliche Zahl größer oder gleich 2 ist, jeweils eine n-te nicht geschlossene Kurve mit einem Radius R und mit ersten und einem zweiten Endbereich einer jeweils gleichgerichteten Wölbung wie die der nicht geschlossenen Kurve entsteht. Der erste Endbereich der n-ten nicht geschlossenen Kurve weist einen ersten Endbereichsradius R_{En1} und der zweite Endbereich einen zweiten Endbereichsradius R_{En2} auf. Dabei ist sowohl der erste Endbereichsradius R_{En1} als auch der zweiten Endbereichsradius R_{En2} kleiner als der Radius R.

Des Weiteren weisen alle Endbereiche der nicht geschlossene Kurven je ein Ende auf. Die nicht geschlossenen Kurven sind dabei so zueinander angeordnet, dass für n von 2 bis N der erste Endbereich der n-ten nicht geschlossenen Kurve den zweiten Endbereich der (n-1)-ten nicht geschlossenen Kurve, sowie zusätzlich der zweite Endbereich der N-ten nicht geschlossenen Kurve (d.h. n gleich N) den ersten Endbereich der ersten nicht geschlossenen Kurve so schneidet, dass die Enden aller Endbereiche im Inneren einer durch die erste bis n-ten nichtgeschlossenen Kurve gebildeten geschlossenen Kurve angeordnet sind.

Damit ein entsprechender Schnitt entsteht, wird die Energie eines Pulses der Kurzpuls-Laserstrahlung in seinem Fokuspunkt so gewählt, dass durch plasmainduzierte Photoablation oder Photodisruption eine Trennung des Gewebes eines Auges ermöglicht wird. Des Weiteren wird der Abstand der Fokuslagen so gewählt, dass sich die Wirkbereiche, die sog. Kavitationsblasen, der jeweiligen Pulse einer Fokuslage und einer benachbarten Fokuslage mindestens berühren, ggf. teilweise überschneiden und so durch plasmainduzierte Photoablation oder Photodisruption eine Trennung des Gewebes ermöglichen, bei der ein Schnitt entsteht.

Vorteilhaft ist dabei eine Überlagerung einer oszillierenden Bewegung des Fokuspunktes entlang der optischen Achse mit einer Amplitude (A), so dass auch Variationen in z-Richtung, beispielsweise der Lage des Kapselsacks, ausgeglichen werden können.

Bezüglich des Radius R sind kleinere Abweichungen zwischen den Radien R der n nicht geschlossenen Kurven möglich.

Um mit Hilfe von N nicht geschlossenen Kurven eine geschlossene Kurve zu erzeugen, werden diese N nicht geschlossenen Kurven, die jeweils einen ersten und einen zweiten Endbereich aufweisen, so zueinander angeordnet, dass der erste Endbereich der n-ten nicht geschlossenen Kurve den zweiten Endbereich der (n-1)-ten nicht geschlossenen Kurve schneidet und schließlich der zweite Endbereich der N-ten nichtgeschlossenen Kurve den ersten Endbereich der ersten nicht geschlossenen Kurve schneidet, also vorzugsweise ab der zweiten nichtgeschlossenen Kurve jeweils der erste Endbereich der zu erzeugenden nichtgeschlossenen Kurve über den zweiten Endbereich der gerade erzeugten nichtgeschlossenen Kurve verlaufend erzeugt wird.

Es kann jedoch auch von einer solchen Reihenfolge des Abarbeitens benachbarter nichtgeschlossener Kurven abgesehen werden und N nichtgeschlossene Kurven so erzeugt werden, dass letztlich nach dem Erzeugen der letzten nichtgeschlossenen Kurve ein solches Muster entstanden ist, wie hier beschrieben und in Summe eine geschlossene Kurve vorliegt.

Zur Lösung der oben genannten Aufgabe trägt auch eine Patienten-Schnittstelle zur Fixierung der Lage eines Auges zu einem System für die Kurzpuls-Laser-Augenchirurgie bei. Eine solche Patienten-Schnittstelle umfasst ein Kontaktglas, kann aber auch als ein Flüssigkeits-Interface ausgeführt sein.

Die Patienten-Schnittstelle ist einteilig und aus einem transparenten oder einem teiltransparenten Material gefertigt. Es enthält einen Saugring, einen Mantel und ein optisches Element an der Oberseite des Mantels, wobei die Oberseite des Mantels die dem Saugring abgewandte Seite des darstellt.

Der Saugring ist an der dem Auge zugewandten Seite der Patienten-Schnittstelle angeordnet und dient der formschlüssigen Auflage und Fixierung der Patienten-Schnittstelle auf dem Auge eines Patienten.

Der Mantel ist bevorzugt konisch in der Form eines Kegelstumpfes ausgebildet. Der untere, dem Auge zugewandte Durchmesser, der optisch genutzt werden kann, sollte mindestens 10mm, bevorzugt größer als 13mm und besonders bevorzugt größer als 14mm sein.

Der Mantel weist seitlich mindestens eine Öffnung, vorzugsweise zwei Öffnungen auf, an die jeweils eine Zuleitung über eine Fixierungshilfe angeschlossen ist, oder die jeweils den Anschluss einer Zuleitung erlaubt.

Die Zuleitung bzw. eine der Zuleitungen erlaubt die Erzeugung eines Unterdrucks in dem Saugring, wofür die entsprechende Öffnung nicht die gesamte Dicke des Mantels durchdringen muss, sondern eine Verbindung zum Saugring wesentlich ist. Eine weitere Zuleitung kann das Zuführen von Flüssigkeit in die Patienten-Schnittstelle erlauben, wofür die entsprechende Öffnung eine gesamte Dicke des Mantels durchdringt. Das Zuführen von Flüssigkeit in die Patienten-Schnittstelle erfolgt dabei vorzugsweise so, dass in einem Zustand, in dem die Patienten-Schnittstelle auf einem Auge eines Patienten aufliegt, das gesamte vom Auge, Mantel und optischen Element begrenzte Volumen mit der Flüssigkeit befüllt ist und das optische Element auf seiner dem Auge zugewandten Seite in diese Flüssigkeit eingetaucht ist.

In einer Ausführung der Patienten-Schnittstelle ist im Kontaktglas eine weitere Saugstruktur aus dem transparenten Material an der dem Saugring abgewandten Seite des Mantels, und damit an der dem Auge abgewandten Seite des Mantels, vorgesehen. Diese weitere Saugstruktur dient dem Halten des Kontaktglas an einem Applikator-Kopf eines Systems für die Kurzpuls-Laser-Augenchirurgie, beispielsweise wie oben beschrieben, mittels eines Unterdrucks.

In einer besonderen Ausführungsform der Patienten-Schnittstelle ist das optische Element, geneigt zur optischen Achse angeordnet. Geneigt angeordnet bedeutet dabei nicht senkrecht zur optischen Achse angeordnet. Dabei ist es möglich, dass das gesamte optische Element oder aber auch nur die Oberfläche des optischen Elements, die dem Auge abgewandt ist, geneigt zur optischen Achse angeordnet ist.

Auch ist es vorteilhaft, wenn die dem Auge zugewandten Oberfläche des optischen Elements, das im Kontaktglas der Patienten-Schnittstelle enthalten ist, hydrophil beschichtet oder hydrophil oberflächenbehandelt ist. Weiterhin ist es von Vorteil, wenn die dem Auge zugewandte Oberfläche des optischen Elements konvex gekrümmt ist. Dies dient der Verbesserung der Benetzung mit einer Flüssigkeit, und damit insbesondere der Verhinderung von Blasenbildung im Bereich des optischen Elements. Durch die konvexe Form der Oberfläche des optischen Elements wandern ggf. gebildete Blasen an den äußeren Rand des optischen Elements, der für die optische Abbildung des Systems irrelevant ist.

Weiterhin ist es vorteilhaft, wenn die dem Auge abgewandte Oberfläche des optischen Elements, das im Kontaktglas der Patienten-Schnittstelle enthalten ist antireflektionsbeschichtet ist. Dies dient der Verhinderung der Reflexion der einfallenden Laserstrahlung.

Insbesondere ist es von Vorteil, wenn die Patienten-Schnittstelle des Weiteren einen Applikator-Kopf-Schutz umfasst. Mit diesem kann der dem Auge zugewandte Teil eines Applikator-Kopfs - mit Ausnahme der Optik - beim Andocken der Patienten-Schnittstelle an einen Applikator-Kopf eines Systems für die Kurzpuls-Laser-Augenchirurgie bedeckt werden und damit die Sterilität unterstützt werden.

Günstig ist eine Patienten-Schnittstelle mit einem Applikator-Kopf-Schutz, der eine Aussparung aufweist, die vorzugsweise mittig im Applikator-Kopf-Schutz realisiert ist, und die weiterhin vorzugsweise kleiner als ein oberer Manteldurchmesser des Mantels ist. Diese Aussparung dient der Durchführung der Optik des Applikator-Kopfes und seiner Kopplung an das optische Element des Kontaktglases.

Von Vorteil ist es, wenn in einer Patienten-Schnittstelle, die einen Applikator-Kopf-Schutz enthält, Kontaktglas und Applikator-Kopf-Schutz zwei getrennte oder trennbare Teile sind.

In einer vorteilhaften Ausgestaltung weist der Applikator-Kopf-Schutz der Patienten-Schnittstelle ein mechanisches Koppelelement auf. Das mechanische Koppelelement ist eingerichtet, den Applikator-Kopf-Schutz mit einem Applikator-Kopf eines Systems für die Kurzpuls-Laser-Augenchirurgie lösbar zu verbinden.

Auch ist es von Vorteil, wenn die Patienten-Schnittstelle, insbesondere der Mantel des Kontaktglases der Patienten-Schnittstelle, weiterhin eine lichtleitende Struktur enthält. Die lichtleitende Struktur dient zur Beleuchtung durch eine zusätzliche Lichtquelle, die an die lichtleitende Struktur anschließbar ist, beispielsweise sichtbares Licht mit Wellenlängen zwischen 350nm und 780nm oder vorzugsweise Licht im Infrarot-Bereich vorzugsweise mit Wellenlängen zwischen 781nm bis 1300nm zur Schonung des Auges.

Auch ist es günstig, wenn das Kontaktglas der Patienten-Schnittstelle mindestens eine Markierung enthält. Diese ist vorzugsweise in einem unteren, also dem Auge nahen, Mantelbereich des Mantels angeordnet und dient zur Orientierung und Ausrichtung.

Weiterhin unterstützt eine optische Einheit aus einem Kurzpuls-Lasersystem und einer Patienten-Schnittstelle die Lösung der oben genannte Aufgabe. In dieser optischen Einheit aus einem Kurzpuls-Lasersystem und einer Patienten-Schnittstelle, die ein Kontaktglas mit einem optischen Element enthält, ist die Tiefenschärfe einer Abbildung in miteinander gekoppeltem Zustand wie auch im direkt hintereinander angeordnetem, nicht miteinander gekoppelten Zustand der optischen Einheit aus Kurzpuls-Lasersystem und Patienten-Schnittstelle mindestens 3 mm, vorzugsweise größer als 5mm. Bevorzugt ist das Kurzpuls-Lasersystem ein oben beschriebenes, Kurzpuls-Lasersystem und die Patienten-Schnittstelle eine oben beschriebene Patienten-Schnittstelle. Aufgrund der Tiefenschärfe können sowohl Referenzstrukturen des Auges als auch die Markierung des Kontaktglases der Patienten-Schnittstelle auch im abgedockten Zustand scharf erfasst werden, beispielsweise mit einer Kamera. Im angedockten Zustand des Auges lassen sich aufgrund der Tiefenschärfe sowohl Referenzstrukturen des Auges als auch mittels Kurzpuls-Lasersystem durchgeführte Schnitte im Auge von dem Bediener, insbesondere einem Arzt, gut erkennen.

Die Lösung der oben genannten Aufgabe wird weiterhin unterstützt durch ein Referenzierungsverfahren für Relaxations- und/oder Zugangsschnitte für ein System zur Kurzpuls-Laser-Augenchirurgie, das die folgenden Schritte umfasst:
a) Ein Bild des Auges mit Referenzstrukturen wird durch eine Kamera im nicht an das Patientenauge angedockten Zustand einer Patienten-Schnittstelle, die vorzugsweise ein Kontaktglas enthält, aufgenommen.
b) Die Patienten-Schnittstelle wird innerhalb weniger Sekunden an das Auge angedockt.

Die Ausrichtung der Relaxations- und/oder Zugangsschnitte wird mittels Erkennungsalgorithmen basierend auf den Referenzstrukturen durchgeführt und dem Bediener, insbesondere einem Arzt, als Information oder Therapieplanungsvorschlag zur Verfügung gestellt.

Die Lösung der oben genannten Aufgabe wird auch unterstützt durch ein Referenzierungverfahren für die Orientierung einer torischen Intraokularlinse bei ihrer Einsetzung in ein Auge, insbesondere in den Kapselsack des Auges nach Fragmentierung und Entfernung einer natürlichen Augenlinse, das folgende Schritte enthält:
a) Ein erstes Bild eines astigmatischen Auges wird mittels Diagnosesystem zur Erkennung der steilen und/oder flachen Achse aufgenommen. Die Orientierung der steilen und/oder flachen Achse wird zusammen mit dem Bild gespeichert oder ist dem Bild zugeordnet. Bei dieser Aufnahme befindet sich der Patient meist in einer sitzenden Position.
b) Ein zweites Bild des gleichen astigmatischen Auges wird mit am Auge angedockter, also gekoppelter, Patienten-Schnittstelle oder mit nicht angedockter Patientenschnittstelle, die jedoch danach angedockt wird, und mit Hilfe des hier beschriebenen Systems für die Kurzpuls-Laser-Augenchirurgie generiert und mit dem ersten Bild verglichen. Bei der zweiten Bildaufnahme befindet sich der Patient meist in einer liegenden Position. Durch Referenzierungs-Algorithmen wird die Orientierung der steilen und/oder flachen Achse des astigmatischen Auges aus dem ersten Bild auf das zweite Bild übertragen.
c) Nach der Laserbehandlung und mit wieder abgedockter Patienten-Schnittstelle wird eine weitere Referenzierung zwischen dem zweiten und dem dritten Bild durchgeführt. Bei der dritten Bildaufnahme befindet sich der Patient in einer liegenden Position. Zwischen dem zweiten und dem dritten Bild hat sich die Augenstruktur verändert, beispielsweise durch leichte Blutungen und/oder Rötungen auf den Sklera, wobei die Orientierung der steilen und/oder flachen Achse des astigmatischen Auges gleich geblieben ist.
d) Weitere Bilder und Videoaufnahmen des Auges werden während der Ausrichtung der in das Auge eingesetzten Intraokularlinse aufgenommen und mit der dritten Bildaufnahme referenziert.
e) Die Intraokularlinse wird unter Zuhilfenahme einer in vorangegangenen Schritten etablierten Beziehung orientiert. Dazu wird vorzugsweise eine Orientierungshilfe für den Arzt im Operations-Mikroskop geschaffen.

Schlussendlich wird die oben genannte Aufgabe auch gelöst durch ein Computerprogramm-Produkt zur Kodierung einer Steuereinheit eines Kurzpuls-Lasersystems für die Augenchirurgie zur Ausführung eines oben beschriebenen Verfahrens, wie
- des Verfahrens zur Positionierung eines Applikator-Kopfes
- des Verfahrens zur Schnittführung mittels eines Kurzpuls-Lasersystems für die korneale Augenchirurgie
- des Verfahrens zur Schnittführung für die Zertrümmerung einer Augenlinse mittels eines Kurzpuls-Lasersystems für die Augenchirurgie
- des Verfahrens zur Schnittführung einer Kapsulotomie mittels eines Kurzpuls-Lasersystems für die Augenchirurgie,
- des Referenzierungsverfahrens für Relaxations- und/oder Zugangsschnitte und
- des Referenzierungverfahrens für die Orientierung einer Intraokularlinse.

Die vorliegende Erfindung soll nun anhand von Ausführungsbeispielen erläutert werden. Es zeigt:
- die Fig. 1: ein erstes System für die Kurzpuls-Laser-Augenchirurgie;
- die Fig. 2: ein zweites System für die Kurzpuls-Laser-Augenchirurgie;
- die Fig. 3: die Positionen beim Verbinden von Applikator-Kopf und Mikroskop-Kopf in Draufsicht;
- die Fig. 4: die Transportposition des Gelenkarms mit Applikator-Kopf in Draufsicht;
- die Fig. 5: eine Vorrichtung für einen unabhängigen Gewichtsausgleich eines Gelenkarms;
- die Fig. 6: ein Verfahren zur Positionierung eines Applikator-Kopfes und eines Mikroskop-Kopfes in einem System für die Kurzpuls-Laser-Augenchirurgie;
- die Fig. 7: ein Kurzpuls-Lasersystem für die Augenchirurgie (Strahlerzeugung und Optik);
- die Fig. 8: einen Aufbau für das Zusammenführen von Kurzpuls-Laserstrahlung aus der Kurzpuls-Laserquelle und OCT-Strahlung aus der OCT-Lichtquelle;
- die Fig. 9a und 9b: die Steuerung eines Kurzpuls-Lasersystems für die Augenchirurgie mittels Signalen aus einem konfokalen Detektor und einem OCT-Modul;
- die Fig. 10: die Bewegung des Fokus der Kurzpuls-Laserstrahlung bei lateral scannendem Objektiv eines Kurzpuls-Lasersystems;- die Fig. 11a: die Schnittlagen im Gewebe eines Auges bei kleinem Feld der x/y-Schnittflächenprojektion;
- die Fig. 11b: ein Verfahren zur Fokusverschiebung der Kurzpuls-Laserstrahlung in einem Kurzpuls-Lasersystem für die Augenchirurgie;
- die Fig. 12a: die Schnittlagen in Gewebe eines Auges bei größerem Feld der x/y-Schnittflächenprojektion;
- die Fig. 12b; ein alternatives Verfahren zur Fokusverschiebung der Kurzpuls-Laserstrahlung in einem Kurzpuls-Lasersystem für die Augenchirurgie;
- die Fig. 13a und 13b: die Fokusführung des Fokus einer Kurzpuls-Laserstrahlung bzw. die Schnittführung mittels einer Kurzpuls-Laserstrahlung im Linsengewebe eines Auges;
- die Fig. 14a und 14b: die Fokusführung des Fokus einer Kurzpuls- Laserstrahlung bei zur optischen Achse des Kurzpuls-Lasersystems schräg stehender Augenlinse;
- die Fig. 15: die Fokusführung des Fokus einer Kurzpuls-Laserstrahlung bei zur optischen Achse des Kurzpuls-Lasersystems leicht schräg stehender Augenlinse;
- die Fig. 16: eine x/y-Projektion der Fokuspunkte einer Kurzpuls-Laserstrahlung bei einer ersten Schnittführung eines Kurzpuls-Lasersystems zur Durchführung der Kapsulotomie;
- die Fig. 17a: eine x/y-Projektion der Fokuspunkte einer Kurzpuls-Laserstrahlung bei einer zweiten Schnittführung eines Kurzpuls-Lasersystems zur Durchführung der Kapsulotomie;
- die Fig. 17b: eine x/y-Projektion der Fokuspunkte einer Kurzpuls-Laserstrahlung bei einer dritten Schnittführung eines Kurzpuls-Lasersystems zur Durchführung der Kapsulotomie;
- die Fig. 17c: eine x/y-Projektion der Fokuspunkte einer Kurzpuls-Laserstrahlung bei einer vierten Schnittführung eines Kurzpuls-Lasersystems zur Durchführung der Kapsulotomie;
- die Fig. 18: eine Patienten-Schnittstelle an einem Kurzpuls-Lasersystem für die Augenchirurgie;
- die Fig. 19a: ein erster Aufbau zur Referenzierung von Laserschnitten mit einer Patienten-Schnittstelle an einem Kurzpuls-Lasersystem;
- die Fig. 19b: ein zweiter Aufbau zur Referenzierung von Laserschnitten mit einer Patienten-Schnittstelle an einem Kurzpuls-Lasersystem;
- die Fig. 20: ein Verfahren zur Referenzierung der Schnittführung in einem Kurzpuls-Lasersystem für die Augenchirurgie;
- die Fig. 22: ein Verfahren zur Referenzierung für die Orientierung einer Intraokularlinse-bei Einsetzung in ein Auge;

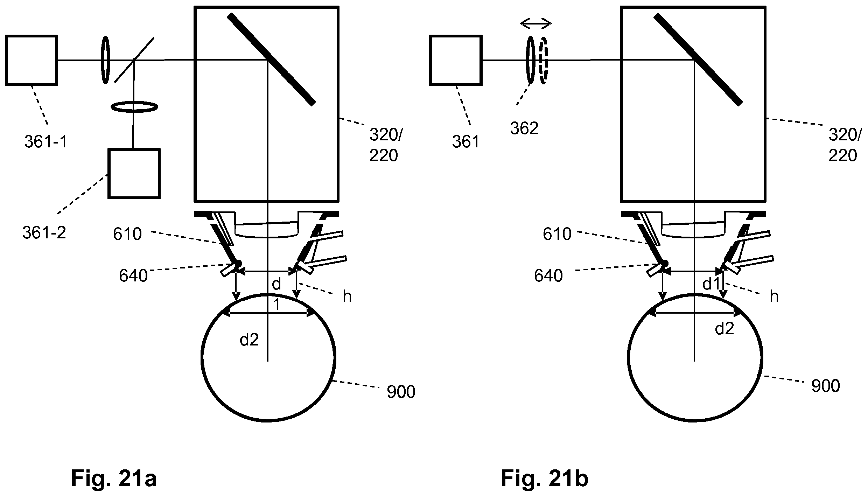

- die Fig. 21a: ein dritter Aufbau zur Referenzierung von Laserschnitten mit einer Patienten-Schnittstelle an einem Kurzpuls-Lasersystem;
- die Fig. 21b: ein vierter Aufbau zur Referenzierung von Laserschnitten mit einer Patienten-Schnittstelle an einem Kurzpuls-Lasersystem;

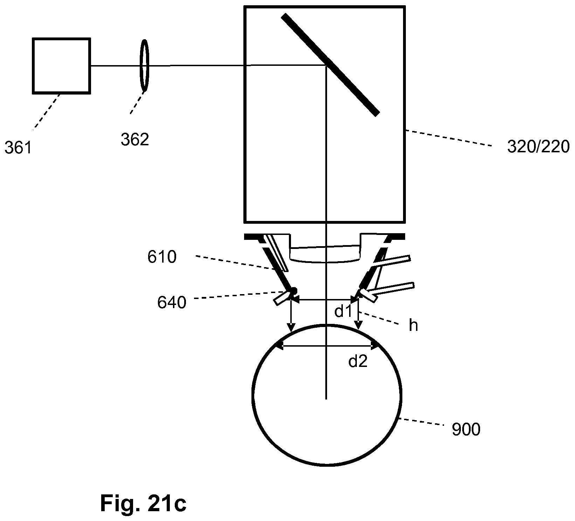

- die Fig. 21c: ein fünfter Aufbau zur Referenzierung von Laserschnitten mit einer Patienten-Schnittstelle an einem Kurzpuls-Lasersystem;

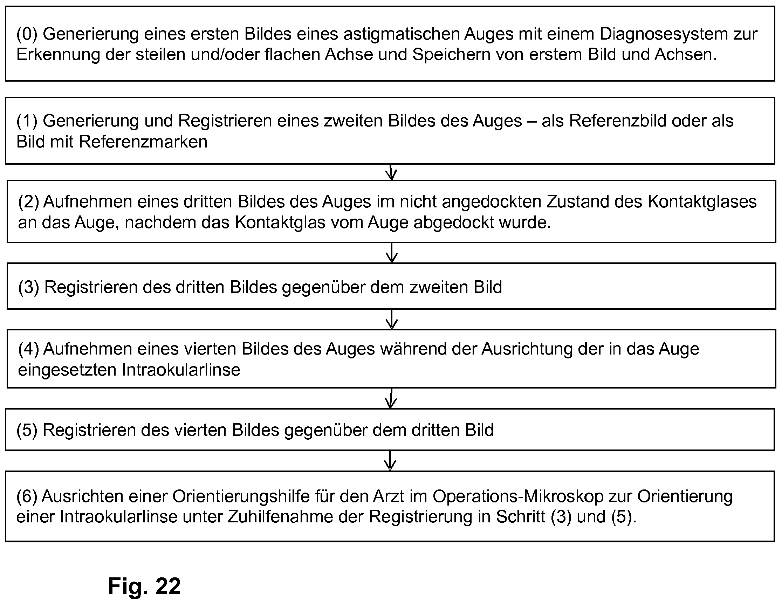

   die Fig. 22: ein Verfahren zur Referenzierung für die Orientierung einer Intraokularlinse bei Einsetzung in ein Auge.

In den folgenden Beispielen für ein System für die Kurzpuls-Laser-Augenchirurgie, für ein Kurzpuls-Lasersystem wie auch für die entsprechenden Verfahren werden Femtosekunden-Laser bzw. fs-Laser als Kurzpuls-Laser eingesetzt, die die am häufigsten im Bereich der Augenchirurgie mittels Laser genutzten Kurzpuls-Laser sind - und damit auch die am besten verstandenen. Nichtsdestotrotz sind alle hier beschriebenen Systeme und Verfahren auch mit anderen Kurzpuls-Lasern umsetzbar. Fs-Laser stehen also, sofern nicht explizit auf die Pulslänge als differenzierendes Merkmal eingegangen wird, als Synonym für Kurz-Pulslaser.

Auch wird im Folgenden von OCT, der Optischen Kohärenztomographie, gesprochen. OCT steht dabei, sofern nicht explizit bzgl. der verschiedenen Varianten von OCT differenziert wird, als Synonym für alle Verfahren, die unter Ausnutzung der optischen Kurzkohärenz Entfernungen im Auge messen oder Bilder vom Auge oder dessen Komponenten erfassen können, wie Time-Domain-Optische Kohärenztomographie (TD-OCT), Spektrometer-basierte Frequenz-Domain-OCT (FD-OCT) oder Wellenlängen-Durchstimmungs-basierte Swept-Source-OCT (SS-OCT).

### Systemauslegung des Gesamtsystems und Workflow

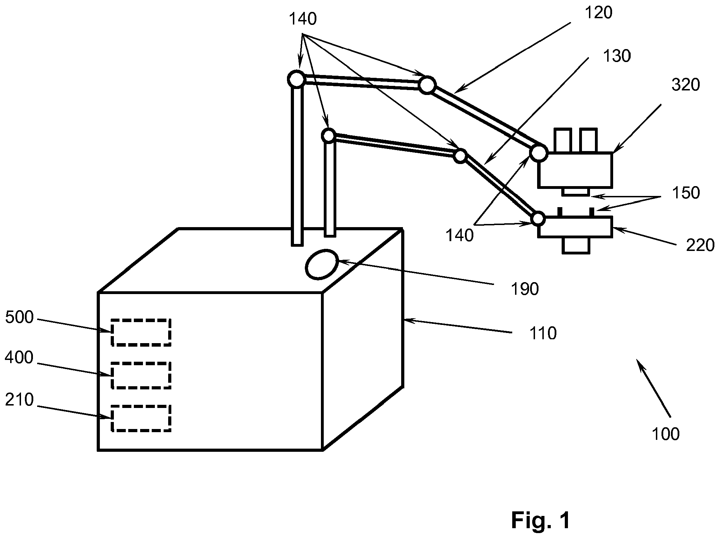

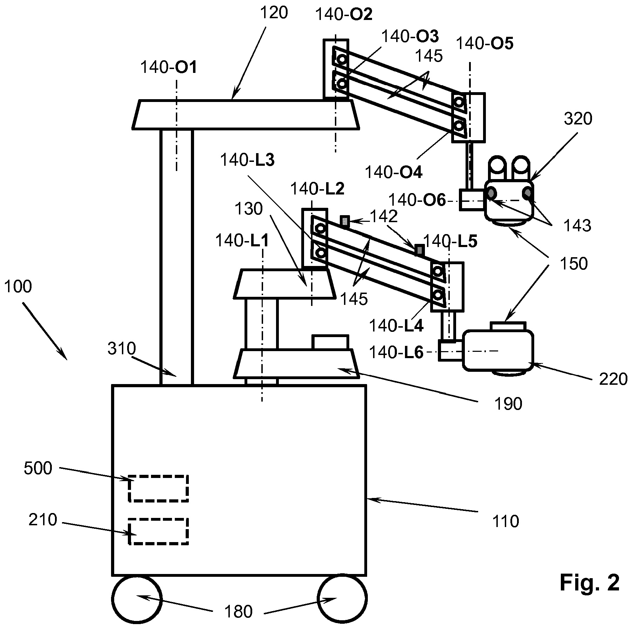

Um die Integration der verschiedenen Komponenten bzgl. eines für den Bediener, also bevorzugt ein Arzt, insbesondere ein Augenchirurg, optimierten Workflows und einer optimierten Arbeitsumgebung zu verbessern, wird in Fig. 1 wie auch in Fig. 2 ein Aufbau eines ersten und eines zweiten Systems für die Kurzpuls-Laser-Augenchirurgie 100 offenbart, das ein fs-Lasersystem als Kurzpuls-Lasersystem 200 mit einer Kurzpuls-Laserquelle 210, hier also eine fs-Laserquelle, einem Strahlführungsmittel 230 und einem Applikator-Kopf 220 zur Leitung der fs-Laserstrahlung auf das zu operierende Auge 900 enthält.

Der Aufbau des ersten und des zweiten Systems für die Kurzpuls-Laser-Augenchirurgie 100 umfasst weiterhin ein Operations-Mikroskop 300 mit einem Operations-Mikroskop-Kopf 320. Dabei ist die gesamte das Operations-Mikroskop und seine Funktion bestimmende Optik im Mikroskop-Kopf 320 angeordnet.

Das erste System für die Kurzpuls-Laser-Augenchirurgie 100 der Fig. 1 umfasst weiterhin OCT-Modul 400, das eine OCT-Lichtquelle 405, ein Interferometer und einen Detektor enthält. Auch das zweite System der Fig. 2 kann prinzipiell ein solches OCT-Modul enthalten. Für das Zusammenwirkung der in der Fig. 1 und Fig. 2 gezeigten Systemkomponenten ist die Anwesenheit eines OCT-Moduls jedoch nicht zwingend erforderlich.

Das erste wie auch das zweite System für die Kurzpuls-Laser-Augenchirurgie der Fig. 1 wie auch der Fig. 2 werden von einem gemeinsamen Steuergerät, also einer Steuereinheit 500, die entweder wie hier zentral angeordnet ist oder in mehreren Untereinheiten über das System verteilt ist, 500 gesteuert werden. Hierzu können Kommunikationspfade zwischen der Steuereinheit und einzelnen Komponenten des Systems bzw. auch zwischen Untereinheiten der Steuereinheit genutzt werden.

Die Systeme für die Kurzpuls-Laser-Augenchirurgie 100 der Fig.1 und Fig. 2 enthalten weiterhin ein Gehäuse 110, das auch als Konsole bezeichnet werden kann. Dieses Gehäuse 110 umschließt die fs-Laserquelle 210 und das Steuergerät als zentrale Steuereinheit 500, im Falle des ersten Systems der Fig. 1 umschließt das Gehäuse 110 zudem das OCT-Modul 400.

Der Mikroskop-Kopf 320 ist an einem ersten Gelenkarm 120 und der Applikator-Kopf 220 ist an einem zweiten, separaten Gelenkarm 130 befestigt, durch den dem Applikator-Kopf 220 das Licht der fs-Laserquelle 210 zugeführt wird. Hierfür verläuft ein Strahlführungsmittel 230 durch den zweiten Gelenkarm 130. Erster Gelenkarm 120 und zweiter Gelenkarm 130 sind am Gehäuse 110 bzw. einer Verlängerung des Gehäuses 110 angebracht.

Eine Schnittstelle 150 ist am oder in der Nähe des Applikator-Kopfes 220 und des Mikroskop-Kopfes 320 vorgesehen, durch die der Applikator-Kopf220 und der Mikroskop-Kopf 320 mechanisch und optisch miteinander verbunden werden können.

Zum Lösen oder Zusammenfügen von Mikroskop-Kopf und Applikator-Kopf 220 mit Hilfe der Schnittstelle 150 ist ein vom Arzt oder automatisch zu schaltender Mechanismus vorgesehen ist.

Der zweite Gelenkarm 130 erhält die gleichen Freiheitsgrade wie der erste Gelenkarm 120, der gleichzeitig das Stativ des Operations-Mikroskops 300 bildet. Durch eine entsprechende Anzahl, Anordnung und Ausgestaltung der Gelenke 140 der Gelenkarme 120 und 130 werden die erforderlichen Freiheitsgrade erzeugt, durch die der Applikator-Kopf 220 und der Mikroskop-Kopf 320 sowohl unabhängig voneinander als auch miteinander verbunden in einem dreidimensionalen Volumen beweglich sind. Im Falle des ersten Systems für die Kurzpuls-Laser-Augenchirurgie 100 der Fig. 1 wird dies durch drei Gelenke 140 mit Kugelgelenksfunktion erreicht.

Im zweiten System für die Kurzpuls-Laser-Augenchirurgie 100 der Fig. 2, sind äquivalente Freiheitsgrade wie die mit drei Gelenken 140 mit Kugelgelenksfunktion durch drei Gelenke für die Rotation um die senkrechten Achsen 140-O1, 140-O2, 140-O5 und 140-L1, 140-L2, 140-L5 sowie einen Paralleltragarm 145, der ein Gelenkglied des ersten bzw. des zweiten Gelenkarms 120, 130 darstellt, mit horizontalen Drehgelenken 140-O3, 140-O4 und 140-L3, 140-L4 für die Auf- und AbBewegung, also eine Kippbewegung.

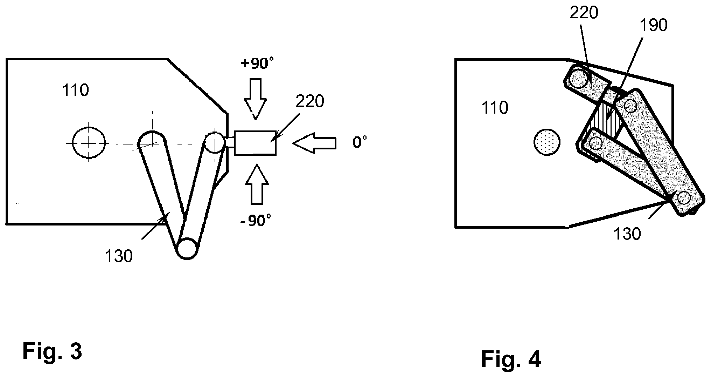

Zusätzlich besitzt der erste Gelenkarm 120 mit dem Mikroskop-Kopf 320 eine horizontale Kippachse für die Neigung des Mikroskop-Kopfes 140-O6. Diese kann für die Ankoppelpositionen +/-90° ebenfalls durch den Applikator-Kopf 220 realisiert werden, indem dieser drehbar auf der horizontalen Achse 140-L6 des letzten Gelenkglieds aufgehängt wird. In der Ankoppelposition 0° kann der Operations-Mikroskop-Kopf 320 nur in senkrechter Position angekoppelt werden. Dies ist in der Fig. 3 dargestellt, die die Positionen beim Verbinden von Applikator-Kopf 220 und Mikroskop-Kopf 320 zeigt. Der dicke Pfeil zeigt dabei die Blickrichtung des Arztes in die Okulare des Operations-Mikroskop-Kopfes 320.

Der Operations-Mikroskop-Kopf 320 hat also eine eigene Kippachse 140-O6, die manuell bedient werden kann. In Koppelstellung +/- 90° kann dieser Kippwinkel durch eine zusätzliche Drehachse 140-L6 im Applikator-Kopf 220 ausgeglichen werden. In Koppelstellung 0° ist das nicht möglich. Weiterhin muss unbedingt ausgeschlossen werden, dass der Bediener, also i.d.R. der Arzt, nach Ankopplung an den Applikator-Kopf 220 die Kippachse 140-O6 des Mikroskop-Kopfes 320 von Hand verstellt. Dadurch könnten die Gelenke des zweiten Gelenkarms 130 verspannt werden, was zu Abweichungen der optischen Achse des zweiten Gelenkarms 130, insbesondere des darin enthaltenen Strahlführungsmittels 230, und damit zu Abweichungen der Position des Fokus der Femtosekunden-Laserstrahlung im Auge 900 führt. Gelöst wird dieses Problem durch eine Motorisierung der Kippachse 140-06 des Mikroskop-Kopfes 320. Die Bedienung erfolgt ähnlich wie die manuelle Bedienung mit einem Drehknopf seitlich an der Mikroskop-Kopf-Aufhängung. Vor dem Ankoppeln, also dem Verbinden von Mikroskop-Kopf 320 und Applikator-Kopf 220 mittels der Schnittstelle 150, kann per Software geprüft werden, ob der Mikroskop-Kopf 320 senkrecht steht. Der Bediener erhält eine Aufforderung, bei Abweichungen zu korrigieren, oder aber der Mikroskop-Kopf 320 wird automatisch in eine senkrechte Position gebracht. Während der Laserbehandlung des Auges 900 des Patienten kann eine Betätigung der Kippachse 140-O6 per Software verhindert werden.

Die Längen der Gelenkglieder des zweiten Gelenkarmes 130 der Fig. 2 sind so ausgelegt, dass der gesamte Arbeitsbereich des Operations-Mikroskop-Kopfes 320 im Halbkreis von 180° vor dem Gerät, also vor dem System für die Kurzpuls-Laser-Augenchirurgie 100, ausgenutzt werden kann. Durch die Länge der Gelenkglieder ergeben sich Bereiche rechts und links vom Gerät 100, die im angekoppelten Zustand, also bei Verbindung von Mikroskop-Kopf 320 und Applikator-Kopf 220 nicht erreicht werden können. Das wird jedoch dadurch kompensiert, dass der abgelegte zweite Gelenkarm 130, an dem der Applikator-Kopf 220 angeordnet ist, je nach Bedarf nach rechts oder nach links ausgeknickt werden kann. Hierzu wird Applikator-Kopf 220 per Hand aus einer Parkschale auf dem Parkarm 190 genommen, auf die andere Seite schwenkt und wieder ablegt. Die elektrische Verriegelung der Parkschale wird dazu mit einem Schalter am Applikator-Kopf 220 gelöst. Zudem sind zwei Griffknöpfe 142 zur Totlagenvermeidung vorgesehen, an denen der zweite Gelenkarm 130 durch eine Totlage, in der seine Gelenkglieder in eine Ebene gestreckt sind, geführt werden kann. Die Griffknöpfe 142 sind bevorzugt an einem Gelenkglied zwischen vorletzten und letzten Gelenk 140 bzw. zwischen vorletztem und letzten Gelenk 140 mit jeweils senkrechter Rotationsachse angeordnet. Um den Gelenkarm 130 aus der Totlage wieder herauszubewegen ergreift ein Bediener beide Griffknöpfe 142 und schwenkt den Gelenkarm 130 damit wieder ein. Die Griffknöpfe 142 können steril überzogen werden, wobei ein entsprechender Überzug nach jeder Operation gewechselt wird, damit auch im Verlauf einer Operation nach einem Operationsschritt der zweite Gelenkarm 130 im Bedarfsfall an den Griffknöpfen 142 ergriffen werden kann ohne dass die Sterilität gefährdet wird.

Das Gelenk 140-L3 des zweiten Gelenkarmes 130, an dem der Applikator-Kopf 220 angeordnet ist, ist höher gelegt, um ein Zureichen von Gegenständen, z.B. durch die Operations-Assistenz unter dem zweiten Gelenkarm 130 hindurch zu ermöglichen. Die Höhe der Anordnung des Gelenks 140-L3 ist so gewählt, dass die Mindestquetschabstände zum ersten Gellenkarm 120, an dem der Operations-Mikroskop-Kopf 320 angeordnet ist, eingehalten werden. Bei Drehwinkeln über 180° besteht die Gefahr von Kollisionen der beiden Gelenkarme 120, 130. Um das auszuschließen, wird der Drehwinkel der E-Box, als des Gelenks 140-O1 des ersten Gelenkarms 120 auf +/-95° beschränkt durch einen Anschlag in der Achse des Gelenks 140-O1. Trotz dieser Beschränkung ist dies ausreichend, um ein ausgedehntes, dreidimensionales Volumen vor dem Gerät 100 zu erreichen und folglich einen Patienten auf einer Liege vor dem Gerät 100 grob zu platzieren und alles weitere mittels der Bewegung der Gelenkarme 120, 130 zu erledigen. Auf diese Art und Weise kann mit unterschiedlichen Lagerungsmöglichkeiten des Patienten gearbeitet werden, und auch spezielle Vorlieben des Arztes bzgl. der Anordnung von Patient und System für die Kurzpuls-Laser-Augenchirurgie 100 berücksichtigt werden.

Der Applikator-Kopf 220 befindet sich zwischen Patient und Mikroskop-Kopf 320 und muss aus diesem Grund sehr kompakt aufgebaut sein. Die notwendigen Aktoren zur Verschiebung des Fokus der Femtosekunden-Laserstrahlung in z-Richtung, d.h., entlang der optischen Achse, im Auge 900 sind sehr platzaufwändig und deswegen ist deren Unterbringung im Applikator-Kopf 220 nicht sinnvoll. Diese Aktoren sind deshalb in der Konsole, also dem Gehäuse 110, vor dem zweiten, den Applikator-Kopf 220 tragenden Gelenkarm 130 angeordnet. Um den dort erzeugten, wandernden Fokus in das Auge 900 zu übertragen, sind Relay-Objektive in jedem Gelenkglied des zweiten Gelenkarms 130 notwendig. Diese Objektive sind afokal. In jedem Relay entsteht ein wandernder Fokus je nach Fokusstellung. Die numerisch Apertur muss möglichst gering sein, um optische Durchbrüche und damit Leistungsverluste im zweiten Gelenkarm 130 bzw. in dem den zweiten Gelenkarm 130 durchlaufenden Strahlführungsmittel 230 zu vermeiden. Das bedingt lange Relay-Systeme. Die Gelenkglied-Längen der einzelnen Gelenkglieder des zweiten Gelenkarms 130 sind an die Lägen der Relay-Systeme angepasst.

An die Genauigkeit der optischen Übertragung des Laserstrahls von der Optik im Gehäuse 110 in den Applikator-Kopf 220, besonders im Zusammenhang mit den Bewegungsmöglichkeiten des zweiten Gelenkarms 130, stehen hohe Anforderungen. Bei der Justierung muss besonders darauf geachtet werden, dass die mechanische Drehachse und die Achse des Laserstrahls sowohl im Winkel als auch im Ort nicht voneinander abweichen. Jede Abweichung führt bei Bewegung des zweiten Gelenkarms 130 zum Taumeln des Laserfokus im Auge 900. Hinzu kommt, dass mit elastischen Deformationen auf Grund des hohen Gewichtes des zweiten Gelenkarms 130 und des daran angeordneten Applikator-Kopfes 220 zu rechnen ist, die stark abhängig sind von der Stellung des zweiten Gelenkarms 130. Deshalb ist die Justierung des zweiten Gelenkarms 130 nur in einer Position möglich. In jeder anderen Stellung sind Abweichungen zu erwarten. Diese werden durch ein automatische Strahlnachführung ausgeglichen, die die Abweichungen misst und die Position des Laserstrahls nachkorrigiert. Diese Korrektur erfolgt in bestimmten Grenzen, die von der Geometrie und den Stellbereichen der Aktoren vorgegeben sind. Der freie Durchmesser der Optik ist so dimensioniert, dass sich bei Ausnutzung des Stellbereiches keine Vignettierung des Laserstrahls ergibt. Die notwendige Steifigkeit der Lager und der Tele des zweiten Gelenkarms 130 ergibt sich aus dem möglichen Verstellbereich der automatischen Strahlnachführung. Elastische Deformationen dürfen die Möglichkeiten der Strahlnachführung nicht überschreiten. Die Steifigkeit des am meisten beanspruchten Gelenkgliedes zwischen den Gelenken 140- L1 und 140-L2 des zweiten Gelenkarms 130 wird erreicht durch eine starke Verrippung, eine kastenförmige Gestaltung und zusätzliche Stahlplatten auf beiden Seiten des Gelenkglieds. Für die Lagerung werden hochsteife Drehverbindungen eingesetzt, die mit zwei Nadellagern axial und einem Nadellager radial spielfrei vorgespannt sind. Alternativ sind Schrägkugellager in O-Konstellation mit großem Abstand der Kugelrollbahnen möglich.

Der zweite Gelenkarm 130 bietet Möglichkeiten zur Durchführung von elektrischen Kabeln, der OCT-Lichtleitfaser 410 sowie den Vakuumschläuchen für das Ansaugen einer Patienten-Schnittstelle 600 an das Auge 900 des Patienten als auch für das Ansaugen der Patienten-Schnittstelle an den Applikator-Kopf 220 . Am Übergang der Gelenke 140-L2/140-L3 sowie 140-L4/140-L5 werden alle Kabel außerhalb der Gelenke 140 geführt, um zu hohe Beanspruchung der Kabel gegen Torsion zu vermeiden. Am Gelenk 140-L1 werden die Kabel konzentrisch zur Optik durch das Gelenk 140 geführt.

Je nach Ausführungsvariante ist auf dem Gehäuse 110 eine Ablagefläche 190 für den Applikator-Kopf 220 markiert oder eine auf die Geometrie des Applikator-Kopfes 220 abgestimmte Ablagestruktur 190 angebracht.

Zum Transport, also beispielsweise dem Fahren des Gerätes 100 mittels einer unter dem Gerät 100 fixierten Transportvorrichtung 180 durch Türen dürfen der zweite Gelenkarm 130 und der Applikator-Kopf 220 nicht seitlich über das Gehäuse 110 überstehen. Das wird so gelöst, dass der Applikator-Kopf 220, abgelegt und verriegelt in einer Parkschale, die auf einem Parkarm 190 angeordnet ist, in ein Position über dem Gehäuse 110 und seitlich zur Säule des Operations-Mikroskops 300 nach hinten geschwenkt wird, siehe Fig. 4. Dazu muss der Parkarm 190 ebenfalls um ca. 60° geschwenkt werden.

Um den Applikator-Kopf 220 in eine Ruhe- bzw. Parkposition zu bringen, deren Anforderungen sich von denen einer Transportposition dadurch unterscheiden, dass das Überstehen des zweiten Gelenkarms 130 und des Applikator-Kopfes 220 über das Gehäuse 110 weniger kritisch ist, wird der Applikator-Kopf 220 einfach zur Seite geschwenkt und an den Gelenkarm angelegt. Die Arretierung von Transport- und Parkposition erfolgt beispielhaft mit kraftschlüssigen Rasten.

Die Parkposition entspricht vorzugsweise einer Ankoppelposition zum Verbinden von Mikroskop-Kopf 320 und Applikator-Kopf 220. Sie soll jedoch auf jeden Fall das Ansetzen einer Patienten-Schnittstelle 600 an den Applikator-Kopf 220 ermöglichen. Hierfür ist sie von beide Seiten uneingeschränkt zugänglich. Das wird erreicht indem eine Parkschale zur Ablage und Arretierung des letzten Gelenkgliedes des zweiten Gelenkarms 130 vor dem Applikator-Kopf 220 angeordnet wird.

Die Parkschale ist +/- 90° drehbar gelagert auf einem Parkarm 190. Der Parkarm 190 ist wiederum ca. 70° drehbar gelagert um die Hauptachse 140-L1 des zweiten Gelenkarms 130. Die Parkschale ist mit Raststellungen für Parkposition und Transportposition versehen. Sie enthält einen elektromechanischen Verriegelungsmechanismus für den Gelenkarm 130 des Applikator-Kopfes 220, einen Kraftsensor und einen induktiven Sensor zur Detektion der Anwesenheit des Applikator-Kopfes 220 bzw. des letzten Gelenkglieds vor dem Applikator-Kopf 220 in der Parkschale. Parkarm 190 und Parkschale sind so dimensioniert, dass der Applikator-Kopf 220 vor dem Gerät 100 überhängt, von unten frei zugänglich ist zur Anbringung der Patienten-Schnittstelle 600 und vorzugsweise gleichzeitig die Möglichkeit besteht, den Mikroskop-Kopf 320 von beiden Seiten ohne Behinderung anzukoppeln. Die Länge des zweiten Gelenkarms 130, an dem der Applikator-Kopf 220 angeordnet ist, ist so dimensioniert, dass ein angekoppelter Mikroskop-Kopf 320 auch mit einem Assistenz-Mikroskop-Kopf nicht mit dem zweiten Gelenkarm 130 kollidieren kann und gleichzeitig die Mindestquetschabstände eingehalten werden.

In einer weiteren Ausführungsvariante sind an dem Mikroskop-Kopf 320 Griffe 143 mit ggf. sterilen, wechselbaren Überzügen zur Positionierung des Mikroskop-Kopfes 320 angebracht. Durch die Positionierung des Mikroskop-Kopfes 320 wird letztlich im gekoppelten Zustand beider auch der Applikator-Kopf 220 positioniert. Die Griffe können als Schalter zum Lösen elektromagnetischer Bremsen des ersten-Gelenkarms 120, an dem der Mikroskop-Kopf 320 angeordnet ist, ausgeführt sein oder als reine mechanische Hebel bei Friktionsbremsen.

In einer weiteren Ausführungsvariante sind am ersten und/oder zweiten Gelenkarm 120, 130 oder am Applikator-Kopf 200 bzw. am Mikroskop-Kopf über das Steuergerät 500 verstellbare Elemente, wie z.B. Motoren, vorgesehen, die eine vom Steuergerät 500 gesteuerte Bewegung des Mikroskop-Kopfes 320 und/oder des Applikator-Kopfes 220 ermöglichen.

Vorteilhaft sind an einem oder an beiden Gelenkarmen 120, 130 Federelemente vorgesehen, die so aufeinander abgestimmt sind, dass sich der jeweilige zugeordnete Applikator-Kopf 220 oder Mikroskop-Kopf 320 innerhalb eines vorgegebenen Raumbereiches um das Gehäuse 110 und das Operationsfeld ohne externe Kräfte jeweils selbst im Raum hält.

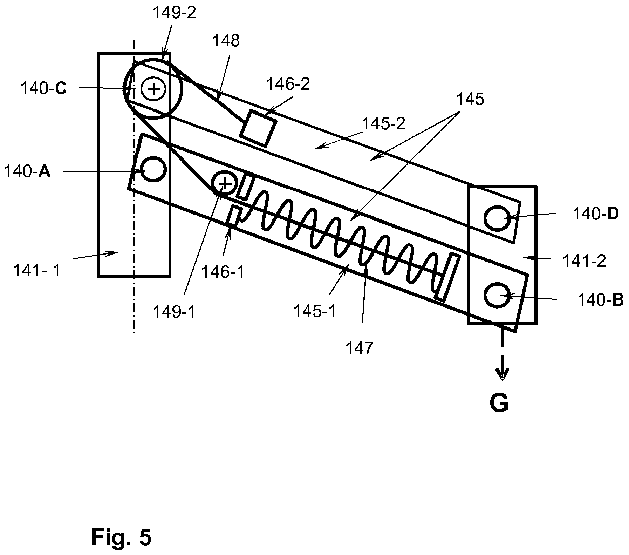

Der Applikator-Kopf 220 wiegt ungefähr 5 kg und kann vom Operations-Mikroskop 300 bzw. Mikroskop-Kopf 320 nicht getragen werden. Der Federausgleich des ersten Gelenkarms 120, an dem der Mikroskop-Kopf 320 angeordnet ist, ist mit Einblick, Okularen und ggf. Monitor bereits bis auf 1 kg ausgelastet. Der zweite Gelenkarm 130, an dem der Applikator-Kopf 220 angeordnet ist, enthält deshalb eine Vorrichtung zum unabhängigen Gewichtsausgleich, wie sie in der Fig. 5 dargestellt ist.

Der Gewichtsausgleich für alle auszugleichenden Massen erfolgt dabei bezüglich des Gelenks 140-L3 (140-A in der Fig. 5). Der Teil des zweiten Gelenkarms 130 zwischen den Gelenken 140-L3 und 140-L4 (140-B in der Fig. 5) wird als Paralleltragarm 145 ausgeführt. Der Paralleltragarm 145 besteht im Wesentlichen aus vier Gelenken 140-A, 140-B, 140-C, 140-D und vier Gelenkgliedern: dem ersten Drehkopf 141-1, dem zweiten Drehkopf 141-2, dem Federarm 145-1 und der Strebe 145-2.
Der Gewichtsausgleich wird realisiert mit einer Druckfeder 147 im unteren Federarm 145-1. Die Druckfeder 147 zieht an einem Zahnriemen 148, der über zwei Zahnriemenräder 149-1 und 149-2 umgelenkt wird in die Strebe 145-2. Dort ist der Zahnriemen 148 an einer Befestigung 146-2 eingehängt. Die Druckfeder 147 erzeugt ein Moment um den das Gelenk 140-A, das dem durch das Gewicht G erzeugte Moment um Punkt A entgegengerichtet ist und dieses kompensiert. Der Hebelarm des Kompensationsmomentes wird erzeugt durch den senkrechten Abstand des Zahnriemens 148 zum Gelenk 140-A. Dieser Hebelarm ist abhängig von der Winkelstellung des Federarmes 145-1. Die Federkonstante der Druckfeder 147 ist so dimensioniert, dass die stellungsabhängige Änderung beider Momente ausgeglichen wird. Dadurch wird erreicht, dass im gesamten Schwenkbereich die Gewichtskompensation innerhalb eines vorgegebenen Toleranzbereiches liegt. Die ausgeglichene Gewichtskraft G ist unabhängig von der Schwenkstellung des Gelenkarms 130 für den Applikator-Kopf 220. Zwar ändert sich durch das Schwenken des Applikators-Kopfes 220 der Abstand des Schwerpunktes zum Drehpunkt 140-A aber das hat keinen Einfluss auf die Gewichtskompensation.

Das sich dadurch ändernde Moment wird abgestützt durch die Strebe 145-2, die in den Drehpunkten 140-C und 140-D aufgehängt ist.

In einer Ausführungsvariante sind eine Video-Aufnahmeeinheit und eine Beleuchtungseinheit vorgesehen. Diesen können alternativ über den Applikator-Kopf 220 oder den Mikroskop-Kopf 320 in den Strahlengang zum bzw. vom Auge 900 eingekoppelt werden.

In einer speziellen Ausführungsvariante wird der zweite Gelenkarm 130, an dem der Applikator-Kopf 220 angeordnet ist, durch eine photonische Kristallfaser mit hohlem Kern als Strahlführungsmittel 230 komplementiert. Die fs-Laserstrahlung wird innerhalb des Hohlkerns und mittels periodischer photonischer Strukturen analog zu einem Bragg-Spiegel in der Faser geleitet. Auf diese Weise findet - ähnlich wie bei der Freistrahlung - nur eine geringe Pulsverbreiterung durch Dispersion statt. Im Vergleich zu einer Durchleitung durch den zweiten Gelenkarm 130 mittels eines Spiegelsystems bietet die photonische Kristallfaser den Vorteil, dass sie eine wesentlich flexiblere Laserstrahlführung gewährleistet und die Komplexität des optischen Aufbaus reduziert. In dieser Ausführungsvariante dient der zweite Gelenkarm 130, an dem der Applikator-Kopf 220 befestigt ist, im Prinzip nur dem mechanischen Halten des Applikator-Kopfes 220, beeinflusst also nicht mehr die Strahlführung durch seinen Aufbau selbst.

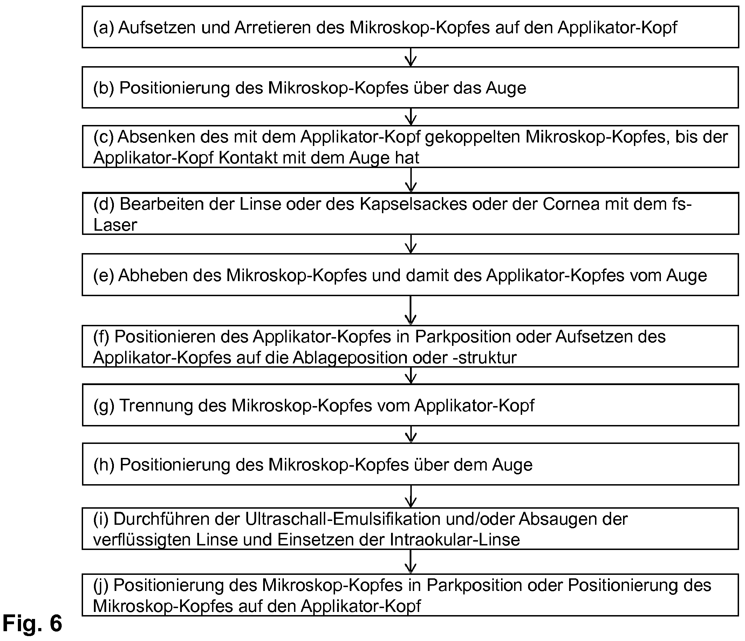

Der hier beschriebene Aufbau eines Systems für die Kurzpuls-Laser-Augenchirurgie 100 unterstützt das im Folgenden und anhand Fig. 6 veranschaulichte Verfahren zur Positionierung des Applikator-Kopfes und des Mikroskop-Kopfes auf das Auge des Patienten, das die folgenden Schritte enthält:
(a) Falls Applikator-Kopf 220 und Mikroskop-Kopf 320 getrennt sind, werden diese durch den Bediener, beispielsweise den Arzt, zusammengeführt. Dazu setzt der Bediener den Mikroskop-Kopf 320 auf den Applikator-Kopf 220 an der Schnittstelle 150 auf, und betätigt eine Verriegelung; oder aber ein Mechanismus führt bei Erreichen der gewünschten Verbindung automatisch zur Verriegelung.
(b) Der Bediener führt und positioniert den Mikroskop-Kopf 320 über das zu operierende Auge 900. Damit ist auch der Applikator-Kopf 220 über dem Auge 900 positioniert.
(c) Der Bediener blickt durch das Okular des Mikroskop-Kopfes320 und senkt den Mikroskop-Kopf 320 und damit auch den Applikator-Kopf 220 soweit ggf. unter weiterer lateraler Ausrichtung des Mikroskop-Kopfes 320 auf das Auge 900 ab, bis der Applikator-Kopf 220 in einer vorab definierten Position über dem Auge 900 steht oder eine am Applikator-Kopf lösbar angebrachte Patienten-Schnittstelle 600, die ein Kontaktglas 610 enthält, Kontakt mit dem Auge 900 hat.
(d) Der Bediener führt die Bearbeitung eines Augengewebes 910, also der Linse und/oder des Kapselsackes und/oder der Cornea mittels fs-Laser durch.
(e) Der Bediener hebt den Mikroskop-Kopf 320 und damit auch den Applikator-Kopf 220 an.
(f) Der Bediener bringt den Applikator-Kopf 220 in die Parkposition, setzt hierbei in einer Ausführungsvariante den Applikator-Kopf 320 auf die Ablagefläche bzw. Ablagestruktur 190 am Gehäuse 110 auf.
(g) Der Bediener löst über den Arretier-Mechanismus den Mikroskop-Kopf 320 vom Applikator-Kopf 220 oder das Lösen erfolgt automatisch bei korrektem Positionieren des Applikations-Kopfes 220 auf der Ablagestruktur 190. Dadurch erfolgt die Trennung des Mikroskop-Kopfes 320 vom Applikator-Kopf 220.
(h) Der Bediener positioniert den Mikroskop-Kopf über dem Auge 900 des Patienten.
(i) Der Bediener führt die weiteren Schnitte der Phacoemulsifikation und/oder des Absaugens der verflüssigten Linse und des Einsetzens der intraokularen Linse durch.
(j) Der Bediener positioniert den Mikroskop-Kopf 320 in einer Parkposition abseits des Operationsfeldes. In einer Ausführungsvariante setzt der Bediener den Mikroskop-Kopf auf den Applikator-Kopf, welcher sich auf der Ablagefläche 190 am Gerät 100 befindet, auf und verriegelt den Arretiermechanismus oder aber der Arretiermechanismus wird automatisch bei Erreichen der Verbindung verriegelt.

In einer Ausführungsvariante des Verfahrens berechnet das Steuergerät 500 unter Zuhilfenahme gewonnener OCT-Bilder und/oder Video-Bilder Steuerbefehle für verstellbare Elemente an den Gelenkarmen 120, 130 bzw. des Applikator-Kopfes 220 und/oder des Mikroskop-Kopfes 320 , so dass insbesondere die Schritte (c) und/oder (e) ggf. auch alle weiteren Schritte, mit Ausnahme des Schrittes (i), automatisch vom Steuergerät 500 gesteuert werden.

In einer weiteren Ausführungsvariante des Verfahrens wird, sobald der Mikroskop-Kopf 320 mit dem Applikator-Kopf 220 verriegelt ist, z.B. über einen Sensor vom Steuergerät 500 bestimmbar der Gerätezustand geändert. Z.B. kann der fs-Laser automatisch eingeschaltet und eine Beleuchtung über das Operations-Mikroskop 300 ausgeschaltet werden. Entsprechend kann im entriegelten, d.h. getrennten Zustand von Mikroskop-Kopf 320 und Applikator-Kopf 220 der fs-Laser ausgeschaltet werden und eine Beleuchtung über das Operations-Mikroskop 300 eingeschaltet werden.

Bautechnisch ist das Gehäuse 110, insbesondere das Gehäuseinnere bevorzugt so gestaltet, dass diejenigen Komponenten des Kurzpuls-Lasersystems 200, die vom Gehäuse umschlossen werden, also die Kurzpuls-Laserquelle 210 (hier eine fs-Laserquelle) und optische Komponenten als Teil des Strahlführungsmittels, im montierten Zustand als Ganzes in und an einem Container seitlich über die Säule 310 des Operations-Mikroskops 300 geschoben werden kann. Die Säule 310 stellt dabei als Verlängerung des Gehäuses 110 eine Stützstruktur für den ersten Gelenkarm 120, an dem der Mikroskop-Kopf 320 angeordnet ist, dar. Die vom Gehäuse 110 umschlossenen Komponenten des Kurzpuls-Lasersystems 200 werden also im montierten Zustand auf der Fußplatte des Operations-Mikroskops 300 abgesetzt und an vier Stellen befestigt. Im zweiten System für die Kurzpuls-Laser-Augenchirurgie 100 der Fig. 2 erfolgt dies möglichst dicht an den Rädern, die unter der Fußplatte als Transportvorrichtung 180 befestigt sind, als starre Befestigung mit ca. 6mm Abstand über der Fußplatte.

Um die Stabilität der Optikjustierung für die Komponenten des Kurzpuls-Lasersystems 200 im Gehäuse 110 wie auch im zweiten Gelenkarm 130 zu garantieren, sind verschiedene Vorkehrungen nötig. Elastische Deformationen der tragenden Teile des Gehäuses 110 durch Stellungsänderungen von erstem und/oder zweiten Gelenkarm 120, 130 dürfen sich nicht auf den Justierzustand der Optik zwischen fs-Laserquelle 210 und Eintritt in den zweiten Gelenkarm 130, an dem der Applikator-Kopf 220 angeordnet ist, auswirken. Diese elastischen Deformationen sind nicht unerheblich besonders wenn man berücksichtigt, dass sowohl der erste Gelenkarm 120 mit dem Mikroskop-Kopf 320 als auch der zweite Gelenkarm 130 mit dem Applikator-Kopf 220, inklusive der Vorrichtung für einen unabhängigen Gewichtsausgleich in Form eines Paralleltragarms145 und dessen Aufbauten jeweils ein Gewicht in der Größenordnung von 50kg aufweisen. Beim Schwenken ergeben sich Schwerpunktverlagerungen, die zu Deformationen im Bereich mehrerer Zehntel Millimeter führen können. Elastische Deformationen des zweiten Gelenkarms 130, an dem der Applikator-Kopf 220 angeordnet ist, bzw. seiner Gelenkglieder, werden durch die eigene Strahlstabilisierung ausgeglichen. Deformationen der Optik des Kurzpuls-Lasersystems 200 im Gehäuse 110, also vor Eintritt in den zweiten Gelenkarm, können dagegen nicht ausgeglichen werden. Die Genauigkeitsanforderungen der Konsolenoptik, also der Optik, die im Gehäuse 110 hinter der Kurzpuls-Laserquelle 210 und vor dem zweiten Gelenkarm 130 angeordnet ist, liegen jedoch im Mikrometerbereich und können ohne besondere konstruktive Maßnahmen nicht eingehalten werden.

Um die Anforderungen einzuhalten ist deshalb die gesamte Optik des Kurzpuls-Lasersystems 200, die sich im Gehäuse 110 vor dem Eintritt in den zweiten Gelenkarm 130 im Strahlengang der Kurzpuls-Laserstrahlung befindet, einschließlich des Ausgangs der fs-Laserquelle 210, auf einer Optikbank angeordnet bzw. sind an ihr angeschraubt. Die Optikbank selbst ist mit drei Punkten auf bzw. an dem Gehäuse 110 befestigt. Alle Deformationen dieser Befestigungsfläche des Gehäuses haben damit keinen Einfluss auf den Justierzustand der Teile auf der Optikbank, jedoch auf die Position der Optikbank zum Eintritt in den zweiten Gelenkarm 130.

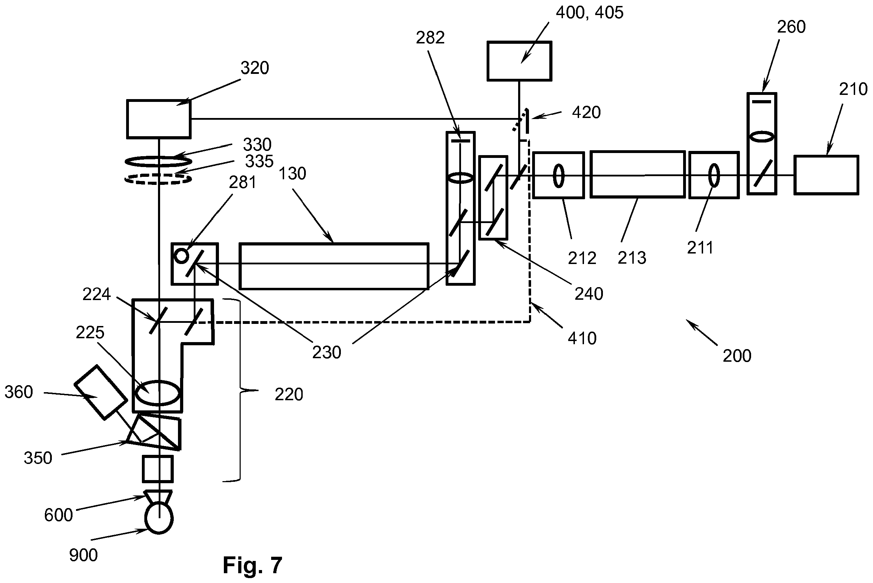

Änderung dieser Position können durch eine Strahlstabilisierung mit einem System zur Stabilisierung eines Strahldurchgangs 280 ausgeglichen werden. Ein erster aktiver Spiegel eines solchen System zur Stabilisierung eines Strahldurchgangs 280 befindet sich mittelbar an der Optikbank. Ein weiterer aktiver Spiegel befindet sich im zweiten Gelenkarm 130. Beide bilden einen Beamwalk. Eine Laserdiode 281 im Applikator-Kopf 220 sendet einen Laserstrahl über alle Spiegel des zweiten Gelenkarms 130 einschließlich der Spiegel des Systems zur Stabilisierung eines Strahldurchgangs 280 auf zwei Quadrantenempfänger 282 im Gehäuse 110, die zur Optikbank fixiert sind. Abweichungen durch Deformationen beim Bewegen des zweiten Gelenkarms oder durch Bewegung der Optikbank werden hier erkannt und können durch Gegensteuern mittels der aktiven Spiegel ausgeglichen werden. Die Optik eines solchen Systems zur Stabilisierung eines Strahldurchgangs 280 ist in der Fig. 7 mit dargestellt.

Wie bereits beschrieben, werden die Komponenten des Kurzpuls-Lasersystems 200, die vom Gehäuse umschlossen sind, bevorzugt an vier Punkten möglichst nahe der an der Fußplatte angebrachten Rädern 180 befestigt. Mittelbar hängt daran auch der zweite Gelenkarm 130 sowie die Elektronik bzw. die Steuereinheit 500. Wechselnde Kräfte durch Schwenken des ersten Gelenkarms 120, an dem der Mikroskop-Kopf 320 angeordnet ist oder des zweiten Gelenkarms 130, an dem der Applikator-Kopf 220 angeordnet ist, werden direkt auf die Räder 180 und den Fußboden übertragen. Das Gerät 100 darf während einer Laserbehandlung nicht fahren. Änderungen der Kraftverhältnisse an den Rädern 180 durch Unebenheiten des Fußbodens wirken sich direkt auf den Justierzustand der Laseroptik aus. Im stationären Betrieb wird dieser Einfluss durch die Strahlstabilisierung einmalig vor jeder Operation ausgeglichen. Die Konsole wird an vier Punkten mit der Fußplatte des Operations-Mikroskops 300 verschraubt. Zwei der vier Punkte, an denen die Komponenten des Kurzpuls-Lasersystems 200 mit der Fußplatte des Operations-Mikroskops 300 verschraubt sind, sind höhenverstellbar. Dadurch können Überbestimmtheiten, die durch die Befestigung an vier Punkten entstehen, ausgeglichen werden. Spannungen durch zu erwartende Unebenheiten zwischen den in der Regel in und an einem Container montierten Komponenten des Kurzpuls-Lasersystems 200 und der Fußplatte des Operations-Mikroskops 300 werden dadurch vermieden, dass zwischen einer Bodenplatte des Containers und der Fußplatte des Operations-Mikroskops 300 ein Abstand von ca. 6 mm hergestellt wird.

Der Container besteht bevorzugt im Wesentlichen aus Boden- und Deckplatte, die mit senkrecht stehenden Wänden zu einem Kasten vernietet werden. Im Vergleich zu einem Rahmengestell können damit, bei gleichzeitig kompakter Bauweise, Querspannungen einfacher aufgenommen werden. Der Container ist fest eingebunden in das Gehäuse 110: Am Container befestigte Komponenten haben damit auch eine feste Beziehung zum Gehäuse 110.

Eine Deckplatte trennt den Optikteil oben von Elektronikkomponenten und Kabeln unten. Sie ist teilweise als Sandwich ausgeführt, um die Kabel in Zwischenräumen nach zu den Elektronikkomponenten zu führen. Auf der Deckplatte ist die Optikbank mit dem Ausgang der fs-Laserquelle 210 sowie der zweite Gelenkarm 130 aufgeschraubt. Der plattenförmige Aufbau des Containers garantiert genügend Stabilität für die Optikbank, nicht aber für den zweiten Gelenkarm 130, an dem der Applikator-Kopf 220 angeordnet ist. Um innerhalb der Möglichkeiten der Strahlstabilisierung zu bleiben muss der zweite Gelenkarm 130 sehr stabil fixiert werden. Das wird erreicht durch vier steife Säulen direkt unter den Anschraubpunkten des zweiten Gelenkarms 130, die Stützkräfte direkt in die Bodenplatte einleiten. Die Säulen werden nur auf Druck belastet und können durch zweifaches Umbiegen der Wände, die i.d.R. aus einem Metallblech bestehen, realisiert werden. Ausknicken wird durch geschickte Lage der Biegekanten verhindert.

Die Rückseite des Container bilden parallel senkrecht stehende Wände, die sowohl zur Versteifung des Containers beitragen als zur Unterbringung von Elektronikkomponenten dienen. Die Elektronikkomponenten stehen senkrecht parallel nebeneinander und können nach hinten aus dem Gerät 100 zwecks Service herausgezogen werden. Zwischen der Rückwand des Gerätes 100 und den Elektronikkomponenten ist Platz für die Verkabelung reserviert.

Durch die senkrechte Anordnung ergibt sich ein natürlicher Schornsteineffekt für warme Luft, der für die Lüftung genutzt werden kann. Deswegen sind in der Nähe den Elektronikkomponenten Öffnungen angebracht, so dass warme Luft von Lüftern durch die Elektronikkomponenten gezogen, und nach hinten rausgedrückt werden kann. Damit bleibt ein operationsnaher Bereich des Raumes weitgehend verschont von Luftbewegungen, die Staub aufwirbeln oder den operationsnahen Bereich austrocknen können. Vorteilhaft ist die Verwendung von Radiallüftern, die platzsparend eingesetzt werden können. Über den Lüftern ist eine geschlossene Metallblechplatte angeordnet, die die Elektronikkomponenten vom oberen Teil des Geräteinneren trennt in dem Komponenten des Kurzpuls-Lasersystems 200 untergebracht sind. Dadurch sind die Komponenten des Kurzpuls-Lasersystems 200 weitgehend von der Wärmeentwicklung im unteren Teil abgeschirmt.

### Systemaufbau des Kurzpuls-Lasersystems: Strahlerzeugung und Optik

Um eine zeitoptimierte Bearbeitung der Cornea hinsichtlich Zugangsschnitten und/oder Relaxationsschnitten, bzw. Schnitten zur Bearbeitung der Linse bzw. einer Kapsulotomie mit einem fs-Laser zu ermöglichen, wird der in Fig. 7 gezeigte Aufbau eines Kurzpuls-Lasersystems 200 offenbart.

Fig. 3 zeigt ein fs-Lasersystem 200 für die Augenchirurgie, insbesondere für die Kataraktchirurgie, das eine fs-Laserlichtquelle 210 enthält. Die Lichtpulse der hierin erzeugte gepulste Laserstrahlung, werden über eine die Divergenz variierende Linse bzw. ein die Divergenz variierendes Linsensystem 211 und weitere fokusverstellende optische Elemente 212, mit denen eine gesteuerte z-Verschiebung des Fokus der gepulsten Laserstrahlung erzielt werden kann, einen x/y-Spiegel-Scanner 240, der einen x-Spiegel-Scanner und einen y-Spiegel-Scanner umfasst, oder aber alternativ über einen kardanisch aufgehängten Spiegel-Scanner oder aber wiederum alternativ über einen x-Spiegel-Scanner mit nachgeschaltetem Element zur Rotationsdrehung um die optische Achse, des Weiteren über einen Spiegel enthaltenden zweiten Gelenkarm 130, eine in x/y-bewegliche Objektivlinse 225 und eine ein Kontaktglas 610 enthaltende Patienten-Schnittstelle 600 in das Auge 900 geleitet und im Auge 900 fokussiert.

Durch die Divergenz variierende Linse bzw. durch das Divergenz variierende Linsensystem 211, das entlang der optischen Achse - die der z-Achse entspricht - über einen vom Steuergerät 500 gesteuerten Verstellmechanismus in der Position (seiner Linsen zueinander und auf der optischen Achse) verändert wird, wird die Divergenz der gepulsten Laserstrahlung und über weitere fest stehende optische Elemente wie eine Relay-Optik 213 und/oder bewegliche Fokussierelemente 212 die Fokuslage der gepulsten Laserstrahlung entlang der optischen Achse, also in z-Richtung, im Auge 900 geändert.

Durch das x/y-bewegliche Objektiv 225 wird die laterale Fokuslage der gepulsten Laserstrahlung senkrecht zur optischen Achse des Gerätes, also in x- und y-Richtung, eingestellt. Bei gegebener Stellung des x/y-Spiegel-Scanners 240 werden die Femtosekunden-Laser-Pulse auf einen ca. 5µm breiten Spot innerhalb des durch den Verfahrbereich des beweglichen Objektivs 225 definierten Bereiches des Auges 900 fokussiert.

Beim Scannen mittels der x/y-Spiegel-Scanner 240 und mit einem in fester Position verharrenden Objektiv 225 wird innerhalb des Bildfeldes des Objektives 225 die Fokuslage der Femtosekunden-Laser-Pulse innerhalb des Auges 900 verschoben.

Beim gleichzeitigem Scannen mittels des x/y-Spiegel-Scanners 240 und Verfahren des beweglichen Objektives 225 kommt es zu einer Überlagerungsbewegung.

In einer bevorzugten Ausführungsvariante ist in das Kurzpuls-Lasersystem 200 für die Augenchirurgie ein System zur Stabilisierung eines Strahlendurchganges 280 durch den zweiten Gelenkarm 130 integriert. Wie Fig. 7 zeigt, umfasst es eine Lichtquelle 281 an einem Ende des zweiten Gelenkarmes 130, die ihr Licht in den zweiten Gelenkarm mit Hilfe der Spiegel einkoppelt, die auch als Strahlführungsmittel die gepulste Laserstrahlung führen und übertragen, sowie einem positionsempfindlichen 282 am anderen Ende des zweiten Gelenkarmes. Die Lichteinkopplung erfolgt unter einem Winkel zur optischen Achse des zweiten Gelenkarmes 130, d.h., dass z.B. die Lichtquelle 281 nicht auf der optischen Achse angeordnet ist oder z.B. die Lichtquelle 281 auf der optischen Achse angeordnet ist, aber in Richtung der optischen Achse nicht symmetrisch abstrahlt.

Diese Strahlstabilisierung erlaubt es, trotz verschiedenen Stellungen des zweiten Gelenkarmes, die Ablenkung des Fokus der Kurzpuls-Laserstrahlung durch den x/y-Spiegel-Scanner 240 des mit der x/y-Positionierung der beweglichen Objektivlinse 225 in jeder Richtung x und/oder y genau zu positionieren und mechanische Toleranzen des zweiten Gelenkarms 130 und der Spiegelausrichtungen auszugleichen.

Dazu werden die Schritte des folgendes Verfahrens angewandt:
1. Bestimmen der Ablageposition des Lichtstrahls der Lichtquelle 281 eines Systems zur Stabilisierung eines Strahldurchgangs 280 auf dem ortsauflösenden Sensor 282 von einer Referenzposition oder einer Referenzwinkelstellung der Gelenkglieder des zweiten Gelenkarms 130. Die Ablageposition hängt von der Drehung der Elemente, also der Gelenkglieder, des zweiten Gelenkarmes 130 zueinander ab.
2. Berechnung einer Steuergröße zur Einstellung des x/y-Spiegel-Scanners 240 für die Fokuspositionierung der Kurzpuls-Laserstrahlung unter Nutzung der Information zur Ablageposition bzw. zu Ablagepositionen verschiedener Stellungen des zweiten Gelenkarms 130. Im Wesentlichen wird durch diese Ablageposition die Phasenlage der schwingenden Spiegel des x/y-Spiegel-Scanners oder der x/y-Schwingungsrichtungen eines kardanischen Spiegels festgelegt. In einer Ausführungsvariante wird, sofern die Ablagepositionen einen vorgegeben Wert überschreiten, die Laserstrahlführung auf das Auge 900 abgebrochen oder unterbrochen.

Zusätzlich ist die oben bereits beschriebene Anordnung derjenigen Optik des Kurzpuls-Lasersystems 200, die sich vordem zweiten Gelenkarm 130 befindet, auf einer Optikbank eine Maßnahme zur Vermeidung der Einflüsse von mechanischen Deformationen auf die Justierung der Laseroptik.

In einer Ausführungsvariante des Kurzpuls-Lasersystems 200 für die Augenchirurgie ist das Bildfeld des Objektives 225 welches durch den x/y-Spiegel-Scanner 240 überstrichen wird, größer als 1mm im Querschnitt aber kleiner als 6 mm. In einer bevorzugten Variante ist es größer als 1,5 mm aber kleiner als 3mm.

Ein zu kleines Bildfeld bedingt, dass z.B. bei lateral kleineren Schnitten im Auge 900, die schnelle Bewegung der x/y-Scanner 240 alleine nicht ausreicht, um einen kompletten Schnitt zu vollziehen. Dies hat zur Folge, dass durch das dann notwendige langsame Verfahren des Objektives 225 die Erzeugung des kompletten Schnitts wesentlich länger dauert. Es sollte deshalb die Feldgröße des Objektives 225 gerade so gewählt werden, dass beispielsweise Zugangsschnitte in der Cornea 910 eines Auges 900 mit einer Länge von etwa 1,5 mm in x-Richtung und beim Schnitt in die Tiefe des Cornea-Gewebes 910 eine projizierte y-Breite von 2mm keiner Bewegung des Objektives 225 bedürfen, sondern lediglich des Scanens mit den Scan-Spiegeln des x/y-Spiegel-Scanners 240. Allerdings sollte das Bildfeld auch nicht zu groß sein, weil sonst das Objektiv 225 zu schwer und damit zu träge und langsam für großräumige Bewegungen wie z.B. bei der Kapsulotomie wird.

Bei Kopplung von Mikroskop-Kopf 320 und Applikator-Kopf 220 mittels einer Schnittstelle 150 führt der Strahlengang für das durch dem Mikroskop-Kopf 320 zu empfangende Licht durch den Applikator-Kopf 220 hindurch. Um dies zu gewährleisten gibt es alternative Lösungen:
In einer ersten Lösung kann eine Laseroptik im Applikator-Kopf 220 so ausgelegt sein, dass der Spiegel 224, dessen Aufgabe es ist, die von der fs-Laserquelle 210 kommende Laserstrahlung auf das Objektiv 225 im Applikator-Kopf 220 umzulenken, eine partielle Transparenz aufweist - insbesondere im Bereich des sichtbaren Lichts, das zur Beobachtung des Auges 900 mit dem Mikroskop-Kopf 320 benötigt wird, während die Kurzpuls-Laserstrahlung nahezu vollständig reflektiert wird. Eine weitere Linse 335 zur Anpassung an die von der Laseroptik kommenden Strahlung kann dabei vor dem Objektiv 330 des Mikroskop-Kopfes 320 im Strahlengang des Operations-Mikroskops 300 beweglich angeordnet sein.

In einer alternativen Lösung ist die Laseroptik, die dann einen vollreflektierenden Spiegel 224 enthält, mittels eines Schlittens in den Applikator-Kopf 220 einfahrbar. Um den Mikroskop-Kopf 320 zur Beobachtung des Auges 900 zu nutzen, wird die Laseroptik aus dem Strahlengang des Operations-Mikroskop 300, der durch den Applikator-Kopf 220 hindurch führt, entfernt. Während der Nutzung der Kurzpuls-Laserstrahlung ist das Operations-Mikroskop 300 zur Beobachtung des Auges 900 nicht einsetzbar. Um dennoch eine Möglichkeit der Beobachtung zu schaffen, wird mittels einer Kamera, bevorzugt mit einer Infrarot-Kamera 360, über ein Strahlteiler-Prisma 350 das Auge 900 mit Licht, für das die Kamera sensibel ist, hier also IR-Licht, beobachtet.

### Optische Kohärenztomographie und Navigation

Für die Festlegung der Bearbeitungsmuster im Auge 900 werden mittels der optischen Kohärenztomographie (OCT) die Strukturen des Auges 900, insbesondere die Strukturen der Vorderkammer des Auges 900 vermessen. Bei der OCT-Bildgebung wird das Licht einer Kurzkohärenzlichtquelle über das Auge 900 lateral, d.h. senkrecht zur optischen Achse des Auges 900 gescannt. Vom Auge 900 reflektiertes oder gestreutes Licht wird mit dem Licht eines Referenzstrahlengangs zur Interferenz gebracht. Das von einem Detektor gemessene Interferenzsignal wird analysiert. Aus diesem lassen sich dann die axialen Entfernungen von Strukturen im Auge 900 rekonstruieren. In Verbindung mit dem lateralen Scannen lassen sich somit Strukturen im Auge 900 dreidimensional erfassen.

Um ein mit dem Fokus einer Kurzpuls-Laserstrahlung zu erzeugendes Schnittmuster im Auge 900 gegenüber den relevanten Strukturen des Auges 900 festzulegen, zeigt die Fig. 7 die (optische) Integration eines OCT-Moduls 400 in den Aufbau eines Kurzpuls-Lasersystems für die Augenchirurgie 200 und folglich auch in ein System für die Kurzpuls-Laser-Augenchirurgie 100.

In einer Variante des Aufbaus wird dieselbe OCT-Lichtquelle 405 wahlweise in den Operations-Mikroskop-Kopf 320 als auch in den Applikator-Kopf 220 eingekoppelt. Entsprechend wird das vom Auge 900 rückreflektierte Licht der OCT-Lichtquelle 405 über dasselbe Interferometer mit dem Referenzlicht überlagert und mit dem gleichen Detektor detektiert. Dies ist in Fig. 7 dargestellt:
Um die Integration der verschiedenen Komponenten in einen für den Arzt optimierten Workflow und einer optimierten Arbeitsumgebung zu verbessern, wird in Fig. 7 ein Aufbau offenbart, bei dem eine fs-Laserquelle 210, ein Applikator-Kopf 220, Strahlführungsmittel 230 (in Fig. 7 sind nur beispielhaft Komponenten des Strahlführungsmittels 230 bezeichnet) zur Leitung des fs-Laserstrahlung in den Applikator-Kopf 220 und von dort auf das zu operierende Auge 900, ein Mikroskop-Kopf 320, ein beweglicher, Spiegel enthaltender, zweiter Gelenkarm 130 und ein OCT-Modul 400, das eine OCT-Lichtquelle 405, einen Referenzstrahlengang, ein Interferometer, einen Detektor und ein oder mehrere Schaltstellen 420 enthält, durch ein Steuergerät 500 - nicht gezeigt in Fig. 7 - gesteuert werden. Die Schaltstellen 420 leiten das von der OCT-Lichtquelle 405 abgegebene Licht und das vom Auge 900 zurückkommende Licht der OCT-Lichtquelle 405 in einem ersten Zustand nur über den Applikator-Kopf 220 und in einem zweiten Zustand nur über den Mikroskop-Kopf 320. Dies ermöglicht beispielsweise die Nutzung des OCT-Moduls zusammen mit dem Mikroskop-Kopf 320 für den Operationsteil des Einsetzens der Intraokularlinse (IOL), bei dem der Applikator-Kopf nicht gebraucht wird und vom Mikroskop-Kopf 320 entkoppelt in einer Parkposition verweilt, und stellt anderseits sicher, dass der Beleuchtungs- und Detektionsstrahlengang des OCT-Moduls 400 für das Setzen der Schnitte mittels des Fokus der fs-Laserstrahlung dem Strahlengang der fs-Laserstrahlung entspricht, wodurch Ausrichtungsfehler vermieden werden können. Durch die Schaltstelle bzw. Schaltstellen 420 ist dies möglich, ohne dass ein weiteres OCT-Modul integriert werden muss.

Um die Integration des OCT-Moduls 400 zu verbessern, zeigt die Fig. 7 ein Kurzpuls-Lasersystem 200 für die Augenchirurgie, das eine aus fs-Laserquelle 210 und ein OCT-Modul 400, das eine Kurzkohärenz-Lichtquelle 405 und ein Interferometer enthält, wobei die fs-Laserstrahlung und die Strahlung der OCT- Kurzkohärenz-Lichtquelle 405 über den gleichen, Spiegel enthaltenden, zweiten Gelenkarm 130 dem Applikator-Kopf 220 und über diesen dem Auge 900 zugeführt werden. Nach Zusammenführung der Strahlung beider Lichtquellen werden dabei beide über den gleichen x/y-Spiegel-Scanner 240 lateral abgelenkt werden. In diesem Fall ist das dem OCT-Modul zugeordnete Interferometer mit einem Strahlteiler und zwei Spiegeln im Strahlengang direkt vor dem Austrittsort am Objektiv 225 angeordnet (in der Fig. 7 nicht dargestellt).

Diese Lösung hat den Vorteil, dass nur ein einziges Strahlführungsmittel 230, hier in Form einer unter Zuhilfenahme von Spiegeln gebildeten Zuleitungsoptik, für die fs-Laserstrahlung und die Strahlung der OCT-Lichtquelle 405 zum Applikator-Kopf 130 notwendig ist. Alternativ zum Spiegel enthaltenden zweiten Gelenkarm 130 kann als Strahlführungsmittel 230 für die Zuführung der fs-Laserstrahlung und der Strahlung der OCT-Kurzkohärenz-Quelle 405 eine photonische Kristallfaser verwendet werden. In diesem Fall können die Gelenkglieder des zweiten Gelenkarms 130 ohne Spiegel ausgeführt werden.

Um die Integration des OCT-Moduls 400 weiter zu verbessern und Alternativen zu bieten, ist in Fig. 7 auch eine weitere Lösung skizziert: Das hier gezeigte Kurzpuls-Lasersystem 200 zeigt auch eine fs-Laserquelle 210 und ein OCT-Modul 400, das eine Kurzkohärenz-Lichtquelle 405 und ein Interferometer enthält, wobei die fs-Laserstrahlung über einen x/y-Spiegel-Scanner 240 zur lateralen Ablenkung und anschließend über einen Spiegel enthaltenden zweiten Gelenkarm 130 einem Applikator-Kopf 220 zugeführt wird, die Strahlung der OCT- Kurzkohärenz-Lichtquelle jedoch über eine Lichtleitfaser 410, ohne über den x/y-Spiegel-Scanner 240 geleitet zu werden, dem Applikator-Kopf 220 zugeführt wird. Dabei wird im Applikator-Kopf 220 der Strahlengang des fs-Lasers und der Strahlung der OCT-Lichtquelle zusammengeführt und über ein lateral bewegliches Objektiv 225 in das Auge 900 geführt werden.

Diese Lösung hat den Vorteil, dass im OCT-Strahlengang keine der vielen optischen Elemente des Spiegel enthaltenden zweiten Gelenkarmes 130 angeordnet sind und somit deren störende Reflexe im OCT-Detektionssignal nicht mehr auftreten.

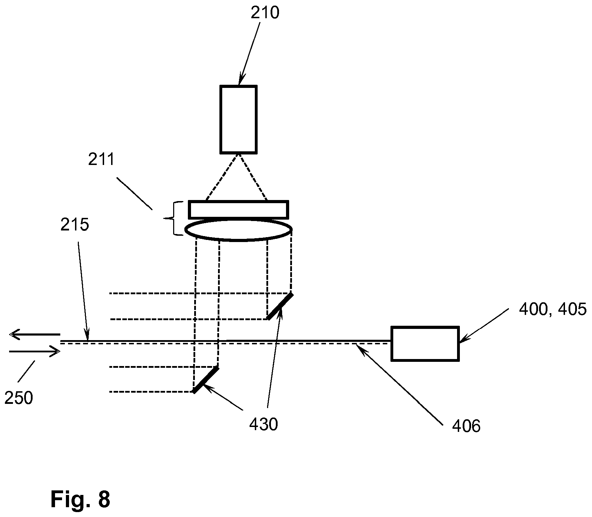

Für eine Integration des OCT-Moduls 400 mit einer OCT-Kurzkohärenz-Lichtquelle 405 und einem Interferometer zeigt die Fig. 8 ein weiteres Detail, das es ermöglicht, in einem Kurzpuls-Lasersystem 200 die Strahlung aus einer fs-Laserquelle 210 und aus einer OCT- Kurzkohärenz-Lichtquelle 405 eines OCT-Moduls 400 auf einer gemeinsamen optischen Achse 215 zu vereinigen und einen gemeinsamen optischen Strahlengang 250 zum und vom Auge 900 zu verfolgen. Hierzu trifft die aus einer fs-Laserquelle 210 kommende fs-Laserstrahlung nach einer fs-Laser-Strahlformungsoptik 211 auf einen Ringspiegel 430 und wird an diesem in Richtung des Auges 900 reflektiert. Die Strahlung der OCT-Kurzkohärenz-Lichtquelle 405 des OCT-Moduls 400 hingegen verläuft durch ein mittig im Ringspiegel 430 angeordnetes Loch in Richtung des Auges 900 und damit auf demselben Weg wie die fs-Laserstrahlung. Auch wird durch einem im OCT-Modul 400 angeordneten OCT-Detektor Licht vom Auge durch das Loch im Ringspiegel 430 detektiert.

Dies hat den Vorteil, dass für die Formung der fs-Laserstrahlung durch die fs-Laser-Strahlformungsoptik 211 vor allem die hohen Aperturbereiche genutzt werden. Dadurch wird einerseits die Fokussierung verbessert. Andererseits werden bei der Fokussierung der fs-Laserstrahlung in die Linse eines Auges 900 beim weiteren Durchgang durch das Auge 900 im Bereich der Retina nur die peripheren Bereiche beleuchtet, wodurch das Risiko für den Patienten sinkt, durch die Strahlung im zentralen Makulabereich geschädigt zu werden. Ferner hat die Ringblenden-Teilung den Vorteil, dass die Strahlung der OCT-Kurzkohärenz-Lichtquelle 405, also der OCT-Mess- und-Detektionsstrahl, ohne eine durch seine Reflexe optisch störende Oberfläche auf die optische Achse 215 des Kurzpuls-Lasersystems 200 geleitet wird. Dies ist bei einer Einkopplung mittels eines dichroitischen Filters oder bei nahezu gleicher Wellenlänge der Strahlung der OCT-Kurzkohärenz-Lichtquelle 405 und der fs-Laserstrahlung bei Einkopplung mittels eines farbneutralen Teilers nicht der Fall. Die farbneutrale Teilung würde außerdem zu zusätzlichen Intensitätsverlusten sowohl für die Strahlung der OCT-Kurzkohärenz-Lichtquelle 405 als auch für die fs-Laserstrahlung führen.

In einer weiteren, hier nicht gezeigten Ausführungsform ist die Achse der Strahlung der der OCT-Kurzkohärenz-Lichtquelle 405 nicht mit der optischen Achse 215 des Kurzpuls-Lasersystems 200 identisch, sondern weist dazu kleine Winkel auf. Dies hat den Vorteil, dass weitere für die Strahlformung der fs-Laserstrahlung zum Auge 900 hin notwendige optische Elemente kein OCT-Beleuchtungslicht in den OCT-Detektionsstrahlengang zurückwerfen und damit das OCT-Signal beeinträchtigen.

Um die Genauigkeit der Kalibrierung der OCT-Bildgebung zur Positionierung des Fokus der gepulsten Laserstrahlung zu verbessern, zeigt Fig. 7 einen konfokalen Detektor 260, dessen fokale Blende konjungiert zur Fokuslage der fs-Laserstrahlung liegt.

Dieser konfokale Detektor 260 erlaubt es, beim Scannen des Fokus der fs-Laserstrahlung in alle Raumrichtungen ebenfalls Strukturen des Auges zu messen.

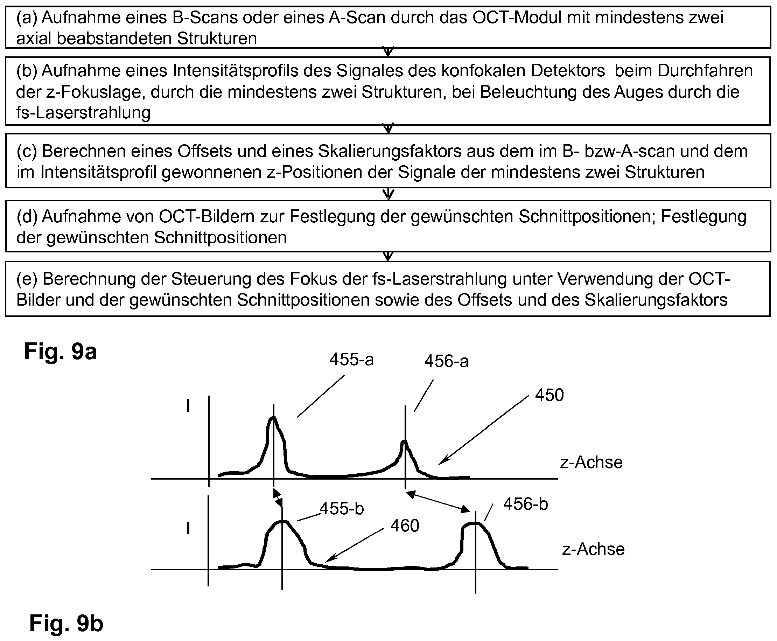

Daher kann in einem Kurzpuls-Lasersystem 200, welches sowohl einen konfokalen Detektor 260 als auch einen OCT-Detektor in einem OCT-Modul 400 enthält, vorteilhaft das folgende Verfahren der Steuerung des Kurzpuls-Lasersystems 200 für die Augenchirurgie durchgeführt werden, siehe auch Fig. 9a und 9b.
a) Aufnehmen eines B-Scans oder eines A-Scan 450 mittels eines OCT-Moduls 400, welcher mindestens zwei Strukturen 455-a und 456-a des Auges 900, z.B. der Cornea-Vorderseite und der Cornea-Rückseite zeigt.
b) Aufnahme eines Intensitätsprofils 460 des Signales des konfokalen Detektors 260 beim Durchfahren der z-Fokuslage durch dieselben zwei Strukturen 455-b und 456-b des Auges 900, bei Beleuchtung des Auges 900 durch die fs-Laserstrahlung.
c) Berechnen eines Offsets und eines Skalierungsfaktors aus den im B- bzw. A-scan und im Intensitätsprofil gewonnenen z-Positionen der Signale der entsprechenden zwei Strukturen
d) Aufnahme von OCT-Bildern zur Festlegung der gewünschten Schnittpositionen; Festlegung der gewünschten Schnittpositionen (anhand dieser OCT-Bilder)
e) Berechnung der Steuerung des Fokus der fs-Laserstrahlung unter Verwendung der OCT-Bilder und der gewünschten Schnittpositionen sowie des Offsets und des Skalierungsfaktors

Durch dieses Verfahren der Steuerung des Kurzpuls-Lasersystems 200 wird sichergestellt, dass die Unterschiede zwischen den Fokuslagen der mit dem konfokalen Detektor 260 ermittelten, durch die fs-Laserstrahlung beleuchteten Strukturen bzw. der mit dem OCT-Modul 400 ermittelten, gleichen Strukturen, z.B. durch unterschiedliche Wellenlängen oder unterschiedliche Aperturen, keine oder nur geringe Auswirkungen auf die Steuerung der fs-Laser-Schnitte und damit auf den Erfolg der Operation haben.

Um die Integration der verschiedenen Komponenten in einen für den Arzt optimierten Workflow und einer optimierten Arbeitsumgebung zu verbessern, wird in Fig. 7 weiterhin ein Aufbau offenbart, bei dem eine fs-Laserquelle 210, ein Strahlführungsmittel 230 zur Leitung der fs-Laserstrahlung über den Applikator-Kopf 220 auf das zu operierende Auge 900 und ein Swept-Source OCT-Modul 400, das eine OCT-Lichtquelle 405 und ein Interferometer enthält, durch ein Steuergerät 500, das niicht in der Fig. 7 gezeigt wird, gesteuert werden. Dabei enthält das Applikator-Kopf 220 ein lateral scannendes, bewegliches Objektiv 225. In einer Ausführungsvariante ist dabei die Kohärenzlänge der OCT-Lichtquelle 405 in Luft größer als 45mm, besonders bevorzugt größer als 60 mm.

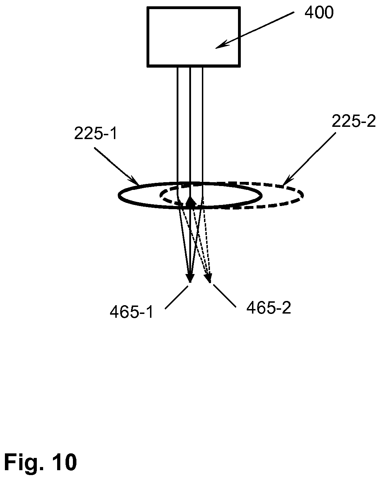

Durch die große Kohärenzlänge der OCT-Lichtquelle 405 wird es möglich, dass der gesamte Vorderkammerabschnitt innerhalb eines durch das Durchstimmen der Swept-Source-Quelle gegebenen A-Scans erfasst wird, selbst dann, wenn sich durch die laterale Objektivbewegung der optische Weg zum Auge 900 verlängert bzw. ändert, ohne dass die optische Weglänge des Referenzstrahlenganges z.B. durch Verschieben eines Referenzspiegels angepasst werden muss. Eine solche Veränderung der optischen Weglänge aufgrund einer Objektivbewegung ist in Fig. 10 dargestellt. Zwischen den Objektivpositionen 225-1 und 225-2 des Objektives 225 verschiebt sich entsprechend der Fokus zwischen den Positionen 465-1 und 465-2, was mit einer Änderung der optischen Weglänge des OCT-Beleuchtungsstrahlengangs einhergeht.

Um den Einfluss der Bewegung des Objektives 225 auf das OCT-Signal zu kompensieren, werden bei der Berechnung der A-Scans aus den OCT-Signalen die Weglängenunterschiede - typischerweise bis zu 6 mm bei unterschiedlichen Objektiv-Positionen - mit berücksichtigt. Dazu werden bei der Gewinnung der A-Scans aus den gemessenen OCT-Signalen, bei einem Aufbau gemäß Fig. 7, neben anderen Schritten auch folgende Schritte durchgeführt:
(1) Detektion der ersten OCT-Signale während des Durchstimmens der OCT-Lichtquelle 405 an Objektivposition 225-1
(2) Detektion der zweiten OCT-Signale während des Durchstimmens der OCT-Lichtquelle 405 an Objektivposition 225-2
(3) Fouriertransformation der ersten OCT-Signale zur Gewinnung des A-Scans; Fouriertransformation der mit einem von der Relativposition der Objektivposition 225-2 zur Objektivposition 225-1 abhängigen Phasenfaktors multiplizierten zweiten OCT-Signale.

Um den Einfluss der Bewegung des Objektives 225 auf das OCT-Signal zu kompensieren, werden bei einer alternativen Lösung die aus den OCT-Signalen gewonnenen A-Scans durch eine positionsabhängige Objektiv-Verschiebung entlang der Messachse korrigiert. Dazu werden folgende Schritte durchgeführt:
(1) Detektion der ersten OCT-Signale während des Durchstimmens der OCT-Lichtquelle 405 an Objektivposition 225-1
(2) Detektion der zweiten OCT-Signale während des Durchstimmend der OCT-Lichtquelle 405 an Objektivposition 225-2
(3) Fouriertransformation der ersten OCT-Signale zur Gewinnung eines ersten A-Scans
(4) Fouriertransformation der zweiten OCT-Signale zur Gewinnung eines zweiten A-Scans
(5) Verschieben des zweiten A-Scans entlang der Messachse um einen von der Relativposition der Objektivposition 225-2 gegenüber der Objektivposition 225-1 abhängigen Betrags.

### Schnittführung

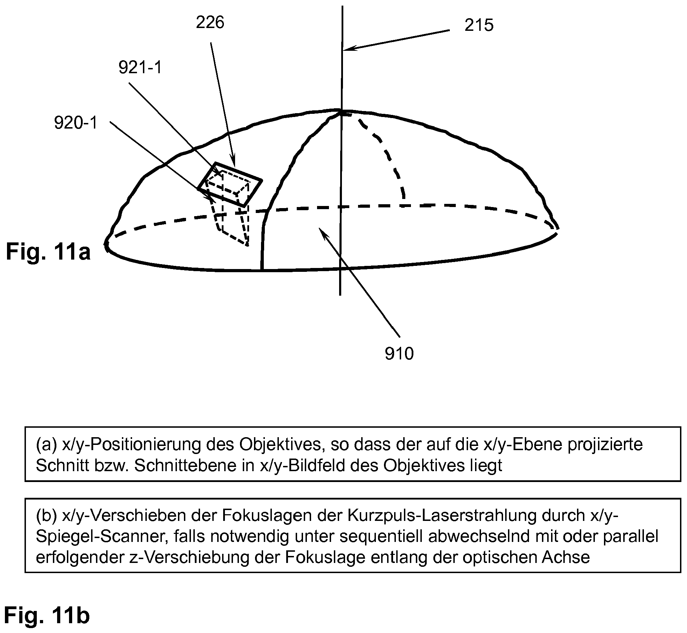

Der oben beschriebene Aufbau des Systems für die Kurzpuls-Laer-Augenchirurgie 100 und des Kurzpuls-Lasersystems 200 unterstützt in besonderer Weise das folgende Verfahren der Laser-Schnittführung, das in der Fig. 11a und 11b dargestellt ist:
Sind die auszuführenden Schnitte 920-1 im Gewebe 910 eines Auges 900 in ihren jeweils auf die x- und y-Achse projizierten Fokuslagen 921-1, also der in eine x/y-Ebene projizierte Schnitt 920-1 kleiner als das Bildfeld 226 des Objektivs 225, z.B. für kleine und steile Zugangsschnitte, wie in Fig. 11a gezeigt, so wird für das Verfahren der Laser-Schnittführung folgende Schritte gewählt, siehe auch Fig. 11b:
(1) x/y-Positionierung des Objektives 225, derart, dass die zu projizierten x/y-Fokuslagen, also die Positionen des jeweiligen Fokus der fs-Laserstrahlung in x und y, innerhalb des Bildfeldes 226 liegen.
(2) Projizieren der Fokuslagen des Schnittmusters durch das in seiner x/y-Lage feststehende Objektiv 225 unter Verwendung des x/y-Scansystems, hier also der x/y-Spiegel-Scanner 240 ggf. unter nach jedem x/y-Scan erfolgender oder parallel zu dem x/y-Scan erfolgender Ablenkung der Fokuslage mittels der Divergenz ändernden Linse bzw. des Divergenz variierenden Linsensystems 211 entlang der optischen Achse 215. Dies ist z.B. für kleine und steile Zugangsschnitte eine bevorzugte Lösung.

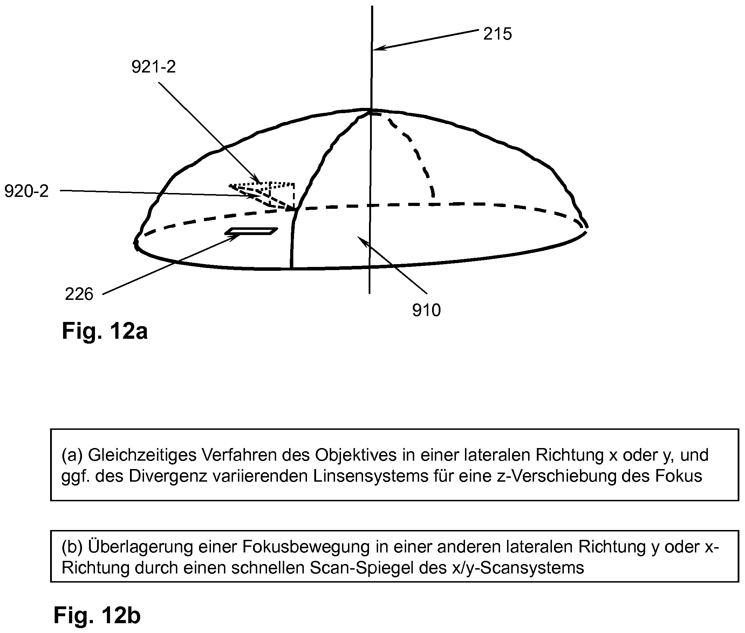

Besteht aus applikativen Gründen die Notwendigkeit, größere Schnitte 920-2 bzw. flachere Schnitte 920-2 mit größerer projizierter x- und/oder y-Ausdehnung durchzuführen als vom Bildfeld 226 des Objektivs 225 gleichzeitig erfassbar, siehe Fig. 12a, dann kann das Objektiv 225 jeweils soweit verschoben werden, dass die neuen Bildfelder und das Bildfeld 226 der ursprünglichen ObjektivPosition den gesamten Schnittbereich 920-2 abdecken. Für das jeweils neue Bildfeld können die noch fehlenden Schnittteile mittels des x/y-Scansystems, hier also der x/y-Spiegel-Scanner 240, ggf. unter Änderung der z-Lage der Fokusposition der fs-Laserstrahlung, nachgeholt werden. D.h. es wird bei sukzessiv anderen lateralen Positionen des Objektives 225 gearbeitet, bis der gesamte Schnitt 920-2 durch die verschiedenen Teilscanfelder erfolgt ist. Die x/y-Spiegel-Scanner 240 für die Teilfelder erlauben dabei, in einfacher Weise größere Felder systematisch und präzise in Teilfeldern, besonders bevorzugt in quadratischen Teilfeldern, mittels einer schrittweisen Objektiv-Bewegung abzufahren.

Für den Fall, dass die Schnitte 920-2 sehr flach im Gewebe 910, z.B. der Cornea, liegen, d.h. die projizierte y-Ausdehnung der in eine x/y-Ebene projizierten Fokuslagen 921-2 des Schnitts 920-2 nicht mit einem y-Scanner bei feststehendem Objektiv 225 vollständig erreicht werden kann, weil das Bildfeld 226 des Objektives 225 zu klein ist, während die Schnittlänge entlang der x-Achse innerhalb des Bildfeldes 226 liegt, dann kann alternativ zum obigen Teilfeld-Scan folgende Methode gewählt werden, siehe auch Fig. 5b:
(1) gleichzeitiges Verfahren des Objektives in y-Richtung und Verfahren der Linse oder des Linsensystems zur Verstellung der z-Fokusposition 211 und
(2) Überlagerung der schnellen x-Spiegel-Scanner-Bewegung.

Sind die Schnitte 920-2 eher lang und steil, weisen also eine große Ausdehnung entlang der x-Achse auf eine geringe Ausdehnung in y-Richtung, dann ist es entsprechend vorteilhaft, diese Schnitte durch gleichzeitiges Verfahren des Objektivs 225 entlang der x-Achse und Verfahren der Linse zur Verstellung der z-Fokusposition 211 unter Überlagerung der schnellen y-Scanner-Bewegung auszuführen.

Verallgemeinert auf einen Schnitt 920-2, dessen projizierte x- und y-Ausdehnung der in eine x/y-Ebene projizierten Fokuslagen 921-2 sowohl in x- als auch in y-Richtung größer ist als das Bildfeld 226 des Objektivs 225, ist dieses Verfahren natürlich auch in beide Richtungen anwendbar.

Während bei obiger Beschreibung die z-Positionierung mittels Divergenz variierender Linse bzw. Divergenz variierendem Linsensystem 211 erfolgt, gilt die obige Beschreibung der Schnittführung generell für jede Art der z-Fokus-Verstellung, z.B. auch wenn die Lage des z-Fokus durch Positionierung bzw. Bewegung des Objektives 225 im Applikator-Kopf 220 entlang der optischen Achse 215 erfolgt.

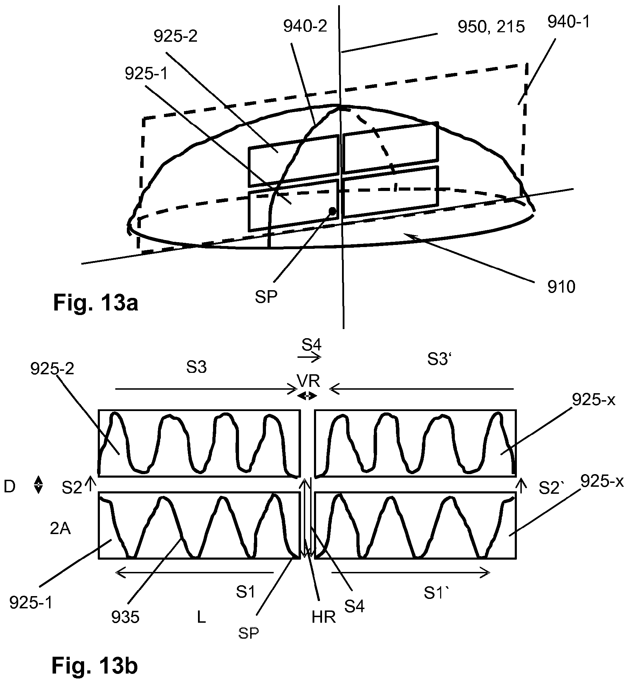

Um für die Zertrümmerung der Linse 910 möglichst wenige fs-Bearbeitungspulse zu setzen und dabei jedoch noch sicherzustellen, dass bei der späterer Phakoemulsifikation keine oder nur geringe Ultraschallenergie angewendet werden muss, wird in Fig. 13a und 13b ein Verfahren bzw. ein Schnittmuster skizziert, welches die Linse 910 eines Auges 900 in ihrer Struktur schwächt. Das Verfahren der Schnittführung umfasst dabei folgende Schritte:
(S0) Positionieren des Fokuspunktes SP einer Kurzpuls-Laserstrahlung, im Beispiel einer fs-Laserstrahlung, in die zu bearbeitende Linse 910 eines Auges 900. In einer Ausführungsvariante wird der Fokuspunkt SP in der Linse 910 mit einem Sicherheitsabstand zum posterioren und anterioren Kapselsack des Auges 900 positioniert.
(S1) Vorschub des Objektives 225 in radialer Richtung in einer Meridianebene 940-1 der Linse 910 für eine Länge L mit Überlagerung einer oszillierenden Fokus-Verschiebung 935 entlang der optischen Achse 215, 950 sowohl des fs-Lasersystems als auch des Auges mit einer Amplitude A, wobei in einer ersten Variante nur bei der posterioren zur anterioren Fokusbewegung Laserpulse und in einer zweiten Variante über den ganzen Zyklus hinweg Laserpulse der fs-Laserstrahlung in das Auge 900 abgegeben werden. Die Meridianebene 940 der Linse 910 ist dabei gegeben durch eine Ebene, die durch das Zentrum der Linse 910 in der Nähe der optischen Achse 950 der Linse 910 oder der optischen Achse 215 des fs-Lasersystems 200 geht und annähernd parallel zur optischen Achse 215, 950 läuft. Dadurch wird eine Schnittfläche 925-1 geschaffen. In einer dritten Variante, basierend auf Variante eins, wird die anterioren zu posterioren Fokusbewegung schneller, d.h. unter geringerer Vorschubdistanz, also geringer lateraler Bewegung, als die Vorschubdistanz bei posteriorer zu anteriorer Fokusbewegung durchgeführt. Dies führt bei konstanter Laserpulsfrequenz dazu, dass die Laserpulse zwischen zwei Aufwärtsbewegungen lateral enger beieinander liegen und eine homogenere Schnittfläche 925-1 entsteht.
(S2) Vorschub des Fokuspunktes der fs-Laserstrahlung entlang der optischen Achse 215, 950 um eine Höhe HR, wobei HR so gewählt wird, dass die im nachfolgenden Schritt gesetzten Foki der Laserpulse sich nicht mit denen aus dem vorherigen Schritt gesetzten Foki der Laserpulse überlappen. In einer Variante wird zwischen den Laserfoki der beiden Schnittflächen 925-1, 925-2 ein Abstand D von 10-50µm eingehalten. Zum einen stellt dieser positive Abstand sicher, dass nicht in entstehende Kavitationsblasen des posterioren Schnittes 925-1 noch weitere, dann unnötige, Schnitte gesetzt werden. Zum anderen sind auch keine Schnitte in diesem Abstandsbereich notwendig, da die Blasenbildung zu einer ausreichend großen Schwächung in dem Gewebe 910 führt und die beiden Schnittflächen 925-1 und 925-2 miteinander ggf. sogar verschmelzen.
(S3) Wiederholen des Schrittes S1 unter Umkehrung der Vorschubrichtung in radialer Richtung und ggf. des Schrittes S2(was in Fig. 13b aufgrund der in diesem Beispiel vorliegenden Größenverhältnisse nicht nötig ist). In einer Ausführungsvariante werden diese Schritte S1 und S2 wiederholt bis ein Sicherheitsabstand zum posterioren Kapselsack erreicht ist. In einer Variante werden zur vollständigen Schwächung der Linse weitere ergänzende Schritte unternommen, beispielsweise durch gleichzeitige, überlagerte Verschiebung der Laserfokusablage mittels schnellem lateralen Scanner während der Durchführung der Schnitte S1 und S2.
(S4) Vorschub des Fokuspunktes radial in der Meridianebene 940 um eine Länge VR und entlang der optischen Achse 215, 950 um die Länge HR, sodass die in den folgenden Schritten entstehenden Schnitte 925-x sich nicht radial mit den vorhergehenden Schnitten 925-1, 925-2, etc. überlappen oder in einer Ausführungsvariante einen radialen Abstand D, mit bevorzugt D zwischen 10-50µm aufweisen. In Fig. 13b sind exemplarisch mögliche Längen VR und HR angegeben, jedoch sind auch andere Längen wählbar, solange sich die noch zu erzeugenden Schnitte 925-x nicht mit den vorhergehenden Schnitten 925-1, 925-2, etc. überlappen. Wiederholen der Schritte S1-S4 (siehe S1', S2', S3') solange bis die Linse 910 in der gesamten Medianebene 940 mit Schnitten 925-1, 925-2, ... 925-x, in einer Ausführungsvariante abgesehen von einem Mindestabstand zum Kapselsack und zur Iris, durchsetzt ist.
(S5) Positionieren des Fokuspunktes an den Rand einer weiteren Meridianebene 940-2. In einer bevorzugten Ausführungsvariante findet der Wechsel der Meridianebenen 940-1, 940-2, ... im Bereich der Schnittlinie der Meridianebenen 940-1, 940-2, ... statt.
(S6) Wiederholen der Schritte S1-S6 solange bis die gesamte Linse 910 mit Schnitten 925-1, 925-2, ... 925-x durchsetzt ist.

Neben diesem Schnittmuster entlang der Medianebenen 940-1, 940-2, ... sind über das Positionieren des Grund-Schnittmusters von Schritt (S1)-(S3) verschiedene Gesamtschnittmuster realisierbar. So lassen sich auch Gitterebenen realisieren, die mit Schnittflächen durchsetzt sind. Bei allen dreidimensionalen Mustern an Schnitten 925-1, 925-2, ... 925-x lässt sich über den Abstand der Schnittflächen 925-1, 925-2, ... 925-x in Verbindung mit der Blasenbildung der Grad der Schwächung des Linsenzusammenhaltes einstellen. Auch der Abstand des in Schritt S2 beschriebenen Schnittes 925-2 zu den anderen vergleichbaren Schnitten 925-1, 925-2, ... 925-x gemäß Schritt S2 kann in allen drei Raumrichtungen dem gewünschten Grad der Schwächung angepasst werden.

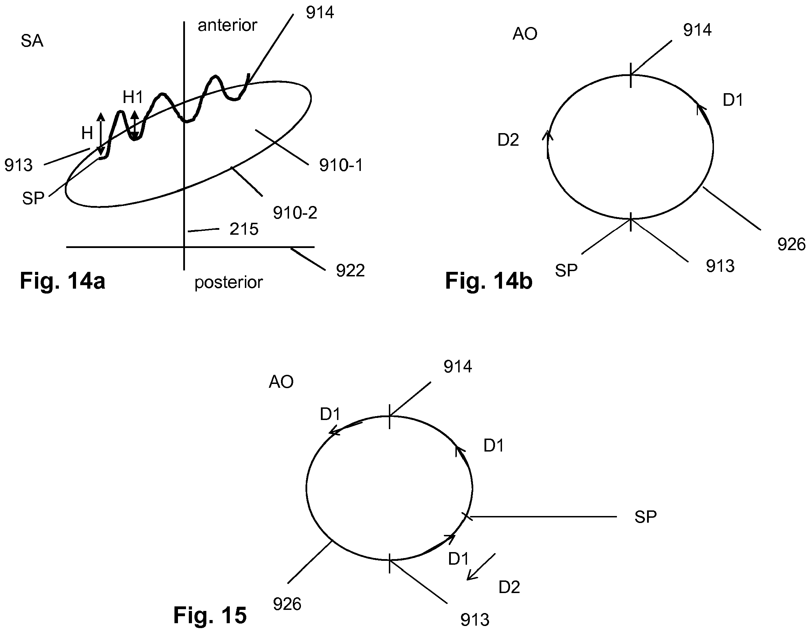

Um ein effizientes Schneiden des Kapselsackes 910-2 bei der Kapsulotomie für ein schnelles z-scannendes System zu erlauben, wird in Fig. 14a ein Verfahren der Fokusführung bei gegenüber der optischen Achse 215 des Lasersystems geneigter Augenlinse 910-1 in Seitenansicht SA und in Fig. 14b in Aufsicht von oben AO beschrieben:
(1) Anfahren des zu schneidenden Kapselsacks 910-2 in eine x/y/z-Fokusposition SP, die eine Startposition vorgibt, hinter der posteriorsten Stelle 913 des Bereiches des Kapselsackes 910-2, den es für die Kapsulotomie mit dem Laser zu schneiden gilt.
(2) Vorschub des Fokuspunkts bzw. Fokus der fs-Laserstrahlung entlang der z-Achse 215 anterior, um insgesamt die Strecke H unter gleichzeitigem Vorschub des Fokuspunktes der fs-Laserstrahlung in einer x/y-Ebene 922 entlang der auf die x/y-Ebene 922 projizierten Randes der Kapsulotomie 926 in Richtung D1, wobei der Fokuspunkt nach Durchfahren der Strecke H anterior des Kapselsackes 910-2 liegt.
(3) Vorschub des Fokuspunktes entlang der z-Achse 215 posterior um insgesamt die Strecke H1 unter gleichzeitigem Vorschub des Fokuspunktes in x/y-Ebene 922entlang der auf die x/y-Ebene 922 projizierten Bahn in Richtung D1, wobei H1 kleiner als H ist, und der Fokus nach dem Durchfahren der Strecke H1 posterior des Kapselsackes 910-2 liegt.
(4) Wiederholen der Schritte (2) und (3), bis die anteriorste Stelle 914 des Bereiches des Kapselsackes 910-2, den es für die Kapsulotomie 926 mit dem der gepulsten fs-Laserstrahlung zu schneiden gilt, erreicht ist.
(5) Für die Vervollständigung der Kapsulotomie 926 werden die obigen Schritte unter Vorschub des Fokuspunktes der fs-Laserstrahlung in x/y-Ebene 922 entlang der auf die x/y-Ebene 922 projizierten Bahn in gegengesetzter Richtung D2 wiederholt (siehe Fig. 14b).

Bei wenig geneigten Linsen 910-1 und Wahl eines größeren Vorschubs in der x/y-Ebene 922 kann der Einfluss der Blasenbildung von vorherigen Laserimpulsen auf den zu setzenden Puls vernachlässigt werden. Dann ist folgende Schrittfolge vorteilhaft, siehe Fig. 15 als Ansicht von oben AO:
(1) Anfahren des zu schneidenden Kapselsackes 910-2 in eine x/y/z-Fokusposition SP posterior zu der Stelle des Bereiches 926 des Kapselsackes, die es für die Kapsulotomie mit dem Laser zu schneiden gilt.
(2) Vorschub des Fokuspunkt der fs-Laserstrahlung entlang der z-Achse anterior um insgesamt die Strecke H unter gleichzeitigem Vorschub des Fokuspunktes in x/y-Ebene 922 entlang des auf die x/y-Ebene 922 projizierten Randes der Kapsulotomie 926 in Richtung D1, wobei der Fokuspunkt nach Durchfahren der Strecke H anterior des Kapselsackes 910-2 liegt.
(3) Vorschub des Fokuspunktes entlang der z-Achse 215 posterior um insgesamt die Strecke H1 unter gleichzeitigem Vorschub des Fokuspunktes in x/y-Ebene 922 entlang der auf die x/y-Ebene 922 projizierten Bahn der Kapsulotomie 926 in Richtung D1, wobei in einer ersten Ausführungsvariante H1 kleiner als H ist, und der Fokuspunkt nach Durchfahren der Strecke H1 posterior des Kapselsackes 910-2 liegt.
(4) Wiederholen der Schritte (2) und (3) bis die anteriorste Stelle 914 des Bereiches 926 des Kapselsackes 910-2, den es für die Kapsulotomie mit dem Laser zu schneiden gilt, erreicht ist.
(5) Wiederholen der Schritte (2) und (3) unter Beibehaltung des Vorschubs in der x/y-Ebene 922 in Richtung D1, wobei in der ersten Ausführungsvariante jedoch H1 größer als H ist und solange, bis der posteriorste Punkt 913 des Bereiches 926 des Kapselsackes 910-2, den es mit dem Laser zu schneiden gilt, erreicht ist.
(6) Wiederholen der Schritte (2) und (3) unter Beibehaltung des Vorschubs in der x/y-Ebene 922 in Richtung D1, wobei in der ersten Ausführungsvariante jedoch H1 nun wieder kleiner als H ist und solange bis die Kapsulotomie 926 im Punkt SP geschlossen ist.

In einer zweiten Ausführungsvariante kann bei wenig geneigten Linsen 910-1 und der Wahl eines größeren Vorschubes in x/y-Ebene 922 auch die Schnittbahn der Kapsolutomie 926 in entgegengesetzter Richtung D2 durchlaufen werden. Siehe ebenfalls Fig. 15:
(1) Anfahren des zu schneidenden Kapselsackes 910-2 in einer x/y/z-Fokusposition SP posterior zu der Stelle des Bereiches 926 des Kapselsackes, den es für die Kapsulotomie mit dem Laser zu schneiden gilt.
(2) Vorschub des Fokus der fs-Laserstrahlung oder einer anderen Kurzpuls-Laserstrahlung entlang der z-Achse 215 anterior um insgesamt die Strecke H unter gleichzeitigem Vorschub des Fokuspunktes in der x/y-Ebene 922 entlang der auf die x/y-Ebene 922 projizierten Randes der Kapsulotomie 926 in Richtung D2, wobei der Fokuspunkt nach dem Durchfahren der Strecke H anterior des Kapselsackes 910-2 liegt.
(3) Vorschub des Fokuspunktes entlang der z-Achse 215 posterior um insgesamt die Strecke H1 unter gleichzeitigem Vorschub des Fokuspunktes in der x/y-Ebene 922 entlang der auf die x/y-Ebene 922 projizierten Bahn der Kapsulotomie 926 in Richtung D2, wobei H1 größer als H ist und der Fokuspunkt nach dem Durchfahren der Strecke H1 posterior des Kapselsackes 910-2 liegt.
(4) Wiederholen der Schritte (2) und (3) bis die posteriorste Stelle 913 des Bereiches 926 des Kapselsackes, den es für die Kapsulotomie mit dem Laser zu schneiden gilt, erreicht ist.
(5) Wiederholen der Schritte (2) und (3) unter Beibehaltung des Vorschubs in der x/y-Ebene 922 in Richtung D2, wobei in der zweiten Ausführungsvariante jedoch H1 kleiner als H ist, und solange bis die anteriorste Stelle 914 des Bereiches des Kapselsacks 910-2, den es mit dem Laser zu schneiden gilt, erreicht ist.
(6) Wiederholen der Schritte (2) und (3) unter Beibehaltung des Vorschubs in der x/y-Ebene 922 in Richtung D2, wobei jedoch H1 nun wieder größer als H ist und solange bis die Kapsulotomie 926 im Punkt SP geschlossen ist.

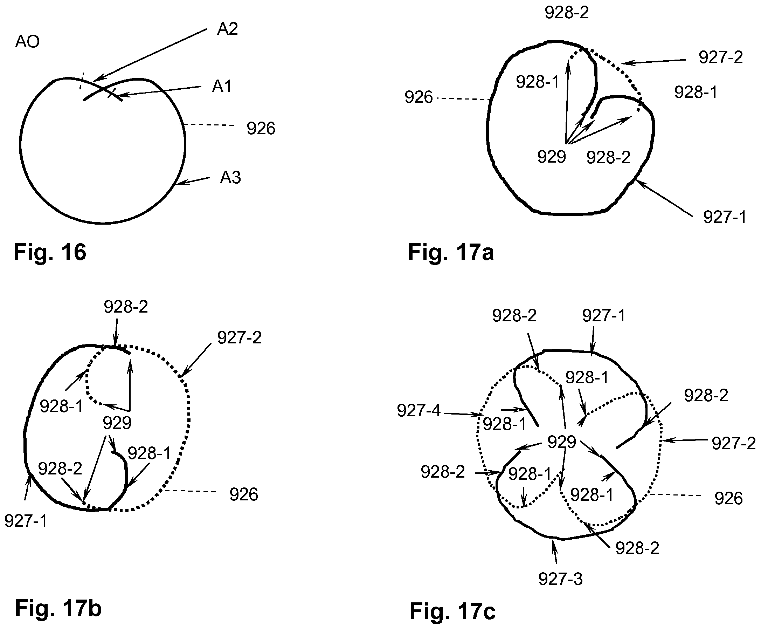

Sowohl bei geneigten als auch gegenüber der Achse 215 senkrecht stehenden Linsen 910-1 kann das Schneiden der Kapsulotomie 926 bei fs-Lasersystemen 200 mit schnellem z-Scan, d.h. einer Geschwindigkeit der z-Fokusablenkung die größer als oder vergleichbar der Geschwindigkeit der x/y-Fokusablenkung ist, einige Zeit in Anspruch nehmen, sodass die relative Bewegung des Auges 900 sich gegenüber der optischen Achse 215 des Lasersystems ändert. Folgende Schritte eines Verfahrens zur Laserfokussteuerung bei einer Kapsulotomie 926, siehe auch Fig. 16 als Ansicht von oben AO, gewährleisten selbst bei leichten Bewegungen des Kapselsackes 910-2 in der x/y-Ebene 922, aber auch in der z-Achse 215, dass ein noch vollständig vom übrigen Kapselsack 910-2 getrenntes Kapselsacksegment entsteht:
(1) Positionieren des Fokus der fs-Laserstrahlung in der x/y-Ebene 922 und bzgl. z-Position in der Nähe des Kapselsacks 910-2.
(2) Führen des Fokuspunktes bzw. Fokus in der x/y-Ebene 922, entlang des Randes des Bereiches des Kapselsackes 910-2, den es zu schneiden gilt, in einem ersten Abschnitt A1 mit einem ersten Radius R1
(3) Führen des Fokus in der x/y-Ebene in einem zweiten Abschnitt A2 mit einem zweiten Radius R2
(4) Führen des Fokus in der x/y-Ebene 922 in einem dritten Abschnitt A3 mit einem dritten Radius R3, wobei der erste Radius R1 und der dritte Radius R3 kleiner als der zweite Radius R2 ist, derart, dass die Bahn des Fokuspunkts, die durch das Setzen einzelner Pulse entsteht den ersten Abschnitt A1 oder den zweiten Abschnitt A2 durchschneidet.

Dabei wird in den Schritten 2, 3 und 4 die z-Position des Fokus der fs-Laserstrahlung oszillierend mit einer so großen Oszillationsamplitude geändert, dass die während der Oszillationen gesetzten Laserpulse durch Photodisruptions-Prozesse den Kapselsack 910-2 durchschneiden.

Bei den Schritten 2, 3 und 4 wird zeitgleich zu dem Führen des Fokus in der x/y-Ebene 922 auch noch in oszillierender Weise mindestens einmal, bevorzugt mehrmals, besonders bevorzugt mehr als fünf Mal, für jeden der Abschnitte A1, A2 und A3 die z-Fokuslage periodisch geändert.

Bei Systemen mit schnellem x/y-Scansystemen 240 zum Schneiden der Kapsulotomie 926, d.h., x/y-Scansystemen 240, deren Geschwindigkeit der x/y-Fokusablenkung größer als die Geschwindigkeit der z-Fokusablenkung ist, tritt das Problem des Treffens der des dritten Abschnitts A3 des Kapsulotomie-Schnittes mit dem ersten Abschnitt A1 des Kapsulotomie-Schnittes in der x/y-Ebene 922 aufgrund der hohen Bewegungsgeschwindigkeit des Fokus in der x/y-Ebene 922 nicht in dem Maße auf, wie bei schnellen z-scannenden Systemen.

Die bisher beschriebene einfache Schnittgeometrie bzw. Strahlführungsgeometrie stellt insbesondere bei langsamer lateraler Scanbewegung in x- und/oder y-Richtung in Bezug zur Augenbewegung nicht sicher, dass die Kapsulotomie annähernd kreisförmig erfolgt.Bei signifikanter Augenbewegung kann es nun vorkommen, dass der dritte Abschnitt A3 den ersten Abschnitt A1 des Kapsulotomie-Schnittes gleich zu dessen Beginn trifft und damit eine erhebliche Eindellung und damit Abweichung von einer annähernd runden Kapsulotoie 926 entsteht, wie in der Fig. 16 gezeigt. Eine kreisförmige Kapsulotomie ist jedoch zur verbesserten Zentrierung der später in den Kapselsack 910-2 eingesetzten Intraokularlinse (IOL) für viele IOL-Typen vorteilhaft.
Daher werden als Alternativen die in den Fig. 17a bis 17c vorgeschlagenen Schnittgeometrien offenbart, bei denen statt eines durchgehenden Schnitts mindestens zwei zeitlich getrennt durchgeführte Schnitte in Form von nicht geschlossenen Kurven 927 erfolgen, wobei jeder der Endbereiche 928-1, 928-2 der nicht geschlossenen Kurven innerhalb der sich später ergebenden Kapsulotomie 926, also innerhalb der kreisförmigen Öffnung, angeordnet ist.

So kann, wie in den Fig. 17a und 17b gezeigt, in einem Verfahren zur Schnittführung einer Kapsulotomie 926 mittels eines Kurzpuls-Lasersystems 200 für die Augenchirurgie eine Öffnung dadurch erzeugt werden, dass Fokuspunkte einer Kurzpuls-Laserstrahlung mittels eines x/y-Scansystems 240 in ihren x- und y-Fokuslagen positioniert werden, und dabei in zwei Schritten eine erste nicht geschlossene Kurve 927-1 mit einem Radius R und eine zweite nicht geschlossene Kurve 927-2 mit einem Radius R und jeweils mit ersten und einem zweiten Endbereich 928-1, 928-2 einer jeweils gleichgerichteten Wölbung entsteht,
wobei der erste Endbereich 928-1 der ersten nicht geschlossenen Kurve 927-1 einen ersten Endbereichsradius R_{E11} und der zweite Endbereich 928-2 der ersten nicht geschlossenen Kurve 927-1 einen zweiten Endbereichsradius R_{E12} aufweist und sowohl der erste Endbereichsradius R_{E11} als auch der zweiten Endbereichsradius R_{E12} kleiner als der Radius R ist und der erste Endbereich 928-1 der zweiten nicht geschlossenen Kurve 927-2 einen ersten Endbereichsradius R_{E21} und der zweite Endbereich 928-2 der zweiten nicht geschlossenen Kurve 927-1 einen zweiten Endbereichsradius R_{E22} aufweist und sowohl der erste Endbereichsradius R_{E21} als auch der zweiten Endbereichsradius R_{E22} kleiner als der Radius R ist, alle Endbereiche 927-1, 927-2 je ein Ende 929 aufweisen, und der erste Endbereich 928-1 der zweiten nicht geschlossenen Kurve 927-2 den zweiten Endbereich 928-2 der ersten nicht geschlossenen Kurve 927-1 sowie der zweite Endbereich 928-2 der zweiten nicht geschlossenen Kurve 927-2 den ersten Endbereich 928-1 der ersten nicht geschlossenen Kurve 927-1 so schneidet, dass die Enden 929 aller Endbereiche 928-1, 928-2 im Inneren einer durch die erste und die zweite nichtgeschlossenen Kurve 927-1, 927-2 gebildeten geschlossenen Kurve der Kapsulotomie 926 angeordnet sind.

Auch kann in vorteilhafter Weise, wie in der Fig. 17c gezeigt, in einem Verfahren zur Schnittführung einer Kapsulotomie mittels eines Kurzpuls-Lasersystems 200 für die Augenchirurgie, eine Öffnung des Kapselsacks 910-2 dadurch erzeugt werden, dass Fokuspunkte einer Kurzpuls-Laserstrahlung mittels eines x/y-Scansystems 240 in ihren x- und y-Fokuslagen positioniert werden, und dabei in vier Schritten eine erste, eine zweite, eine dritte und eine vierte nicht geschlossene Kurve 927-1, 927-2, 927-3, 927-4 mit einem Radius R und jeweils mit ersten und einem zweiten Endbereich 928-1, 928-2 einer jeweils gleichgerichteten Wölbung entsteht, wobei der erste Endbereich 928-1 der ersten nicht geschlossenen Kurve 927-1 einen ersten Endbereichsradius R_{E11} und der zweite Endbereich 928-2 der ersten nicht geschlossenen Kurve 927-1 einen zweiten Endbereichsradius R_{E12} aufweist, der erste Endbereich 928-1 der zweiten nicht geschlossenen Kurve 927-2 einen ersten Endbereichsradius R_{E21} und der zweite Endbereich 928-2 der zweiten nicht geschlossenen Kurve 927-2 einen zweiten Endbereichsradius R_{E22} aufweist, der erste Endbereich 928-1 der dritten nicht geschlossenen Kurve 927-3 einen ersten Endbereichsradius R_{E31} und der zweite Endbereich 928-2 der dritten nicht geschlossenen Kurve 927-3 einen zweiten Endbereichsradius R_{E32} aufweist und der erste Endbereich 928-1 der vierten nicht geschlossenen Kurve 927-4 einen ersten Endbereichsradius R_{E41} und der zweite Endbereich 928-2 der vierten nicht geschlossenen Kurve 927-4 einen zweiten Endbereichsradius R_{E42} aufweist und alle Endbereichsradien R_{E11}, R_{E12}, R_{E21}, R_{E22}, R_{E31}, R_{E32}, R_{E41} und R_{E42} kleiner als der Radius R sind, wobei alle Endbereiche 928-1, 928-2 je ein Ende 929 aufweisen, und der erste Endbereich 928-1 der zweiten nicht geschlossenen Kurve 927-2 den zweiten Endbereich 928-2 der ersten nicht geschlossenen Kurve 927-1, der erste Endbereich 928-1 der dritten nicht geschlossenen Kurve 927-3 den zweiten Endbereich 928-2 der zweiten nicht geschlossenen Kurve 927-2, der erste Endbereich 928-1 der vierten nicht geschlossenen Kurve 927-4 den zweiten Endbereich 928-2 der dritten nicht geschlossenen Kurve 927-3 sowie der zweite Endbereich 928-2 der vierten nicht geschlossenen Kurve 927-4 den ersten Endbereich 928-1 der ersten nicht geschlossenen Kurve 927-1 so schneidet, dass die Enden aller Endbereiche 928-1, 928-2 im Inneren einer durch die erste, die zweite, die dritte und die vierte nichtgeschlossenen Kurve 927-1, 927-2, 927-3, 927-4 gebildeten geschlossenen Kurve der Kapsulotomie 926 angeordnet sind.

Wird der Gesamtschnitt der Kapsulotomie 926, wie hier beschrieben und auch mit einer höheren Anzahl nichtgeschlossener Kurven 927-1 ... 927-n ausführbar, auf mehrere getrennt durchgeführte Schnitte verteilt, so führt dies zu einer kürzeren Schnittlänge für jede der nicht geschlossenen Kurven 927-1 ... 927-n. Bei gegebener lateraler Scangeschwindigkeit in x- und/oder y-Richtung führt dies zu einer kürzeren Schnittdauer für eine einzelne nicht geschlossene Kurve 927-1 ... 927-n. Während dieser kürzeren Schnittdauer führen die Augenbewegungen zu einer geringeren Abweichung von einer kreisförmigen Schnittkurve für jeden der getrennt durchgeführten Schnitte einer nicht geschlossenen Kurve 927-1 ... 927-n. Vor der Durchführung eines nächsten Schnittes, kann dieser lateral neu ausgerichtet werden. Selbst ohne eine solche Neuausrichtung ist diese Schnittgeometrie insbesondere, aber nicht nur, für solche Kurzpuls-Lasersysteme 200 vorteilhaft, deren Scansystem bzw. Scansysteme den Fokus einer Kurzpuls-Laserstrahlung zum Durchschneiden des Kapselsackes schneller entlang der optischen Achse 215 bewegt als in lateraler Richtung, oder die einen lateralen Teilfeldscanner aufweisen, da mechanische Toleranzen der lateralen Scanner bzw. der Scannerführung des Kurzpuls-Lasersystems 200 besser kompensiert werden können.

Für jedes Paar sich schneidender nicht geschlossener Kurven 927-n-1, 928-n gilt also: Der Schnittpunkt liegt in einem zweiten Endbereich 928-2 einer nicht geschlossener Kurve 927-(n-1) und einem ersten Endbereich 928-1 einer folgenden nicht geschlossener Kurve 927-n und die Krümmungsradien der Endbereiche R_{En1}, R_{En2} sind kleiner als der Radius R der nicht geschlossenen Kurven 927-n-1, 928-n, der den Krümmungsradius des Zentralbereichs einer nicht geschlossenen Kurve 927-1, ...928-n zwischen den beiden Endbereichen 928-1, 928-2 beschreibt.

Die Forderung, dass die Krümmungsradien der Endbereiche R_{En1}, R_{En2} kleiner sein sollen als der Radius R beinhaltet dabei jedoch auch den Fall, dass sich die Krümmungsradien der Endbereiche R_{En1}, R_{En2} von unten dem Krümmungsradius R nähern, also R_{En1}, R_{En2} → R. Alle nicht geschlossenen Kurven 927-n-1, 928-n weisen in ihrem Zentralbereich den Radius R auf. Kleinere, also nichtsignifikante Abweichungen zwischen den Radien R zweier nicht geschlossenen Kurven 927-n-1, 928-n sind jedoch möglich, ohne das Ziel zu verfehlen, durch das Zusammenwirken der nicht geschlossenen Kurven in oben beschriebener Art und Weise eine geschlossene Kurve 926 zu erzeugen, die etwa kreisförmig mit einem Radius R ausgestaltet ist, und somit die Forderungen für einen Kapsulotomie-Schnitt erfüllt. Auch kann die Ausdehnung der Endbereiche 928-1, 928-2 zwischen zwei nicht geschlossenen Kurven 927-n-1, 928-n variieren.

Für einen Teilfeldscanner ist es dabei besonders vorteilhaft, wenn die Anzahl der getrennten Schnitte, also die Anzahl der der nicht geschlossenen Kurven 927-1, ...928-n, mit der Anzahl der nötigen Teilfelder zur Überdeckung der Gesamtfläche einer Kapsulotomie 926 übereinstimmt.

### Patienten-Schnittstelle / Kontaktglas

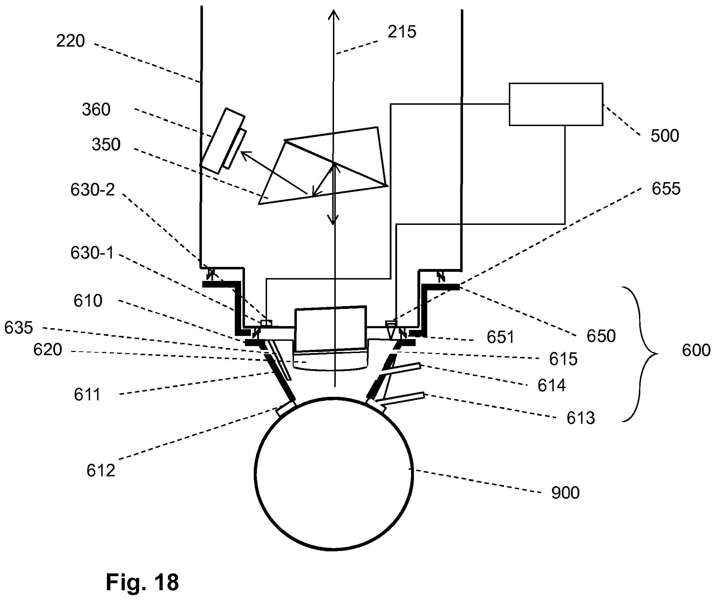

Um den Workflow für den Bediener möglichst einfach zu gestalten, ist - für die aus optischen Gründen notwenige Patientenschnittstelle 600, die ein Kontaktglas 610 enthält - der in Fig. 18 gezeigte Aufbau zur Bearbeitung des Auges 900 mittels eines Systems für die Kurzpuls-Laser-Augenchirurgie 100 gezeigt. Der hier gezeigte Aufbau beinhaltet eine Patienten-Schnittstelle 600, einen Applikator-Kopf 220 des Systems für die Kurzpuls-Laser-Augenchirurgie 100, wobei die Patienten-Schnittstelle 600 in Fig. 18 sowohl am Auge 900 des Patienten als auch am Applikator-Kopf 220 des Systems für die Kurzpuls-Laser-Augenchirurgie 100 fixiert ist und somit die relative Lage des Auges 900 zum Systems für die Kurzpuls-Laser-Augenchirurgie 100 und folglich auch zum Strahlengang der Kurzpuls-Laserstrahlung fixiert.

Die Patienten-Schnittstelle 600 enthält ein Kontaktglas 610, das im hier gezeigten Ausführungsbeispiel als Flüssigkeits-Interface ausgestaltet ist. Das Kontaktglas 610 ist einteilig, aus einem vorzugsweise einheitlichen, transparenten Material gefertigt und enthält einen Saugring 612, einem Mantel 611 und ein optisches Element 620 an der Oberseite des Mantels 611. Es umfasst weiterhin ,
zwei Öffnungen 613,614, an die zwei Zuleitungen über Fixierungshilfen angeschlossen sind bzw. den Anschluss von zwei Zuleitungen erlauben, wobei je eine Zuleitung an eine der Öffnungen 613, 614 angeschlossen ist oder wird.

Ein einteiliges Kontaktglas 610, bei dem alle Funktionselemente integriert sind, erlaubt eine einfachere Handhabung als mehrkomponentige Kontaktgläser 610, die erst auf dem Patientenauge 900 zusammengesetzt werden. Solche mehrkomponentigen Kontaktgläser 610 sind z.B. in den Dokumenten US 7,955,324 B2, US 8,500,723 B2, US 2013/053837 A1, WO 2012/041347 A1 beschrieben.

Die beiden Zuleitungen dienen zum Einen der Applikation von Unterdruck, hier über die untere Öffnung 613, und zum Anderen dem Zu-oder Abführen von Flüssigkeit in das Kontaktglas 610, wenn das Kontaktglas 610 an das Auge 900 angedockt ist, über die obere Öffnung 614.

In einer bevorzugten Variante ist weiterhin ein Überlaufaustritt 615 im oberen, dem Auge 900 fernen Mantelbereich des Kontaktglases 610 vorgesehen, über den überschüssige Flüssigkeit oder Luft beim Befüllen aus dem Kontaktglas 610 austreten kann.

Bevorzugt enthält die Patienten-Schnittstelle 600 ein mechanisch lösbares Koppelelement 651 zur mechanischen Fixation des Kontaktglases 610 am Applikator-Kopf 220. Alternativ ist es möglich, dass die Patienten-Schnittstelle 600 anstelle einer mechanischen Schnittstelle mit mechanisch lösbarem Koppelelement 651, ein Kontaktglas 610 mit einer weiteren Saugstruktur enthält, die aus dem gleichen Material wie das Kontaktglas 610 gefertigt ist. Diese weitere Saugstruktur hält bei Applikation von Unterdruck das Kontaktglas 610 am Applikator-Kopf 220. Da es sich um eine Alternativlösung handelt, ist dies nicht in Fig. 18 gezeigt.

Weiterhin vorteilhaft ist es, wenn eine Oberfläche des optischen Elements 620, die dem Mantel 611 abgewandt und dem Applikator-Kopf 200 zugewandt ist, nicht senkrecht sondern geneigt zur optischen Achse 215 angeordnet ist.

Dadurch wird vermieden, dass bei Vermessung der Augenstrukturen mittels optischer Kohärenz-Tomographie (OCT) durch das Kontaktglas 610 hindurch die Reflexe einer OCT-Kurzkohärenz-Lichtquelle an der Oberfläche des optischen Elementes 620, direkt in den OCT-Detektionsstrahlengang zurück reflektiert werden und in einem kritischen OCT-Bildbereich, die eigentlich zu messenden Augenstrukturen überstrahlen und damit verfälschen. Diese Gefahr besteht bei einer senkrechter Ausrichtung der Oberfläche des optischen Elements 620 zur optischen Achse 215.

Die Oberfläche des optischen Elements 620, die dem Mantel 611 und somit auch dem Auge 900 zugewandt ist, ist bevorzugt konvex gekrümmt. Dadurch wird zum einen eine optische Wirkung erzielt, zum andern wandern sich bildende Luftblasen entlang der gekrümmten Wände nach oben und an den Rand bzw. über den Rand der Linse und damit außerhalb der Apertur einer Kurzpuls-Laserstrahlung bzw. des OCT-Beleuchtungs- und Detektionsstrahles.

Weiterhin kann die Oberfläche des optischen Elements 620, die dem Mantel 611 und dem Auge 900 zugewandt ist, hydrophil beschichtet oder oberflächenbehandelt sein. Dadurch wird die Benetzung mit Wasser oder einer anderen Flüssigkeit wie beispielsweise einer "balanced salt solution" (BSS) und das zur-Seite-Abwandern von Luftblasen verbessert.

Es ist günstig, wenn die dem Applikator-Kopf 220 zugewandte Oberfläche des optischen Elements 620 antireflexionsbeschichtet ist, sodass die hohe Intensität der einfallenden Kurzpuls-Laserstrahlung nicht in die Gerätoptik des Systems für die Kurzpuls-Laser-Augenchirurgie 100 zurück reflektiert wird.

Insbesondere für die Sterilität ist eine Patienten-Schnittstelle 600, die zusätzlich einen Applikator-Kopf-Schutz 650 enthält, der vorzugsweise mittig eine Aussparung aufweist, von Vorteil. Dieser Applikator-Kopf-Schutz 650 kann über die dem Auge 900 zugewandten Seite des dem Applikator-Kopfs 220 aufgesetzt und mit fixiert werden, wie in der Fig. 18 gezeigt. Dieser Applikator-Kopf-Schutz verhindert, dass der Applikator-Kopf 220 während der Operation z.B. durch Flüssigkeiten verunreinigt wird. Die Aussparung erlaubt es die Patienten-Schnittstelle 600 mit dem Kontaktglas 610 direkt an dem Applikator-Kopf 220 zu befestigen, so dass der Applikator-Kopf-Schutz 650 kein Hindernis im Strahlengang der Kurzpuls-Laserstrahlung zwischen dem System für die Kurzpuls-Laser-Augenchirurgie 100 und dem optischen Element 620 des Kontaktglases 610 darstellt.

Ist die Aussparung dabei mittig im Applikator-Kopf-Schutz 650 realisiert, wird ein räumlich gleichmäßiger Schutz des Applikator-Kopfes 220 erreicht.

Bevorzugt sind das Kontaktglas 610 und der Applikator-Kopf-Schutz 650 der Patienten-Schnittstelle 600 zwei getrennte oder trennbare Teile. Ein vom Kontaktglas 610 getrennter Applikator-Kopf-Schutz 650 hat den Vorteil, dass die unterschiedlichen Anforderungen an das Kontaktglas 610, wie eine hohe Präzision bzgl. geometrischer und optischer Eigenschaften, von denen des Umfeldschutzes, wie eine möglichst einfach und kostengünstige Ausführung, getrennt und damit besser realisiert werden können.

Vorteilhaft wird der Applikator-Kopf-Schutz 650 durch ein mechanisch lösbares Koppelelement 651 mit dem Applikator-Kopf 220 verbunden.

Bevorzugt ist der obere Manteldurchmesser des Kontaktglases 610 größer als die Aussparung im Applikator-Kopf-Schutz 650. Dadurch ist ein lückenloser Schutz für die Applikator-Kopf-Oberfläche sichergestellt.

Um das Andocken und insbesondere die laterale Ausrichtung des Applikations-Kopfes 220 zu unterstützen, wird in Fig. 18 ein für die Kurzpuls-Laser-Augenchirurgie besonders geeignetes Beleuchtungssystem offenbart: In den Mantel 611 des Kontaktglases 610 ist eine lichtleitende Struktur 635 eingelassen. In den Applikator-Kopf 220 eines des Systems für die Kurzpuls-Laser-Augenchirurgie 100 wiederum ist eine Lichtquelle 630-1, die sichtbares Licht emittiert und/oder eine Lichtquelle 630-2, die infrarotes Licht emittiert, integriert. Insbesondere während des chirurgischen Eingriffs unter Einsatz einer Kurzpuls-Laserstrahlung im Auge 900, bei der die Kurzpuls-Laserstrahlung über optische Elemente des Applikator-Kopfs 220 in das Auge 900 geleitet wird und dadurch der optische Weg in einen darüber befindlichen Mikroskop-Kopf 320 versperrt oder beeinträchtigt wird, kann das Auge 900 beispielsweise mit dem infrarotem Licht 630-2 beleuchtet werden, und das vom Auge 900 reflektierte infrarote Licht über ein Strahlteiler-Prisma 350, das selektiv infrarotes Licht reflektiert, in eine Kamera 360, mit der infrarotes Licht detektiert werden kann, geleitet werden. Das Prisma 350 reflektiert jedoch nicht das sichtbare Licht bzw. Wellenlängen die von der Kurzpuls-Laserquelle 210 oder von der OCT-Lichtquelle 405 genutzt werden. Licht dieser nicht vom Prisma 350 reflektierten Wellenlängen verlaufen ohne Störung durch das Prisma 350 hindurch.

Dieser Aufbau hat den Vorteil, dass gegenüber der alternativen Lösung der Beleuchtung durch eine im Operations-Mikroskop 300 vorhandene Beleuchtung, keine Reflexe durch die zusätzlichen im Beleuchtungsstrahlengang befindlichen optischen Elemente des Applikator-Kopfs 220 hinzukommen und das Bild beeinträchtigen.

Weiterhin ist es von Vorteil, wenn im Applikator-Kopf 220 ein Kraftsensor 655 integriert ist, der bei einem Andocken der Patienten-Schnittstelle 600 in Kontakt mit dem Kontaktglas 610 steht. Der Kraftsensor 655 und sowohl die sichtbares Licht emittierende Lichtquelle 630-1 als auch die infrarotes Licht emittierende Lichtquelle 630-2 sind vorteilhaft mit einem Steuergerät 500 verbunden, das auch das System für die Kurzpuls-Laser-Augenchirurgie 100 steuert, oder aber mit einer zusätzlichen Steuereinheit 500', die mit dem Steuergerät des Systems für die Kurzpuls-Laser-Augenchirurgie 100 über Kommunikationswege in Kontakt steht..

Obige Anordnung erlaubt dann das folgende Verfahren des automatischen Umschaltens der Beleuchtung beim Andocken des Applikator-Kopfes 220 an das Auge 900:
(1) Anschalten der Lichtquelle des sichtbaren Lichtes 630-1
(2) Messung des Druckes und Leitung des Drucksignales durch den Kraftsensor 655 an das Steuergerät 500
(3) Abschalten der Lichtquelle des sichtbaren Lichtes 630-1und Anschalten der Lichtquelle des infraroten Lichts 630-2, durch das Steuergerät 500 sobald das Drucksignal des Kraftsensors 655 einen vorbestimmten Wert übersteigt.

Durch dieses automatisierte Umschalten wird verhindert, dass das Patientenauge 900 nach dem Andocken an den Applikator-Kopfes 900 mittels der Patienten-Schnittstelle 600 an das Auge 900 permanent mit potentiell den Patienten schädigendem sichtbaren Licht 630-1 beleuchtet wird. Eine Beleuchtung des Patientenauges 900 erfolgt dann mit dem weniger schädlichen infraroten Licht630-2.

### Referenzierung und Registrierung

Um die Übertragung von präoperativ gemessenen Daten z.B. die Achslage des präoperativ gemessenen Astigmatismus des Auges 900 bzw. der Hornhaut 910 oder die Soll-Lage von Zugangsschnitten oder Relaxationsschnitten gegenüber den präoperativ gemessenen Astigmatismus-Achsen des Auges 900 bzw. der Hornhaut 910 korrekt auch während der Operation auf das Auge ausrichten zu können, werden im Stand der Technik die präoperativen Daten oder gewünschten Soll-Lagen relativ zu präoperativ gewonnenen Referenzmarken festgelegt bzw. referenziert. Als Referenzmarken werden dabei künstlich eingebrachte Markierungen, wie Farbstoff-Punkte oder Corneaschnitte aber auch natürlich vorhandene Marken wie Gefäßstrukturen in der Sklera oder Irisstrukturen oder einfach nur ein Gesamtbild des Auges 900 mit seinen vorhandenen Strukturen verwendet.

Wird, wie bei der Laser-Kataraktchirurgie, ein Kontaktglas 610 verwendet, ergibt sich dabei das Problem, dass diese Marken oft durch das Kontaktglas 610, insbesondere durch die Saugringstrukturen 612 des Kontaktglases 610 verdeckt oder beeinflusst werden.

Um die Referenzmarken 640 bzw. die mit den Marken verbundene Referenzierung der präoperativen Daten oder Soll-Lagen auch bei Verwendung eines Kontaktglases 610 nutzen zu können, werden folgende Lösungen offenbart.

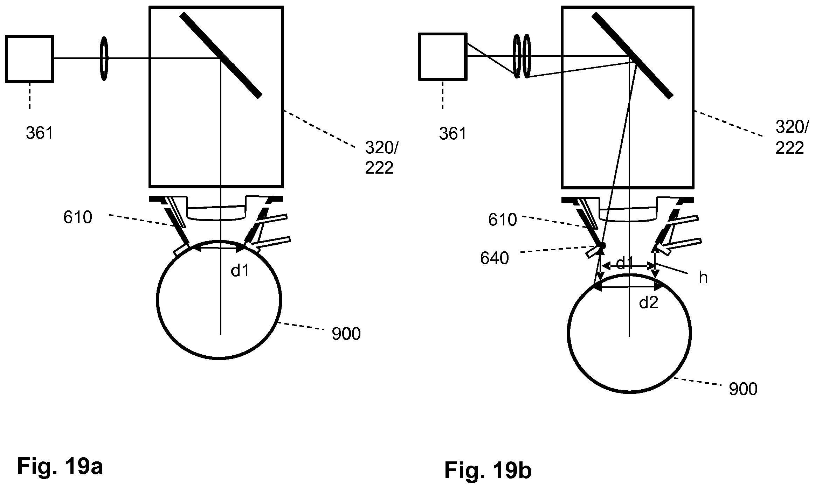

Fig. 19a offenbart einen ersten Aufbau zur Referenzierung von Laserschnitten mit einer Patienten-Schnittstelle 600 an einem Kurzpuls-Lasersystem 200. Dieser Aufbau enthält einen Mikroskop-Kopf mit einem Applikator-Kopf 320/220 oder einen Applikator-Kopf 220, eine Kamera 361 und eine Patienten-Schnittstelle 600 mit einem Kontaktglas 610. Der Abbildungsstrahlengang der Kamera 361 ist so ausgelegt, dass das Beobachtungsfeld den zentralen Teil des Kontaktglases 610 erfasst, wobei der erfasste, freie, nicht durch Ränder des Kontaktglases 610 etc. verdeckte Durchmesser d1 des Kontaktglases 610 mindestens 14mm beträgt.

Durch diesen großen freien Durchmesser wird sichergestellt, dass das ungestörte Beobachtungsfeld so groß ist, dass genügend ausgeprägte und deutlich erkennbare Marken bzw. Strukturen des Auges 900 mit der Kamera 361 sichtbar sind, und die Referenzierung der präoperativen Daten oder Soll-Lagen mit ausreichender Sicherheit erfolgen kann. Allerdings kann für kleinere Augen 900 ein solches Kontaktglas 610 insgesamt schon zu groß sein für eine zuverlässige praktische Anwendung.

Daher offenbart Fig. 19b einen zweiten Aufbau zur Referenzierung von Laserschnitten mit einer Patienten-Schnittstelle 600 an einem Kurzpuls-Lasersystem 200 im nicht angedockten Zustand. Dieser Aufbau enthält einen Mikroskop-Kopf mit einem Applikator-Kopf 320/220 oder einen Applikator-Kopf 220, eine Kamera 361 und eine Patienten-Schnittstelle 600 mit einem Kontaktglas 610, das mindestens eine Markierung 640 aufweist. Der Abbildungsstrahlengang der Kamera 361 ist so ausgelegt ist, dass das Beobachtungsfeld den zentralen Teil des Kontaktglases 610 und die Markierung 640 erfasst, wobei der freie sichtbare Durchmesser des Kontaktglases d1 mindestens 10mm, bevorzugt mindestens11mm, beträgt und die Tiefenschärfe h der Abbildung bei geeigneter Vergrößerung mindestens 5 mm beträgt.

Durch den freien Durchmesser des Kontaktglases 610 in Verbindung mit der Tiefenschärfe der Abbildung wird sichergestellt, dass selbst in abgedockten Zustand der Patienten-Schnittstelle 600, die ein Kontaktglas 610 enthält, das scharfe Bildfeld mit Durchmesser d2 auf dem Auge 900 groß genug ist, um die präoperativ gemessenen Referenzmarken des Auges 900 im Bildfeld der Kamera 361 scharf zu erfassen, und anderseits die Markierung 640 des Kontaktglases 610 ebenfalls scharf im Bildfeld sichtbar ist.

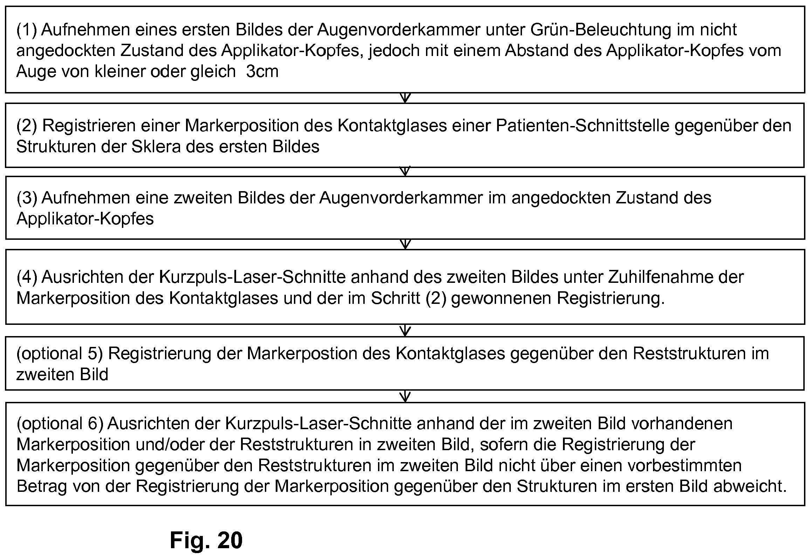

Mit diesem oder einem ähnlichem Aufbau wird das im Folgenden offenbarte Verfahren der Referenzierung für Relaxations- oder Zugangsschnitte in der Cornea ermöglicht, siehe auch Fig. 20:
(1) Aufnehmen eines ersten Bildes des Auges 900 unter Beleuchtung im nicht angedockten Zustand des Patienten-Schnittstelle 600 mit einem Kontaktglas 610 an das Patientenauge 900,
(2) Registrieren der Position der Markierung 640 des Kontaktglases 610 relativ zu den Referenzmarken des Auges 900 im ersten Bild,
(3) Aufnehmen eines zweiten Bildes des Auges 900 im angedockten Zustand der Patienten-Schnittstelle 600 mit dem Kontaktglases 610,
(4) Ausrichten der Kurzpuls-Laser-Schnitte, bevorzugt durch eine Femtosekunden-Laserstrahlung, anhand der erkennbaren Position der Markierung 640 des Kontaktglases 610 im zweiten Bild und der im Schritt (2) gewonnenen Registrierung.

Sind durch eine präoperative Diagnostik die gewünschten Schnitte relativ zu den Referenzmarken im Auge 900 festlegbar oder referenzierbar, so kann unter Zuhilfenahme obiger Schritte die Zuordnung der Schnitte zu den Referenzmarken erfolgen, auch wenn diese nicht mehr sichtbar sind, sondern durch das Kontaktglas 610 verdeckt werden.

In einer Variante des Aufbaus ist der freie Durchmesser des Kontaktglases 610 größer als 13 mm, und selbst bei angedocktem Zustand des Kontaktglases 610 an das Auge 900 sind noch Teile der für die Referenzierung notwendigen Referenzmarken im Auge 900 sichtbar.

Ist dies der Fall, dann kann das obige Verfahren weiter verbessert werden, indem die Schritte 1-3 des obigen Verfahrens durch folgende Schritte ergänzt werden:
(4) Registrierung der Position der Markierung 640 des Kontaktglases 610 gegenüber den sichtbaren Referenzstrukturen des Auges 900 im zweiten Bild.
(5) Ausrichten der Kurzpuls-Laser-Schnitte, bevorzugt durch eine Femtosekunden-Laserstrahlung , anhand der im Bild vorhandenen Position der Markierung 640 des Kontaktglases 610 oder den sichtbaren Referenzmarken im zweiten Bild, sofern die Registrierung der Position der Markierung 640 gegenüber den sichtbaren Referenzmarken des Auges 900 im zweiten Bild nicht über einen vorbestimmten Betrag von der Registrierung der Position der Markierung 640- gegenüber den Referenzmarken des Auges 900 im ersten Bild abweicht.

Ein Nachteil des obigen Aufbaus ist, dass die Optik aufwändig auf einen großen Tiefenschärfe-Bereich ausgelegt werden muss.

Daher offenbart Fig. 21a einen dritten Aufbau zur Referenzierung von Laserschnitten mit einer Patienten-Schnittstelle 600 an einem Kurzpuls-Lasersystem 200 im nicht angedockten Zustand. Dieser Aufbau enthält einen Mikroskop-Kopf mit einem Applikator-Kopf 320/220 oder einen Applikator-Kopf 220, zwei Kameras 361-1, 361-2 und eine Patienten-Schnittstelle 600 mit einem Kontaktglas 610, das mindestens eine Markierung 640 aufweist. Der Abbildungsstrahlengang der ersten Kamera 361-1 ist so ausgelegt, dass er die Markierung 640 des Kontaktglases 610 scharf erfasst, und der Abbildungsstrahlengang der zweiten Kamera 361-2 ist so ausgelegt, dass er Referenzstrukturen des Auges 900 im abgedockten Zustand scharf erfasst. Zudem beträgt der freie sichtbare Durchmesser d1 des Kontaktglases 610 mindestens 10mm, bevorzugt mindestens 11mm.

Durch den freien Durchmesser des Kontaktglases 610 in Verbindung mit der Differenz in der Fokuslage der Abbildung wird sichergestellt, dass selbst in abgedockten Zustand das scharfe Bildfeld der zweiten Kamera 361-2 mit einem Durchmesser d2 auf dem Auge 900 groß genug ist um die präoperativ gemessenen Referenzmarken des Auges 900 zu erkennen.

Mit diesem oder einem ähnlichen Aufbau wird das folgende Verfahren der Referenzierung für Relaxations- oder Zugangsschnitte offenbart:
(1) Aufnehmen eines ersten Bildes des Kontaktglases 610 mit seiner Markierung 640 durch die erste Kamera 361-1 und nahezu zeitgleiches oder zeitgleiches Aufnehmen eines zweiten Bildes des Auges 900 mit Referenzmarken des Auges 900 durch die zweite Kamera 361-2 im nicht angedockten Zustand der Patienten-Schnittstelle 600, die das Kontaktglas 610 mit seiner Markierung 640 enthält, an das Patientenauge 900.
(2) Registrieren der Position der Markierung 640 des Kontaktglases 640 im ersten Bild relativ zu den Referenzmarken des Auges 900 im zweiten Bild bei bekannter, durch den Aufbau vorgegebener Zuordnung in Ausrichtung und Vergrößerung der Bildfelder von Kamera 361-1 und 361-2.
(3) Aufnehmen eines dritten Bildes des Auges 900 im angedockten Zustand der Patienten-Schnittstelle 600 mit dem eine Markierung 640 enthaltenden Kontaktglas 610 durch die erste Kamera 361-1.
(4) Ausrichten der Kurzpuls-Laser-Schnitte, in der Regel der Femtosekunden-Laser-Schnitt, anhand der erkennbaren Position der Markierung 640 des Kontaktglases 610 im dritten Bild und der in Schritt (2) gewonnenen Registrierung.

In einer Variante dieses obigen Aufbaus und Verfahrens wird die erste Kamera 361-1 durch ein bildgebendes OCT-System ersetzt.

Alternativ hierzu kann, anstatt zwei Kameras 361-1, 361-2 parallel zu verwenden, nur eine Kamera 361 mit sequentieller Fokusverstellung verwendet werden.

Die Fig. 21b offenbart einen solchen vierten Aufbau zur Referenzierung von Laserschnitten mit einer Patienten-Schnittstelle 600 an einem Kurzpuls-Lasersystem 200 im nicht angedockten Zustand. Dieser Aufbau enthält einem Mikroskop-Kopf mit einem Applikator-Kopf 320/220 oder aber einen Applikator-Kopf 220, eine Kamera 361, eine Fokussierlinse 362 und eine Patienten-Schnittstelle 600 mit einem Kontaktglas 610, das mindestens eine Markierung 640 aufweist. Der Abbildungsstrahlengang der Kamera 361 ist so ausgelegt, dass bei einer ersten Position der Fokussierlinse 362 die Markierung 640 des Kontaktglases 610 durch die Kamera scharf erfasst wird, und bei einer zweiten Position der Fokussierlinse 362 die Referenzmarken des Auges 900 im abgedockten Zustand scharf erfasst werden. Dabei beträgt der freie sichtbare Durchmesser d1 des Kontaktglases mindestens 10mm, bevorzugt mindestens 11mm.

Durch den freien Durchmesser des Kontaktglases 610 in Verbindung mit der Differenz in der Fokuslage der Abbildung wird sichergestellt, dass selbst in abgedockten Zustand das scharfe Bildfeld mit Durchmesser d2 bei der zweiten Position der Fokussierlinse 362 auf dem Auge 900 groß genug ist, um die Referenzmarken des Auges 900 zu erfassen.

Mit diesem oder einem ähnlichen Aufbau wird das folgende Verfahren der Referenzierung für Relaxations- oder Zugangsschnitte offenbart:
(1) Aufnehmen eines ersten Bildes des Kontaktglases 610 mit seiner Markierung 640 durch die Kamera 361 in einer ersten Position der Fokussierlinse und zeitversetztes, besonders bevorzugt innerhalb von Sekunden nach Aufnahme des ersten Bildes, Aufnehmen eines zweiten Bildes des Auges 900 mit seinen Referenzmarken durch die Kamera 361 in einer zweiten Position der Fokussierlinse , beides im nicht angedockten Zustand der die Patienten-Schnittstelle 600 enthaltenden Kontaktglases 610 an das Patientenauge 900;
(2) Registrieren der Position der Markierung 640 des Kontaktglases 610 im ersten Bild relativ zu den Referenzmarken des Auges 900 im zweiten Bild bei bekannter, durch den Aufbau vorgegebener Zuordnung der Bildfelder der Kamera 361 in der ersten und in der zweiten Position der Fokussierlinse 362;
(3) Aufnehmen eines Bildes 3 des Auges im angedockten Zustand des Kontaktglases durch die Kamera in oder nahe Fokussierposition 1
(4) Ausrichten der fs-Schnitte anhand der erkennbaren Markierungs-Position des Kontaktglases in Bild 3 und der in Schritt (2) gewonnenen Registrierung.

Insgesamt sind alle der obigen Aufbauten und Verfahren zur Referenzierung und Registrierung gerätetechnisch aufwändig.

Daher offenbart Fig. 21c einem fünften Aufbau zur Referenzierung von Laserschnitten mit einer Patienten-Schnittstelle 600 an einem Kurzpuls-Lasersystem 200 im nicht angedockten Zustand. Dieser Aufbau enthält einen Mikroskop-Kopf mit einem Applikator-Kopf 320/220 oder einen Applikator-Kopf 220, eine Kamera 361, eine Fokussierlinse 362 und eine Patienten-Schnittstelle 600 mit einem Kontaktglas 610, das mindestens eine Markierung 640 enthält. Der Abbildungsstrahlengang der Kamera 361 ist so ausgelegt, dass Referenzstrukturen des Auges 900 im abgedockten Zustand scharf erfasst werden, und der freie sichtbare Durchmesser d1 des Kontaktglases 610 mindestens 11mm beträgt.

Durch den freien Durchmesser des Kontaktglases 610 in Verbindung mit der Fokuslage der Abbildung wird sichergestellt, dass selbst in abgedockten Zustand mit Abstand h des Kontaktglases 610 vom Auge 900 das scharfe Bildfeld mit Durchmesser d2 auf dem Auge 900 groß genug ist, um die Referenzmarken des Auges 900 zu erfassen.

Mit diesem oder ähnlichen Aufbau wird folgendes Verfahren der Referenzierung für Relaxations- oder Zugangsschnitte offenbart:
(1) Aufnehmen eines ersten Bildes des Auges 900 mit Referenzmarken durch die Kamera 361 im nicht angedockten Zustand der Patienten-Schnittstelle 600 mit Kontaktglas 610 an das Patientenauge 900;
(2) Andocken und fixieren einer Patienten-Schnittstelle 600 mit einem Kontaktglas 610 innerhalb von wenigen Sekunden an das Auge 900;
(3) Ausrichten der Kurzpuls-Laser-Schnitte anhand der erkennbaren Referenzstrukturen im ersten Bild.

Obige Aufbauten zur Referenzierung und Registrierung erlauben auch die Orientierung von Intraokular-Linsen (IOL), nachdem diese in den Kapselsack 910-2 eingesetzt worden sind, anhand von Referenzmarkern, die präoperativ bestimmt werden. Typischerweise erfolgt diese Referenzierung der Orientierung an Referenzmarkern, die in präoperativ gewonnen Bildern des Auges 900 erkannt werden oder an den präoperativen Bildern selbst. Werden diese präoperativen Bilder des Auges 900 bzw. deren Referenzmarker mit intraoperativen gewonnen Bildern bzw. Referenzmarken registriert, also die jeweils darin enthaltenen Strukturen einander zugeordnet und Abweichungen bestimmt, so lässt sich die Referenzieren der Orientierung an den präoperativen Bildern und Referenzmarkern mittels der Registrierung auf das intraoperativ gewonnene Bild bzw. dessen Referenzmarkern übertragen.

Beim Andocken der Patienten-Schnittstelle 600 mit Kontaktglas 610 wird jedoch oft das Aussehen der präoperativen Referenzmarken des Auges 900 bzw. des Auges 900 selbst geändert. So treten z.B. Deformationen und Blutungen auf. Daher ist die Registrierung der präoperativ gewonnenen Bilder des Auges 900 zu den intraoperativen, bei der Orientierung der Intraokularlinse (IOL) gewonnenen Bildern des Auges 900 fehleranfällig.

Diese Fehleranfälligkeit kann, wie im Folgenden beschrieben, vermieden werden:
Mittels der vorherigen Aufbauten und Verfahren zur Referenzierung und Registrierung wird das präoperative Bild zu einem Bild des Auges 900, welches für das Setzen der Kurzpuls-Laser-Schnitte bei einer angedockten, das Kontaktglas 610 enthaltenden Patienten-Schnittstelle 600verwendet wird, registriert. Dieses Bild des Auges 900, welche für das Setzen der Kurzpuls-Laser -Schnitte bei angedocktem Patienten-Schnittstelle 600 mit Kontaktglas610 verwendet wird, kann dann als neues Referenzbild betrachtet werden.

Nun wird nach der Kurzpuls-Laser-Operation, also nachdem die Patienten-Schnittstelle 600 mit dem Kontaktglas 610 auf dem Auge 900 angedockt war, im abgedockten Zustand des Kontaktglases 610 nochmals ein Bild des Auges 900 aufgenommen. Dieses Bild zeigt alle geänderten Strukturen im Auge 900. Das Bild lässt sich mit den gleichen Aufbauten zu dem neuen Referenzbild registrieren. Es wird im weiteren Verlauf der Operation, nämlich beim Einsetzen der Intraokularlinse (IOL) und deren Orientierung im Kapselsack 910-2 mit einem Bild des Auges 900, welches während der Orientierung der Intraokularlinse (IOL) aufgenommen wurde, registriert. Damit lässt sich die Referenzierung der gewünschten Orientierung der Intraokularlinse (IOL) an den präoperativen Bildern des Auges 900 über die Kette der Registrierungen verschiedener Bilder in eine Referenzierung der gewünschten Orientierung der Intraokularlinse (IOL) an intraoperativ beim Einsetzen der Intraokularlinse (IOL) gewonnenen Bildern des Auges 900 übertragen.
Für die Referenzierung der Orientierung einer Intraokularlinse (IOL) bei vorhergehender Kurzpuls-Laser_Kataraktoperation, insbesondere Femtosekunden-Laser-Kataraktoperation der Linse wird folgendes Verfahren offenbart; siehe Fig. 22:
(0) Generierung eines ersten Bildes eines astigmatischen Auges 900 mit einem (in der Regel externen) Diagnosesystem zur Erkennung der steilen und/oder flachen Achse und Speichern von erstem Bild und Achsen 900.
(1) Generierung eines zweiten Bildes als - Referenzbild oder als Bild mit Referenzmarken des Auges 900 - für die Orientierung einer Behandlung bei an das Auge 900 angedockter Patienten-Schnittstelle 600 mit Kontaktglas 610, z.B. gemäß der oben beschriebenen Verfahren;
(2) Aufnehmen eines dritten Bildes des Auges des Auges 900 im nicht angedockten Zustand des Kontaktglases 610 an das Auge 900, nachdem das die Patienten-Schnittstelle 600 mit dem Kontaktglas 610 vom Auge 900 abgedockt wurde;
(3) Registrieren des dritten Bildes gegenüber dem zweiten Bild;
(4) Aufnehmen eines vierten Bildes des Auges 900 während der Ausrichtung der in das Auge 900eingesetzten Intraokularlinse (IOL);
(5) Registrieren des vierten Bildes gegenüber dem dritten Bild;
(6) Ausrichten einer Orientierungshilfe für den Arzt im Operationsmikroskop 300 zur Orientierung einer Intraokularlinse (IOL) unter Zuhilfenahme der Registrierung in Schritt (3) und (5).

Die vorstehend genannten und in verschiedenen Ausführungsbeispielen erläuterten Merkmale der Erfindung sind dabei nicht nur in den beispielhaft angegebenen Kombinationen, sondern auch in anderen Kombinationen oder allein einsetzbar, wenn sie innerhalb des in den vorliegenden Ansprüchen definierten Umfangs der Erfindung sind.

Eine auf Vorrichtungsmerkmale bezogen Beschreibung gilt bezüglich dieser Merkmale analog für das entsprechende Verfahren, während Verfahrensmerkmale entsprechend funktionelle Merkmale der beschriebenen Vorrichtung darstellen.

## Patentansprüche

1. System für die Kurzpuls-Laser-Augenchirurgie (100) umfassend
- ein Kurzpuls-Lasersystem, das eine Kurzpuls-Laserquelle (210), ein Strahlführungsmittel (230) und einen Applikator-Kopf (220) zur Leitung einer Kurzpuls-Laserstrahlung von der Kurzpuls-Laserquelle (210) auf das zu operierende Auge (900) enthält,
- ein Operations-Mikroskop (300) mit einem Mikroskop-Kopf (320) und
- eine Steuereinheit (500), eingerichtet zur Steuerung des Systems für die Kurzpuls-Laser-Augenchirurgie,
- ein Gehäuse (110), das mindestens die Kurzpuls-Laserquelle (210) umschließt, sowie einen ersten (120) und einen zweiten Gelenkarm (130), der am Gehäuse (110) oder an einer Verlängerung des Gehäuses angeordnet ist,
- wobei der Mikroskop-Kopf (320) am ersten Gelenkarm (120) und der Applikator-Kopf (220) am zweiten Gelenkarm (130) angeordnet ist,
- wobei eine Schnittstelle (150) zwischen Applikator-Kopf (220) und Mikroskop-Kopf (320) vorgesehen ist,
- und das Strahlführungsmittel (230) den zweiten Gelenkarm (130) durchläuft und so ausgestaltet ist, dass es allen Bewegungen des zweiten Gelenkarms (130) folgen kann und in jeder Position des zweiten Gelenkarms (130) die Kurzpuls-Laserstrahlung zu ihrem Austrittsort am Applikator-Kopf (220) in gleicher Qualität führen kann,
- **dadurch gekennzeichnet, dass** der Applikator-Kopf (220) und der Mikroskop-Kopf (320) über die Schnittstelle (150) im Ablauf eines Verfahrens mechanisch und optisch miteinander verbunden und wieder gelöst werden können, und der Applikator-Kopf (220) und der Mikroskop-Kopf (320) sowohl unabhängig voneinander als auch miteinander verbunden in einem dreidimensionalen Volumen beweglich sind.

2. System für die Kurzpuls-Laser-Augenchirurgie (100) nach Anspruch 1, weiterhin umfassend ein optisches Kohärenztomographie (OCT) -Modul (400), das eine OCT-Lichtquelle (405), ein Interferometer und einen Detektor enthält.

3. System für die Kurzpuls-Laser-Augenchirurgie (100) nach Anspruch 2, eingerichtet für eine wahlweise Einkopplung einer von der OCT-Lichtquelle (405) ausgesendeten Strahlung in den Mikroskop-Kopf (320) oder in den Applikator-Kopf (220).

4. System für die Kurzpuls-Laser-Augenchirurgie (100) nach Anspruch 2 oder 3, das weiterhin einen Ringspiegel (430) zum Zusammenführen der Kurzpuls-Laserstrahlung und der von der OCT-Lichtquelle (405) ausgesendeten Strahlung enthält.

5. System für die Kurzpuls-Laser-Augenchirurgie (100) nach einem der Ansprüche 1 bis 4, wobei der erste Gelenkarm (120) und der zweite Gelenkarm (130) jeweils mindestens drei Gelenke (140, 140-O1...140-O6, 140-L1 ...140-L6) aufweisen.

6. System für die Kurzpuls-Laser-Augenchirurgie (100) nach einem der Ansprüche 1 bis 5, wobei die Steuereinheit (500) kodiert ist für eine automatische Nachführung der Lage der Kurzpuls-Laserstrahlung in Abhängigkeit von der Stellung des zweiten Gelenkarms (130).

7. System für die Kurzpuls-Laser-Augenchirurgie (100) nach einem der Ansprüche 1 bis 6, wobei der zweite Gelenkarm (130) mindestens eine Vorrichtung für einen vom ersten Gelenkarm (120) unabhängigen Gewichtsausgleich (145) aufweist.

8. System für die Kurzpuls-Laser-Augenchirurgie (100) nach einem der Ansprüche 1 bis 7, das eine Parkposition und/oder eine Transportposition für den zweiten Gelenkarm (130) mit dem Applikator-Kopf (220) am Gehäuse (110) aufweist, die auf die Geometrie des zweiten Gelenkarms (130) und/oder des Applikator-Kopfes (220) abgestimmt ist.

9. System für die Kurzpuls-Laser-Augenchirurgie (100) nach einem der Ansprüche 1 bis 8, wobei zum Lösen oder Verbinden der Schnittstelle (150) zwischen Applikator-Kopf (220) und Mikroskop-Kopf (320) ein von einem Bediener zu schaltender oder ein automatisch zu schaltender Mechanismus vorgesehen ist.

10. System für die Kurzpuls-Laser-Augenchirurgie (100) nach einem der Ansprüche 1 bis 9, wobei am ersten Gelenkarm (120) und/oder am zweiten Gelenkarm (130) und/oder am Applikator-Kopf (220) und/oder am Mikroskop-Kopf (320) verstellbare Elemente vorgesehen sind, die eine von der Steuereinheit (500) gesteuerte Bewegung des Mikroskop-Kopfes (320) und/oder des Applikator-Kopfes (220) ermöglichen.

11. System für die Kurzpuls-Laser-Augenchirurgie (100) nach einem der Ansprüche 1 bis 10, wobei das Strahlführungsmittel (230) eine photonische Kristallfaser mit hohlem Kern aufweist.

12. System für die Kurzpuls-Laser-Augenchirurgie (100) nach einem der Ansprüche 1 bis 11, wobei die Kurzpuls-Laserquelle (210) eine Femtosekunden (fs)-Laserquelle ist.

13. System für die Kurzpuls-Laser-Augenchirurgie (100) nach einem der Ansprüche 1 bis 12, dessen Kurzpuls-Lasersystem des Weiteren ein die Divergenz einer Kurzpuls-Laserstrahlung der Kurzpuls-Laserquelle (210) variierendes Linsensystem (211), ein x/y-Scansystem (240) und ein in x- und γ-Richtung bewegliches Objektiv (225) enthält.

14. System für die Kurzpuls-Laser-Augenchirurgie (100) nach Anspruch 13, wobei das Bildfeld des beweglichen Objektivs (225) größer als 1,0mm im Querschnitt aber kleiner als 6,0mm, insbesondere größer als 1,5mm aber kleiner als 3,0mm ist.

15. System für die Kurzpuls-Laser-Augenchirurgie (100) nach einem der Ansprüche 1 bis 14, das weiterhin ein System zur Stabilisierung eines Strahldurchgangs (280) durch den zweiten Gelenkarm (130) umfasst, das eine Lichtquelle (281) an einem Ende des zweiten Gelenkarms (130) und einen positionsempfindlichen Lichtsensor (282) am anderen Ende des zweiten Gelenkarms (130) enthält.

## Claims

1. System for short-pulse laser eye surgery (100), comprising
- a short-pulse laser system containing a short-pulse laser source (210), a beam guiding means (230) and an applicator head (220) for guiding short-pulse laser radiation from the short-pulse laser source (210) to the eye (900) to undergo surgery,
- a surgical microscope (300) with a microscope head (320) and
- a control unit (500), set up for controlling the system for short-pulse laser eye surgery,
- a housing (110), which surrounds at least the short-pulse laser source (210), and a first articulated arm (120) and a second articulated arm (130), arranged at the housing (110) or at an extension of the housing,
- wherein the microscope head (320) is arranged on the first articulated arm (120) and the applicator head (220) is arranged on the second articulated arm (130),
- wherein an interface (150) is provided between applicator head (220) and microscope head (320),
- and the beam guiding means (230) passes through the second articulated arm (130) and is configured such that it can follow all movements of the second articulated arm (130) and can guide the short-pulse laser radiation to its emergence location at the applicator head (220) with the same quality in any position of the second articulated arm (130),
- **characterized in that** the applicator head (220) and the microscope head (320) can be mechanically and optically interconnected and separated again via the interface (150) during the course of a procedure, and the applicator head (220) and the microscope head (320) are movable in a three-dimensional volume, both independently of one another and when connected to one another.

2. System for short-pulse laser eye surgery (100) according to Claim 1, furthermore comprising an optical coherence tomography (OCT) module (400), which contains an OCT light source (405), an interferometer and a detector.

3. System for short-pulse laser eye surgery (100) according to Claim 2, set up for alternatively coupling radiation emitted by the OCT light source (405) into the microscope head (320) or into the applicator head (220).

4. System for short-pulse laser eye surgery (100) according to Claim 2 or 3, furthermore containing a ring mirror (430) for merging the short-pulse laser radiation and the radiation emitted by the OCT light source (405).

5. System for short-pulse laser eye surgery (100) according to any one of Claims 1 to 4, wherein the first articulated arm (120) and the second articulated arm (130) each have at least three joints (140, 140-01 ... 140-O6, 140-L1 ... 140-L6).

6. System for short-pulse laser eye surgery (100) according to any one of Claims 1 to 5, wherein the control unit (500) is encoded for automatic tracking of the relative position of the short-pulse laser radiation on the basis of the position adopted by the second articulated arm (130).

7. System for short-pulse laser eye surgery (100) according to any one of Claims 1 to 6, wherein the second articulated arm (130) comprises at least one apparatus for weight balancing (145) independently of the first articulated arm (120).

8. System for short-pulse laser eye surgery (100) according to any one of Claims 1 to 7, containing a park position and/or transport position on the housing (110) for the second articulated arm (130) with the applicator head (220), said position(s) being matched to the geometry of the second articulated arm (130) and/or of the applicator head (220).

9. System for short-pulse laser eye surgery (100) according to any one of Claims 1 to 8, wherein a mechanism to be switched by an operator or a mechanism to be switched automatically is provided for detaching or connecting the interface (150) between applicator head (220) and microscope head (320).

10. System for short-pulse laser eye surgery (100) according to any one of Claims 1 to 9, wherein adjustable elements are provided on the first articulated arm (120) and/or on the second articulated arm (130) and/or on the applicator head (220) and/or on the microscope head (320), said adjustable elements facilitating a movement controlled by the control unit (500) of the microscope head (320) and/or of the applicator head (220).

11. System for short-pulse laser eye surgery (100) according to any one of Claims 1 to 10, wherein the beam guiding means (230) comprises a photonic crystal fibre with a hollow core.

12. System for short-pulse laser eye surgery (100) according to any one of Claims 1 to 11, wherein the short-pulse laser source (210) is a femtosecond (fs) laser source.

13. System for short-pulse laser eye surgery (100) according to any one of Claims 1 to 12, the short-pulse laser system of which furthermore contains a lens system (211) that varies the divergence of short-pulse laser radiation of the short-pulse laser source (210), an x/y-scanning system (240) and an objective (225) that is movable in the x- and γ-direction.

14. System for short-pulse laser eye surgery (100) according to Claim 13, wherein the image field of the movable objective (225) is larger than 1.0 mm in cross section but smaller than 6.0 mm, more particularly larger than 1.5 mm but smaller than 3.0 mm.

15. System for short-pulse laser eye surgery (100) according to any one of Claims 1 to 14, furthermore comprising a system for stabilizing a beam passage (280) through the second articulated arm (130), said system containing a light source (281) at one end of the second articulated arm (130) and a position-sensitive light sensor (282) at the other end of the second articulated arm (130).

## Revendications

1. Système de chirurgie oculaire au laser à impulsions courtes (100), ledit système comprenant
- un système laser à impulsions courtes qui contient une source laser à impulsions courtes (210), un moyen de guidage de faisceau (230) et une tête d'application (220) destinée à guider un rayonnement laser à impulsions courtes de la source laser à impulsions courtes (210) à l'œil (900) à opérer,
- un microscope chirurgical (300) pourvu d'une tête de microscope (320) et
- une unité de commande (500) conçue pour commander le système de chirurgie oculaire au laser à impulsions courtes,
- un boîtier (110) qui renferme au moins la source laser à impulsions courtes (210), ainsi qu'un premier bras articulé (120) et un deuxième bras articulé (130) qui sont disposés au niveau du boîtier (110) ou d'un prolongement du boîtier,
- la tête de microscope (320) étant disposée au niveau du premier bras articulé (120) et la tête d'application (220) étant disposée au niveau du deuxième bras articulé (130),
- une interface (150) étant prévue entre la tête d'application (220) et la tête de microscope (320),
- et le moyen de guidage de faisceau (230) s'étendant à travers le deuxième bras articulé (130) et étant conçu de manière à pouvoir suivre tous les mouvements du deuxième bras articulé (130) et à pouvoir guider avec une qualité égale le rayonnement laser à impulsions courtes vers son point de sortie au niveau de la tête d'application (220) dans chaque position du deuxième bras articulé (130),
- **caractérisé en ce que** la tête d'application (220) et la tête de microscope (320) peuvent être reliées mécaniquement et optiquement l'une à l'autre, et désolidarisées, par le biais de l'interface (150) au cours d'une opération, et la tête d'application (220) et la tête de microscope (320) sont mobiles dans un volume tridimensionnel aussi bien en étant indépendantes l'une de l'autre et qu'en étant reliées l'une à l'autre.

2. Système de chirurgie oculaire au laser à impulsions courtes (100) selon la revendication 1, comprenant en outre un module de tomographie par cohérence optique (OCT) (400) qui contient une source de lumière OCT (405), un interféromètre et un détecteur.

3. Système de chirurgie oculaire au laser à impulsions courtes (100) selon la revendication 2, conçu pour injecter par couplage un rayonnement, émis par la source de lumière OCT (405), au choix dans la tête de microscope (320) ou dans la tête d'application (220) .

4. Système de chirurgie oculaire au laser à impulsions courtes (100) selon la revendication 2 ou 3, qui contient en outre un miroir annulaire (430) destiné à réunir le rayonnement laser à impulsions courtes et le rayonnement émis par la source de lumière OCT (405).

5. Système de chirurgie oculaire au laser à impulsions courtes (100) selon l'une des revendications 1 à 4, le premier bras articulé (120) et le deuxième bras articulé (130) comportant chacun au moins trois articulations (140, 140-01...140-06, 140-L1...140-L6) .

6. Système de chirurgie oculaire au laser à impulsions courtes (100) selon l'une des revendications 1 à 5, l'unité de commande (500) étant codée pour le suivi automatique de la position du rayonnement laser à impulsions courtes en fonction de la position du deuxième bras articulé (130).

7. Système de chirurgie oculaire au laser à impulsions courtes (100) selon l'une des revendications 1 à 6, le deuxième bras articulé (130) comportant au moins un dispositif de compensation de poids (145) indépendant du premier bras articulé (120).

8. Système de chirurgie oculaire au laser à impulsions courtes (100) selon l'une des revendications 1 à 7, qui présente une position de stationnement et/ou une position de transport du deuxième bras articulé (130), pourvu de la tête d'application (220), au niveau du boîtier (110), lesquelles positions sont adaptées à la géométrie du deuxième bras articulé (130) et/ou de la tête d'application (220).

9. Système de chirurgie oculaire au laser à impulsions courtes (100) selon l'une des revendications 1 à 8, un mécanisme à commutation automatique ou à commutation par le biais d'un opérateur étant prévu pour désolidariser ou relier l'interface (150) entre la tête d'application (220) et la tête de microscope (320) .

10. Système de chirurgie oculaire au laser à impulsions courtes (100) selon l'une des revendications 1 à 9, des éléments réglables au niveau du premier bras articulé (120) et/ou du deuxième bras articulé (130) et/ou de la tête d'application (220) et/ou de la tête de microscope (320) étant prévus qui permettent un mouvement de la tête de microscope (320) et/ou de la tête d'application (220) qui est commandé par l'unité de commande (500).

11. Système de chirurgie oculaire au laser à impulsions courtes (100) selon l'une des revendications 1 à 10, le moyen de guidage de faisceau (230) comprenant une fibre à cristal photonique à âme creuse.

12. Système de chirurgie oculaire au laser à impulsions courtes (100) selon l'une des revendications 1 à 11, la source laser à impulsions courtes (210) étant une source laser femtoseconde (fs).

13. Système de chirurgie oculaire au laser à impulsions courtes (100) selon l'une des revendications 1 à 12, dont le système laser à impulsions courtes comprend en outre un système de lentilles (211) qui fait varier la divergence d'un rayonnement laser à impulsions courtes de la source laser à impulsions courtes (210), un système de balayage x/y (240) et un objectif (225) qui peut être déplacé dans les directions x et y.

14. Système de chirurgie oculaire au laser à impulsions courtes (100) selon la revendication 13, le champ image de l'objectif déplaçable (225) étant supérieur à 1,0 mm en coupe transversale mais inférieur à 6,0 mm, notamment supérieur à 1,5 mm mais inférieur à 3,0 mm.

15. Système de chirurgie oculaire au laser à impulsions courtes (100) selon l'une des revendications 1 à 14, comprenant en outre un système de stabilisation d'un passage de faisceau (280) à travers le deuxième bras articulé (130), lequel système de stabilisation contient une source de lumière (281) à une extrémité du deuxième bras articulé (130) et un capteur de lumière (282), sensible à la position, à l'autre extrémité du deuxième bras articulé (130).
